# FASCICULE DE BREVET EUROPEEN

(11) **EP 0 737 685 B1**
(45) Date de publication et mention de la délivrance du brevet: **19.07.2000**
(21) Numéro de dépôt: 96400778.5
(22) Date de dépôt: 11.04.1996
(51) Int. Cl.: C07D 471/04, C07D 491/04, C07F 7/08, A61K 31/435, A61K 31/695, C07D 495/04

(54) **Composés pyridiniques, leurs procédés de préparation et les compositions pharmaceutiques qui les contiennent**
Pyridinderivate, Verfahren zu ihrer Herstellung und diese enthaltende pharmazeutische Zubereitungen
Pyridine derivatives, process for their preparation and pharmaceutical compositions containing them

(30) Priorité: 14.04.1995 FR 9504504
(43) Date de publication de la demande: 16.10.1996
(73) Titulaire: ADIR ET COMPAGNIE, 92415 Courbevoie Cédex (FR)
(72) Inventeur: Viaud, Marie-Claude, 45000 ORLEANS (FR); Guillaumet, Gérald, 45650 Saint Jean le Blanc (FR); Mazeas, Daniel, 28630 Morancez (FR); Vandepoel, Hervé, 45000 Orléans (FR); Renard, Pierre, 78000 Versailles (FR); Pfeiffer, Bruno, 95600 Eaubonne (FR); Delagrange, Philippe, 92130 Issy les Moulineaux (FR)

(56) Documents cités:
- EP-A- 0 527 687
- WO-A-95/33748
- WO-A-96/04266

## Description

L'invention concerne de nouveaux composés pyridiniques, leurs procédés de préparation et les compositions pharmaceutiques qui les contiennent.

L'invention décrit de nouveaux composés pyridiniques qui s'avèrent être de puissants ligands des récepteurs mélatoninergiques.

De nombreuses études ont mis en évidence ces dix dernières années, le rôle capital de la mélatonine (5-méthoxy N-acétyl tryptamine) dans le contrôle du rythme circadien et des fonctions endocrines, et les récepteurs de la mélatonine ont été caractérisés et localisés.

Outre leur action bénéfique sur les troubles du rythme circadien (J. Neurosurg 1985, 63, pp 321-341) et du sommeil (Psychopharmacology, 1990, 100 pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement de la maladie de Parkinson (J. Neurosurg 1985, 63, pp 321-341) et d'Alzheimer (Brain Research, 1990, 528, pp 170-174). De même, ces composés ont montré une activité sur certains cancers (Melatonin - Clinical Perspectives, Oxford University Press, 1988, pp 164-165), sur l'ovulation (Science 1987, 227, pp 714-720, et sur le diabète (Clinical endocrinology, 1986, 24, pp 359-364).

Des composés permettant d'agir sur le système mélatoninergique sont donc pour le clinicien d'excellents médicaments pour le traitement des pathologies liées aux troubles du système mélatoninergique. Ainsi l'art antérieur décrit des ligands mélatoninergiques à structure non pyridinique, comme par exemple dans la demande EP 0 527 687.

On connaît dans la littérature la N-[2-(1H-pyrrolo[3,2-c]pyridin-3-yl)éthyl]acétamide (Farmaco Ed. Sci. 1964, 19, pp 741-750).

On connaît également dans la littérature des dérivés 4-méthyl-1-phényl-pyrrolo[2,3-b]pyridine (YAKHONTOV L.N. et al : Chemical Abstracts, vol 64 (1) 1966 colonne 19584 a (Khim. Geterotsikl. Soedin., Akad, Nauk. Latv. SSR, 1966 (1) pp 80-4). et Chemical Abstracts, vol. 64 (1) 1966 colonne 5057d (Biol. Aktivn. Soedin. Akad. Nauk. SSR, 1965, pp 83-90) mais aucun activité pharmacologique n'est mentionnée à leurs propos, ces composés étant présentés comme intermédiaires de synthèse pour obtenir des dérivés 12-aza-β-carboline.

L'invention concerne les composés de formule (I) : 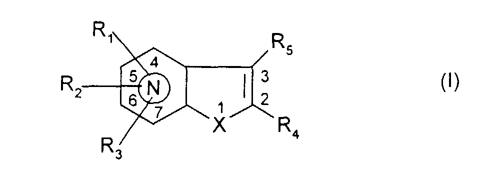 dans laquelle
- R₁, R₂, R₃ et R₄ représentent, chacun indépendamment l'un de l'autre, un hydrogène ou un radical choisi parmi halogène, hydroxy, Ra et -O-Ra ; avec Ra choisi parmi alkyl, alkyl substitué par un ou plusieurs halogènes, trialkylsilyl, alcényl, alcynyl, cycloalkyl, cycloalkylalkyl, aryl, aryl substitué, arylalkyl et arylalkyl substitué ;
- R₅ représente un groupement de formule -A-B-Y dans lequel
   - A représente une chaîne (C₁-C₆) alkylène non substituée ou substituée par un ou plusieurs alkyles,
   - B représente un groupement B₁, B₂ ou B₃: 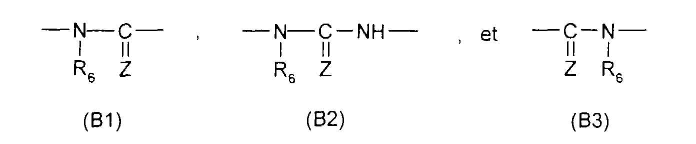 dans lesquels Z représente un oxygène ou un soufre et R₆ représente un hydrogène ou un radical choisi parmi alkyl, cycloalkyl, cycloalkylalkyl, aryl et arylalkyl,
   - Y représente un radical Y₁ choisi parmi alkyl, alkyl substitué par un ou plusieurs halogènes, alcényl, alcynyl, cycloalkyl et cycloalkylalkyl ; Y peut également représenter un hydrogène lorsque B représente un groupement B₁ ou B₂,
- et X représente un oxygène, un soufre ou un groupement 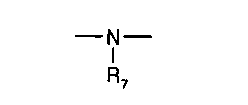 dans lequel R₇ représente un hydrogène ou un radical choisi parmi alkyl, aryl, aryl substitué, arylalkyl et arylalkyle substitué ; ou R₇ représente un groupement de formule -A-B-Y tel que défini précédemment et dans ce cas R₅ représente une valeur choisie parmi celles définies pour R₁, R₂, R₃ et R₄, telles que définies précédemment,
avec la réserve
- que le composé de formule (I) ne peut être le N-[2-(1H-pyrrolo[3,2-*c*]pyridin-3-yl)éthyl]acétamide,
- et que R₇ ne peut pas représenter un phényl lorsque l'azote du noyau pyridine de la formule (I) est en position 7 de l'hétérocycle, R₁ est un groupement alkyle en position 4 de l'hétérocycle et R₂, R₃ et R₄ représentent des hydrogènes,
étant entendu que :
- les termes "alkyl" et "alkoxy" désignent des groupements linéaires ou ramifiés contenant de 1 à 6 atomes de carbone,
- les termes "alcényl" et "alcynyl" désignent des groupements insaturés linéaires ou ramifiés contenant de 2 à 6 atomes de carbone,
- le terme "cycloalkyl" désigne un groupement de 3 à 8 atomes de carbone,
- le terme "aryl" désigne un radical phényl, naphthyl ou pyridyl,
- le terme "substitué" affecté aux expressions "aryl" et "arylalkyl" signifie que ces groupements peuvent être substitués sur les noyaux aromatiques par un ou plusieurs radicaux choisis parmi halogène, alkyl, alkoxy, hydroxy et alkyl substitué par un ou plusieurs halogènes ;
leurs énantiomères et diastéréoisomères, et leurs sels d'addition à un acide ou une base pharmaceutiquement acceptable.

L'invention concerne particulièrement, les composés de formule (I) dans lesquels, pris ensemble ou séparément,
- R₁, R₂ et R₃ représentent simultanément des hydrogènes,
- un des substituants R₁, R₂ et R₃ représente un radical choisi parmi halogène, alkoxy et alkyl et les deux autres représentent des hydrogènes,
- A représente une chaîne méthylène,
- A représente une chaîne éthylène,
- A représente une chaîne triméthylène,
- B représente un groupement -N(R₆)-C(=Z)-
- B représente un groupement -N(R₆)-C(=Z)-NH-
- B représente un groupement -C(=Z)-N(R₆)-
- Z représente un oxygène,
- Z représente un soufre,
- Y représente un alkyle,
- Y représente un cycloalkyle,
- X représente un oxygène,
- X représente un soufre,
- et X représente un groupement -N(R₇)-.

Par exemple, l'invention concerne les cas particuliers de composés de formule (I) répondant aux formules (1) à (11) suivantes : 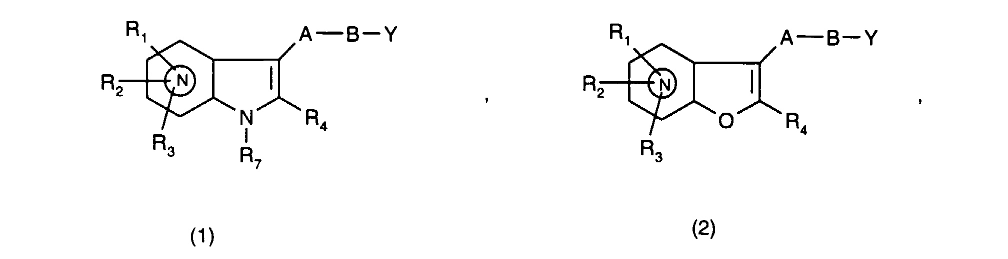 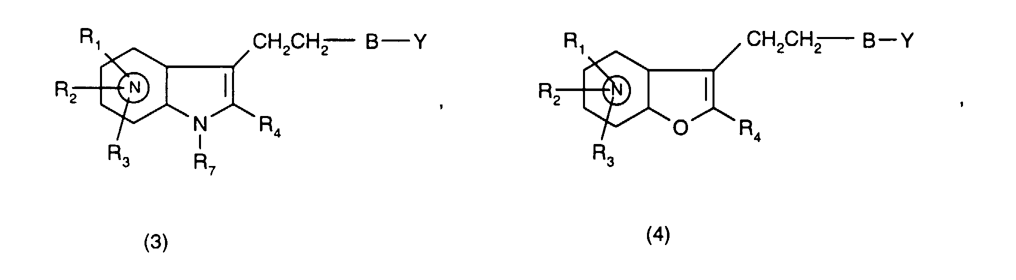 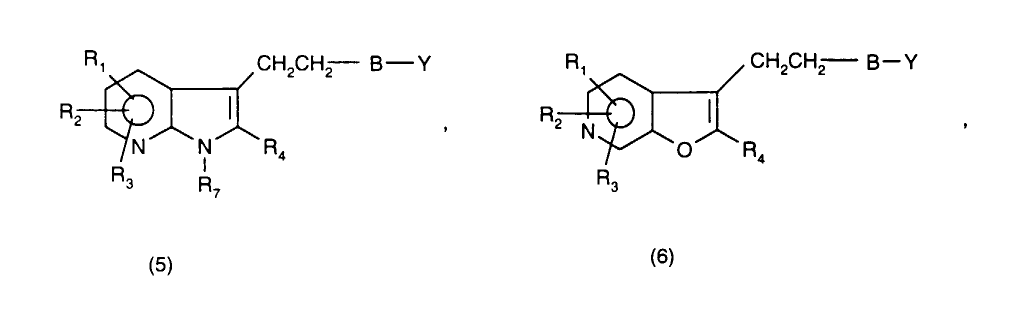 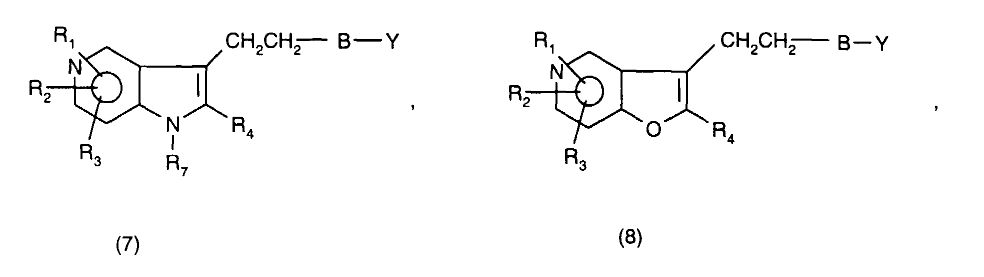 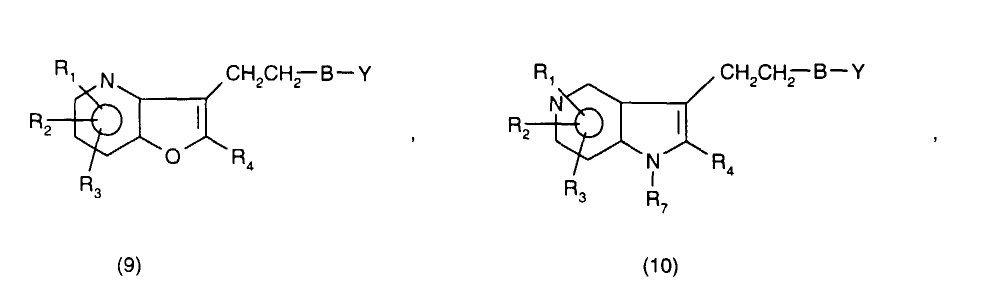 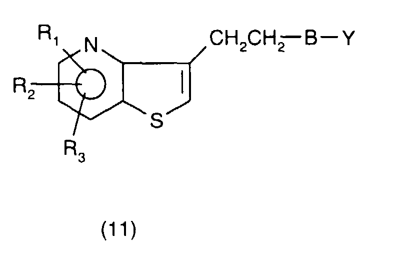 dans lesquelles R₁, R₂, R₃, R₄, R₇, A, B et Y sont tels que définis dans la formule (I).

L'invention concerne plus particulièrement les cas des composés de formules (1) à (11) dans lesquels R₁, R₂, R₃ et R₄ représentent simultanément des hydrogènes ou un des substituants R₁, R₂, R₃ ou R₄ est un radical choisi parmi halogène, alkoxy et alkyl et les autres substituants R₁, R₂, R₃ ou R₄ restants sont des hydrogènes.

Par exemple, l'invention concerne les cas particuliers des composés de formule (I) répondant aux formules (12) et (13) suivantes : 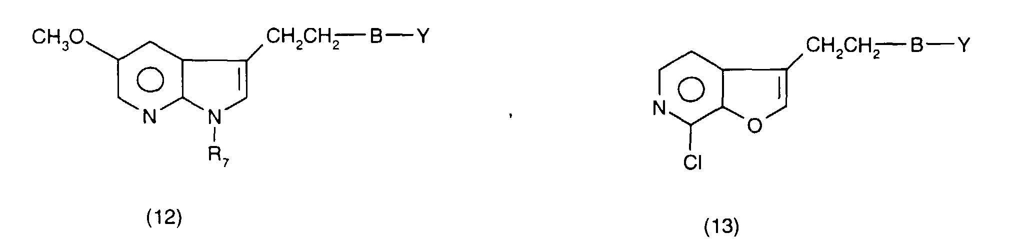 dans lesquelles R₇, B et Y sont tels que définis dans la formule (I).

De façon particulière, les radicaux alkyles présents dans la formule (I) peuvent être choisis parmi méthyle, éthyle, n-propyle, isopropyle, n-butyle, isobutyle, sec-butyle, tert-butyle, pentyle et hexyle,
les radicaux alkoxy présents dans la formule (I) peuvent être choisis parmi méthoxy, éthoxy, n-propoxy, isopropoxy, n-butoxy, iso-butoxy, sec-butoxy, tert-butoxy, pentyloxy et hexyloxy,
les halogènes présents dans la formule (I) peuvent être choisis parmi le brome, le chlore, le fluor et l'iode,
les cycloalkyles présents dans la formule (I) peuvent être choisis parmi cyclopropyle, cyclobutyle, cyclopentyle, cyclohexyle, cycloheptyle et cydooctyle ;
et les groupements alkylène présents dans la formule (I) peuvent être choisis parmi méthylène, éthylène, triméthylène, tétraméthylène, pentaméthylène et hexaméthylène.

Parmi les acides pharmaceutiquement acceptables que l'on peut utiliser pour former un sel d'addition avec les composés de l'invention, on peut citer, à titre d'exemples et de façon non limitative, les acides chlorydrique, sulfurique, phosphorique, tartrique, malique, maléique, fumarique, oxalique, méthanesulfonique, éthanesulfonique, camphorique et citrique.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine et l'arginine.

L'invention concerne également le procédé de préparation des composés de formule (I) caractérisé en ce que, lorsque B représente un groupement B₁ ou B₂ tels que définis dans la formule (I), on fait réagir une amine de formule (II) : 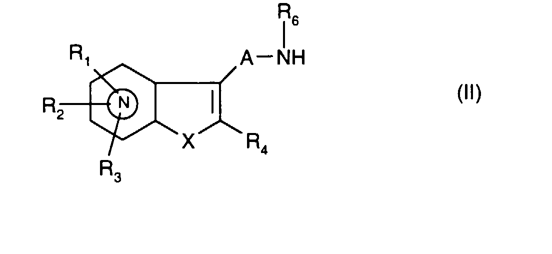 dans laquelle R₁, R₂, R₃, R₄, R₆, A et X sont tels que définis dans la formule (I)
- soit avec un composé de formule (IIIa) ou (IIIb) : 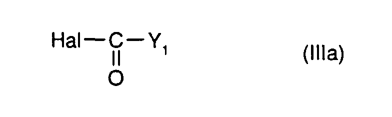 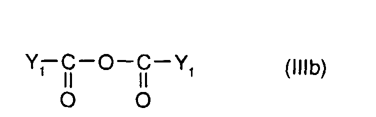 l'anhydride (IIIb) pouvant être mixte ou symétrique,
   dans lesquelles Y₁ est tel que défini dans la formule (I) est Hal représente un atome d'halogène afin d'obtenir les composés de formule (I/a) : 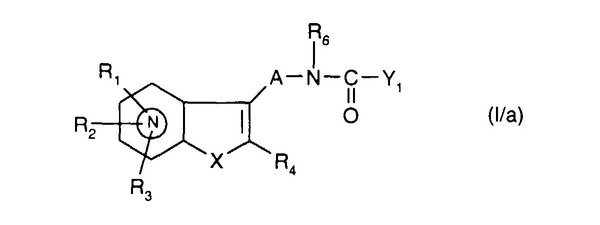 dans laquellle R₁, R₂, R₃, R₄, R₆, A, Y₁ et X sont tels que définis précédemment,
   composé de formule (I/a) qui est ensuite soumis au réactif de Lawesson pour obtenir les composés de formule (I/b) : 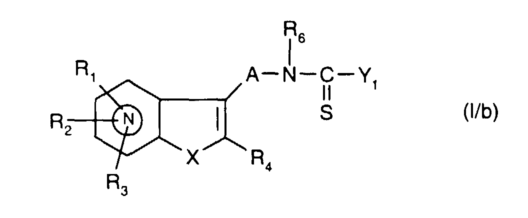 dans laquelle R₁, R₂, R₃, R₄, R₆, A, Y₁ et X sont tels que définis précédemment,
- soit avec l'acide formique pour obtenir un composé de formule (I/c) : 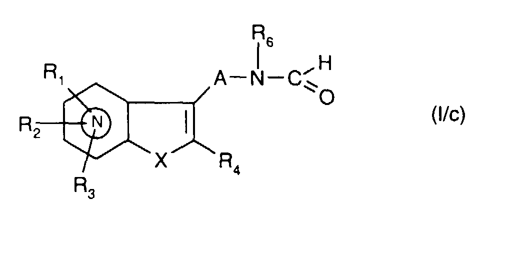 dans laquelle R₁, R₂, R₃, R₄, R₆, A et X sont tels que définis précédemment,
- soit avec un composé de formule (IV) :

   Z=C=N-Y (IV)

   dans laquelle Y et Z sont tels que définis dans la formule (I) afin d'obtenir les composés de formule (I/d) : 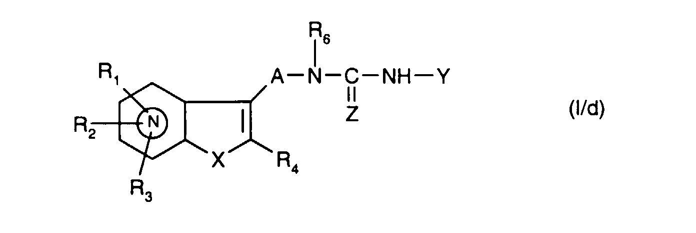 dans laquelle R₁, R₂, R₃, R₆, A, X, Y et Z sont tels que définis précédemment,
   et caractérisé en ce que, lorsque B représente un groupement B₃ tel que défini dans la formule (I) on fait réagir un composé de formule (V) : 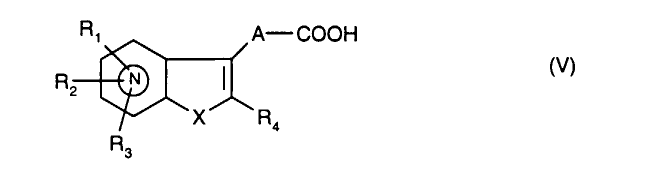 dans laquelle R₁, R₂, R₃, R₄, A et X sont tels que définis dans la formule (I) avec une amine de formule (VI) : 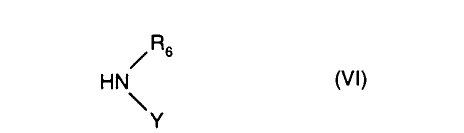 dans laquelle R₆ et Y sont tels que définis dans la formule (I) pour obtenir le composé de formule (I/e) : 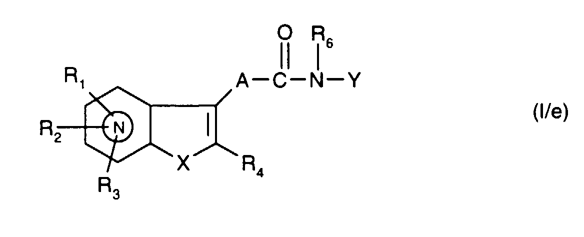 dans laquelle R₁, R₂, R₃, R₄, R₆, A, X et Y sont tels que définis précédemment,
   composés de formule (I/e) qui sont soumis au réactif de Lawesson pour obtenir les composés de formule ((I/f) : 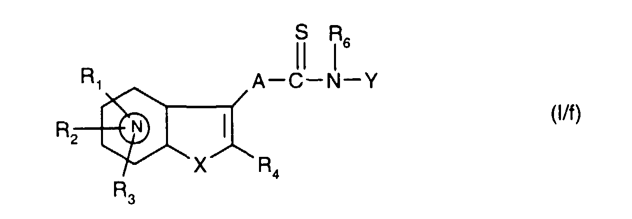 dans laquelle R₁, R₂, R₃, R₄, R₆, A, X et Y sont tels que définis précédemment,
étant entendu que, lorsque R₇ tel que défini dans la formule (I) représente un groupement de formule -A-B-Y tel que défini dans la formule (I), le procédé de préparation est analogue à celui décrit ci-dessus, les réactifs de formule (IIIa), (IIIb) et (IV) et l'acide formique d'une part ou (VI) d'autre part réagissant respectivement sur des groupements de formule -A-NH-R₆ ou -A-COOH, fixés en position 1 de l'hétérocycle pyrrolopyridine présent dans la formule (I),
les composés de formule (I) obtenus pouvant être :
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

L'invention concerne également l'obtention des composés de formule (I/g), cas particuliers des composés de formule (I) dans lesquels R₆ est différent de l'hydrogène par alkylation d'un composé de formule (I/h), cas particulier des composés de formule (I) dans lesquels R₆ est un hydrogène.

L'invention concerne également le procédé de préparation des composés de formule (I) caractérisé en ce qu'on condense un composé de formule (VII) 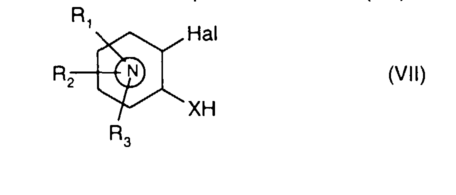 dans laquelle R₁, R₂, R₃ et X sont tels que définis dans la formule (I) et Hal représente un atome d'halogène
avec un composé de formule (VIII) : 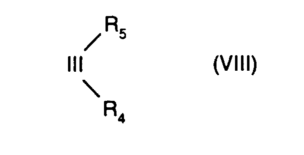 dans laquelle R₄ et R₅ sont tels que définis dans la formule (I),
les composés de formule (I) obtenus pouvant être :
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

Par exemple, l'invention concerne le procédé de préparation des composés de formule (I') : 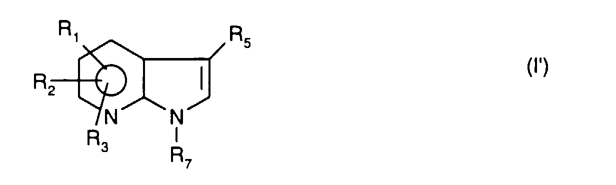 dans laquelle R₁, R₂, R₃, R₅ et R₇ sont tels que définis dans la formule (I)
caractérisé en ce que, lorsque B représente un groupement B₁ ou B₂ tels que définis dans la formule (I), on fait réagir une amine de formule (II') : 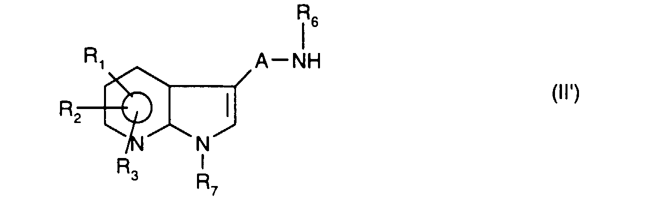 dans laquelle R₁, R₂, R₃, R₆, R₇ et A sont tels que définis dans la formule (I)
- soit avec un composé de formule (IIIa) ou (IIIb) : 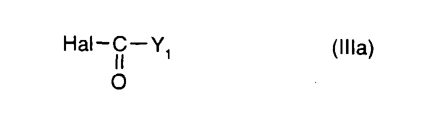 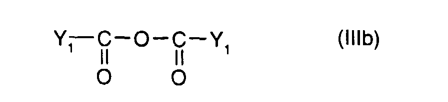 l'anhydride (Illb) pouvant être mixte ou symétrique,
   dans lesquelles Y₁ est tel que défini dans la formule (I) et Hal représente un atome d'halogène afin d'obtenir les composés de formule (I/a') : 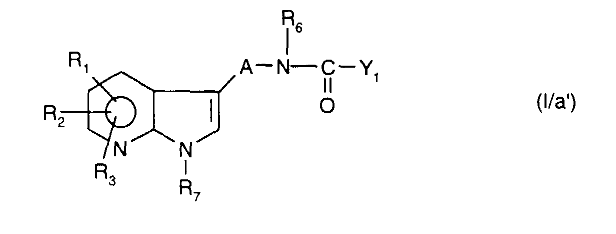 dans laquelle R₁, R₂, R₃, R₆, R₇, A et Y₁ sont tels que définis précédemment,
   composé de formule (I/a') qui est ensuite soumis au réactif de Lawesson pour obtenir les composés de formule (I/b') : 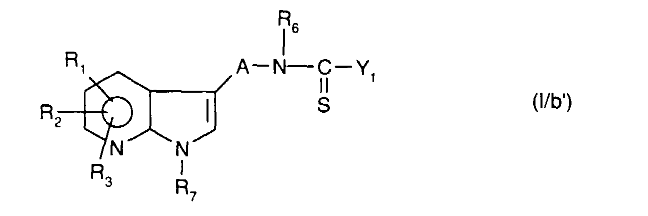 dans laquelle R₁, R₂, R₃, R₆, R₇, A et Y₁ sont tels que définis précédemment
- soit avec l'acide formique pour obtenir un composé de formule (I/c') : 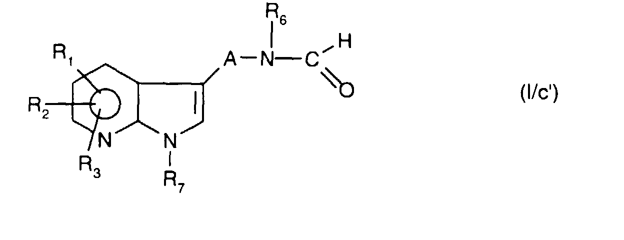 dans laquelle R₁, R₂, R₃, R₆, R₇ et A sont tels que définis précédemment,
- soit avec un composé de formule (IV) :

   Z=C=N-Y (IV)

   dans laquelle Y et Z sont tels que définis dans la formule (I) afin d'obtenir les composés de formule (I/d') : 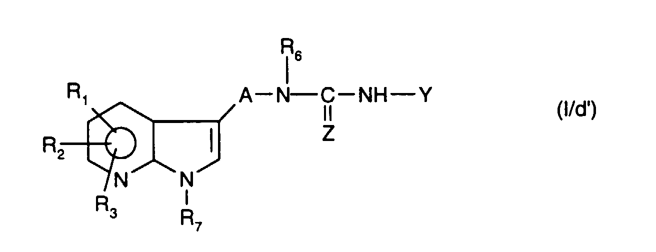 dans laquelle R₁, R₂, R₃, R₆, A, Y et Z sont tels que définis précédemment,
   et caractérisé en ce que, lorsque B représente un groupement B₃ tel que défini dans la formule (I) on fait réagir un composé de formule (V') : 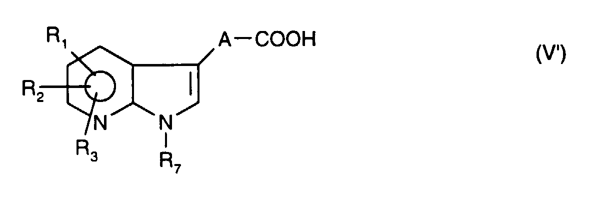 dans laquelle R₁, R₂, R₃, R₇ et A sont tels que définis dans la formule (I) avec une amine de formule (VI) : 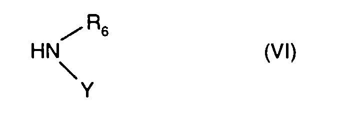 dans laquelle R₆ et Y sont tels que définis dans la formule (I) pour obtenir le composé de formule (I/e') : 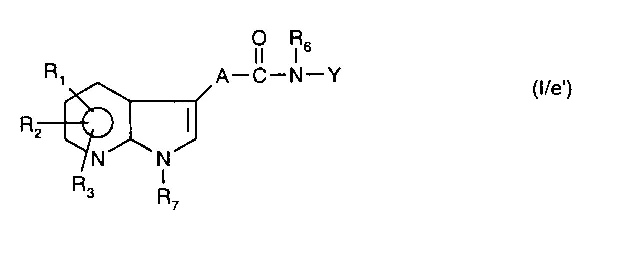 dans laquelle R₁, R₂, R₃, R₆, R₇, A et Y sont tels que définis précédemment,
   composés de formule (I/e') qui sont soumis au réactif de Lawesson pour obtenir les composés de formule ((I/f') : 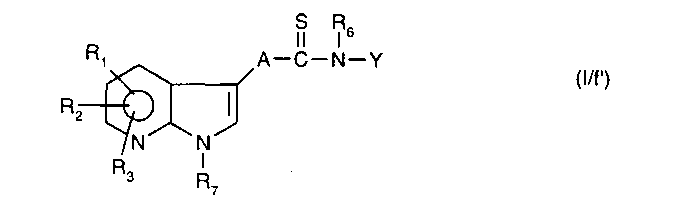 R₁, R₂, R₃, R₆, R₇, A et Y sont tels que définis précédemment,
les composés de formule (I/a'), (I/b'), (I/c'), (I/d'), (I/e') et (I/f') formant l'ensemble des composés de formule (I'),
composés de formule (I') qui sont, le cas échéant
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

L'invention concerne également l'obtention des composés de formule (I/g'), cas particuliers des composés de formule (I') dans lesquels R₆ est différent de l'hydrogène par alkylation d'un composé de formule (I/h'), cas particulier des composés de formule (I') dans lesquels R₆ est un hydrogène.

Par exemple, l'invention concerne le procédé de préparation des composés de formule (I") : 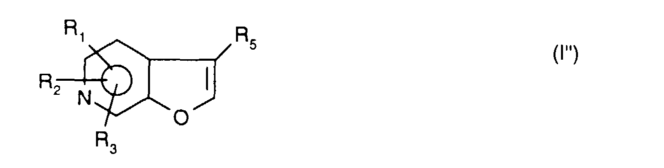 dans laquelle R₁, R₂, R₃ et R₅ sont tels que définis dans la formule (I)
caractérisé en ce que, lorsque B représente un groupement B₁ où B₂ tels définis dans la formule (I), on fait réagir une amine de formule (II") : 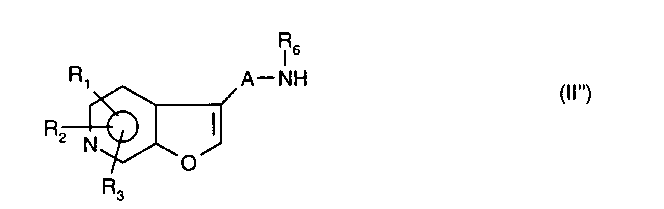 dans laquelle R₁, R₂, R₃, R₆ et A sont tels que définis dans la formule (I) soit avec un composé de formule (IIIa) ou (IIIb) : 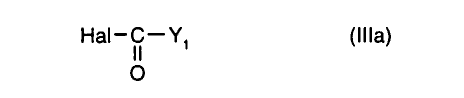 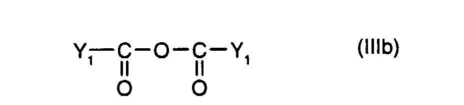 l'anhydride (IIIb) pouvant être mixte ou symétrique,
dans lesquelles Y₁ est tel que défini dans la formule (I) et Hal représente un atome d'halogène afin d'obtenir les composés de formule ((I/a") : 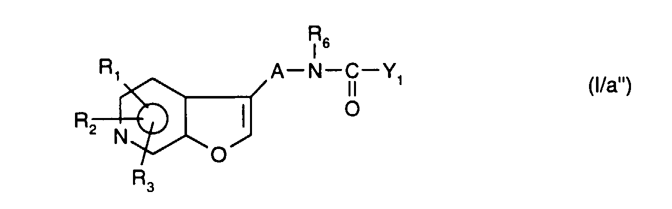 dans laquelle R₁, R₂, R₃, R₆, A et Y₁ sont tels que définis précédemment,
composé de formule (I/a) qui est ensuite soumis au réactif de Lawesson pour obtenir les composés de formule (I/b") : 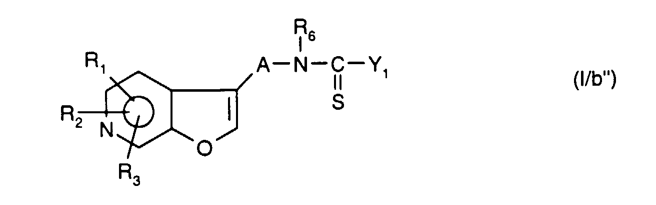 dans laquelle R₁, R₂, R₃, R₆, A et Y₁ sont tels que définis précédemment,
- soit avec l'acide formique pour obtenir un composé de formule (I/c") : 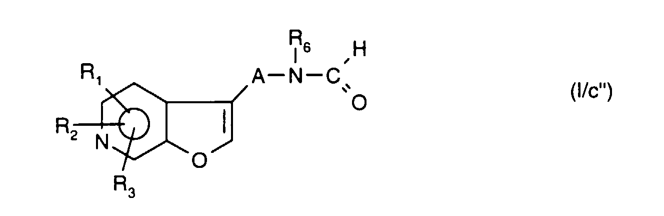 dans laquelle R₁, R₂, R₃, R₆ et A sont tels que définis précédemment,
- soit avec un composé de formule (IV) :

   Z=C=N-Y (IV)

   dans laquelle Y et Z sont tels que définis dans la formule (I) afin d'obtenir les composés de formule (I/d") : 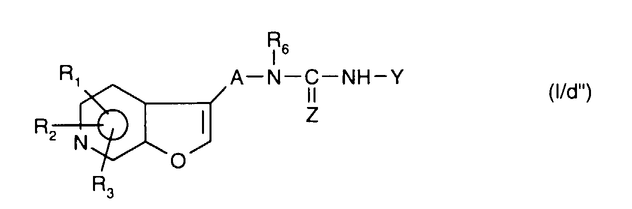 dans laquelle R₁, R₂, R₃, R₆, A, Y et Z sont tels que définis précédemment,
   et caractérisé en ce que, lorsque B représente un groupement B₃ tel que défini dans la formule (I), on fait réagir un composé de formule (V") : 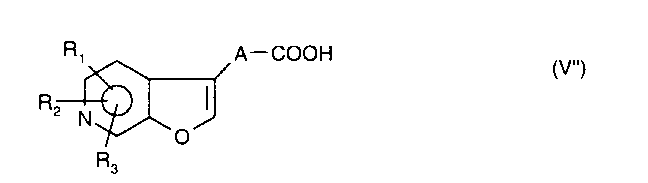 dans laquelle R₁, R₂, R₃ et A sont tels que définis dans la formule (I) avec une amine de formule (VI) : 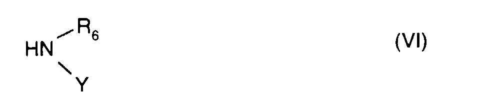 dans laquelle R₆ et Y sont tels que définis dans la formule (I) pour obtenir le composé de formule (I/e") : 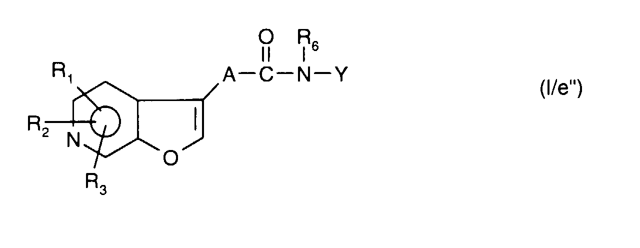 dans laquelle R₁, R₂, R₃, R₆, A et Y sont tels que définis précédemment,
   composés de formule (I/e") qui sont soumis au réactif de Lawesson pour obtenir les composés de formule (I/f") : 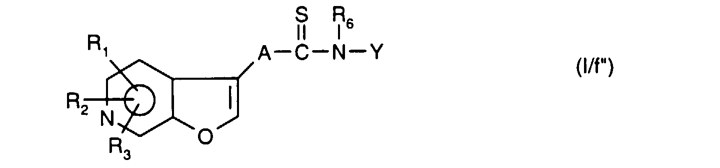 R₁, R₂, R₃, R₆, A, X et Y sont tels que définis précédemment,
les composés de formule (I/a"), (I/b"), (I/c"), (I/d"), (I/e") et (I/f") formant l'ensemble des composés de formule (I")
composés de formule (I") qui sont, le cas échéant
- purifiés suivant un ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

L'invention concerne également l'obtention des composés de formule (I/g"), cas particuliers des composés de formule (I") dans lesquels R₆ est différent de l'hydrogène par alkylation d'un composé de formule (I/h"), cas particulier des composés de formule (I") dans lesquels R₆ est un hydrogène.

L'invention concerne également le procédé de préparation des composés de formule (I/i), cas particulier des composés de formule (I) : 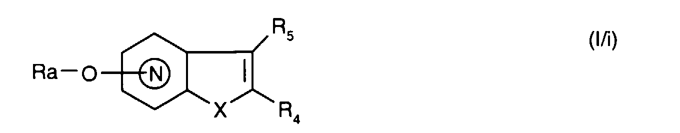 dans laquelle Ra, R₄, R₅ et X sont tels que définis dans la formule (I) par greffage du radical Ra sur un composé de formule (I/j) : 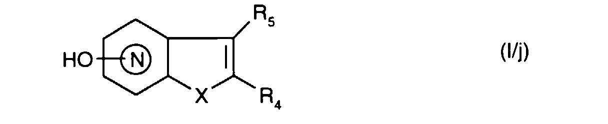 dans laquelle R₄, R₅ et X sont tels que définis précédemment,
composés de formule (I/i) qui sont, le cas échéant
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou un base pharmaceutiquement acceptable.

Le greffage du radical Ra est par exemple réalisé en utilisant un composé de formule (VII) :

Ra-W (VII)

dans laquelle Ra est tel que défini précédemment et W représente un halogène ou un groupement partant.

Les composés de formule (I/j) : 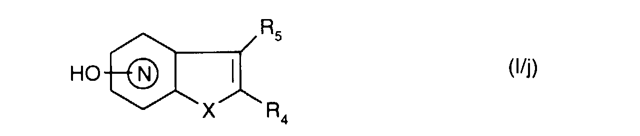 dans laquelle R₄, R₅ et X sont tels que définis dans la formule (I) peuvent être obtenus par décarboxylation du composé alkoxylé correspondant.

La décarboxylation mentionnée ci-dessus peut-être réalisée par exemple par action du BBr₃ ou d'un mélange AIX"₃, R_{b} SH dans lequel X" est un halogène et R_{b} est un alkyl.

L'invention concerne également le procédé de préparation des composés de formule (I/k), cas particulier des composés de formule (I) : 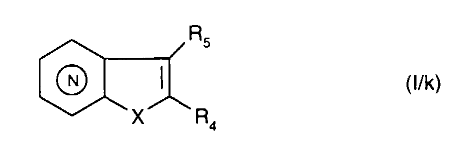 dans laquelle R₄, R₅ et X sont tels que définis dans la formule (I), par traitement acide d'un composé de formule (I/ℓ), cas particulier des composés de formule (I) : 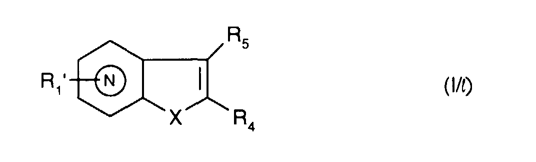 dans laquelle R₄, R₅ et X sont tels que définis précédemment et R_{1'} est un atome d'halogène,
composés de formule (I/k) qui sont, le cas échéant
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou un base pharmaceutiquement acceptable.

L'invention concerne également le procédé de préparation des composés de formule (I/k"), cas particulier des composés de formule (I) : 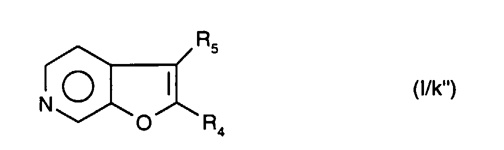 dans laquelle R₄ et R₅ sont tels que définis dans la formule (I), par traitement acide d'un composé de formule (I/ℓ"), cas particulier des composés de formule (I) : 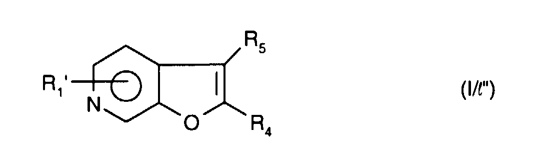 dans laquelle R₄ et R₅ sont tels que définis précédemment et R₁' est un atome d'halogène,
composés de formule (I/k") qui sont, le cas échéant
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

Les matières premières utilisées dans les procédés décrits ci-dessus sont soit commerciales, soit accessibles à l'homme du métier d'après la littérature et d'après les exemples de préparation données ci-après.

Les composés de formule (Il) sont accessibles, par exemple, à l'homme du métier par réduction d'un composé nitré de formule (Il/a) : 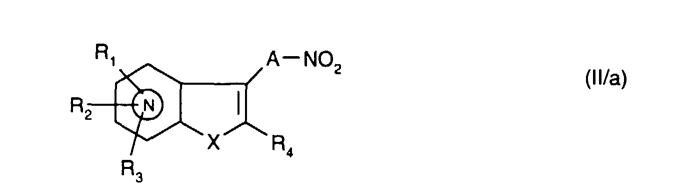 dans laquelle R₁, R₂, R₃, R₄, A et X sont tels que définis dans la formule (I)
ou par hydrogénation d'un composé de formule (Il/b) : 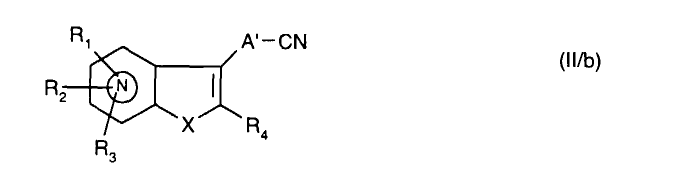 dans laquelle R₁, R₂, R₃, R₄ et X sont tels que définis dans la formule (I) et A' représente une chaîne (C₁ -C₅)alkylène non substituée ou substituée par un ou plusieurs alkyls.

Les composés de formule (II') sont ainsi accessibles, par exemple, à l'homme du métier par réduction d'un composé nitré de formule (Il/a') : 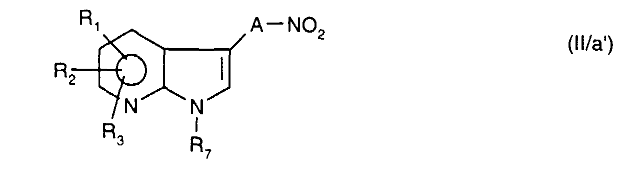 dans laquelle R₁, R₂, R₃, R₇ et A sont tels que définis dans la formule (I)
ou par hydrogénation d'un composé de formule (Il/b') : 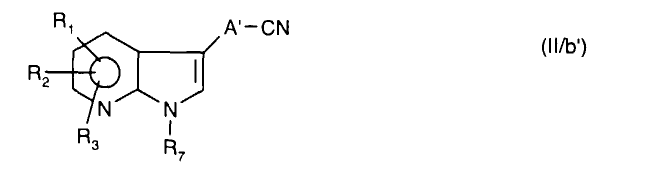 dans laquelle R₁, R₂, R₃ et R₇ sont tels que définis dans la formule (I) et A' représente une chaîne (C₁ -C₅)alkylène non substituée ou substituée par un ou plusieurs alkyls.

Les composés de formule (II") sont accessibles, par exemple, à l'homme du métier par réduction d'un composé nitré de formule (Il/a") : 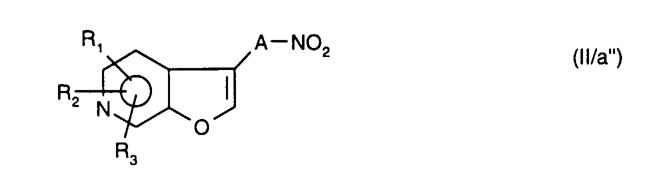 dans laquelle R₁, R₂, R₃ et A sont tels que définis dans la formule (I)
ou par hydrogénation d'un composé de formule (Il/b") : 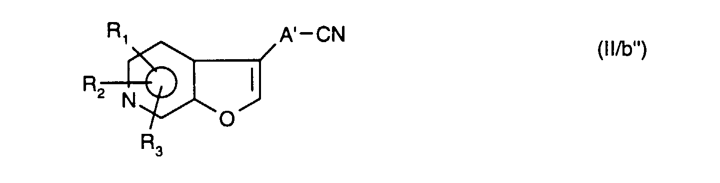 dans laquelle R₁, R₂ et R₃ sont tels que définis dans la formule (I) et A' représente une chaîne (C₁ -C₅) alkylène non substituée ou substituée par un ou plusieurs alkyls.

Les composés de formule (Il/a) sont par exemple aisément accessibles lorsque R₄ est un hydrogène par réduction d'un composé de formule (Il/c) : 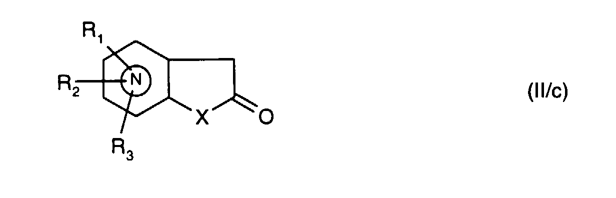 dans laquelle R₁, R₂, R₃ et X sont tels que définis dans la formule (I) pour obtenir un composé de formule (II/d) : 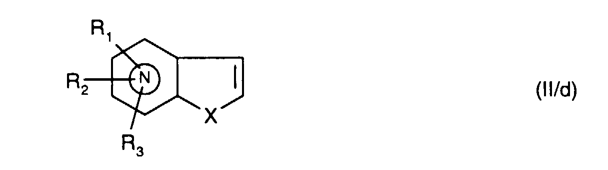 dans laquelle R₁, R₂, R₃ et X sont tels que définis précédemment,
qui est ensuite mis en réaction avec le N,N-diméthyl formamide pour obtenir le composé de formule (Il/e) : 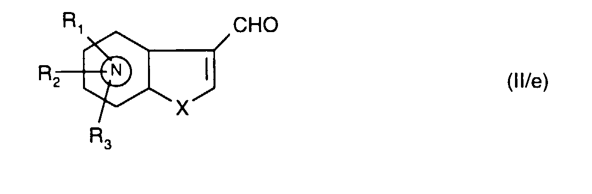 dans laquelle R₁, R₂, R₃, et X sont tels que définis précédemment,
qui est ensuite soumis à l'action du composé nitré de formule (II/f) :

A₂-NO₂ (IV)

dans laquelle A₂ est un radical (C₁ -C₅)alkyl non substitué ou substitué par un ou plusieurs alkyls pour obtenir après une éventuelle hydrogénation un composé de formule (Il/a) tel que défini précédemment.

Les composés de formule (II/a') sont par exemple aisément accessibles par réduction d'un composé de formule (Il/c') : 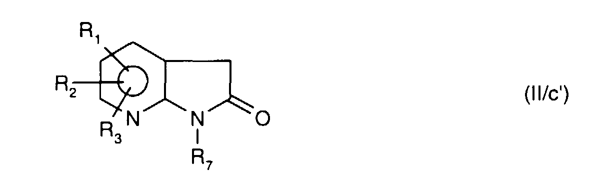 dans laquelle R₁, R₂, R₃ et R₇ sont tels que définis dans la formule (I) pour obtenir un composé de formule (II/d') : 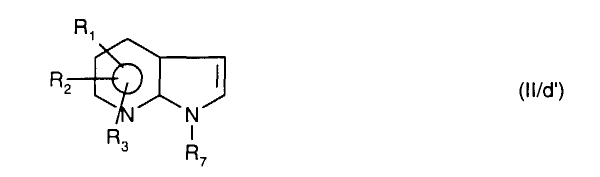 dans laquelle R₁, R₂, R₃ et R₇ sont tels que définis précédemment,
qui est ensuite mis en réaction avec le N,N-diméthylformamide pour obtenir le composé de formule (II/e') : 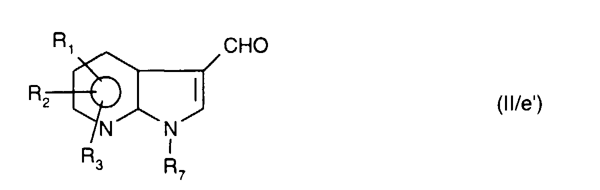 dans laquelle R₁, R₂, R₃ et R₇ sont tels que définis précédemment,
qui est ensuite soumis à l'action du composé nitré de formule (Il/f') :

A₂-NO₂ (II/f')

dans laquelle A₂ est un radical (C₁ -C₅)alkyl non substitué ou substitué par un ou plusieurs alkyls pour obtenir après une éventuelle hydrogénation un composé de formule (Il/a') tel que défini précédemment.

Les composés de formule (II/b), lorsque A représente un éthylène, sont également aisément accessibles à l'homme du métier par cyclisation d'un composé de formule (II/g) : 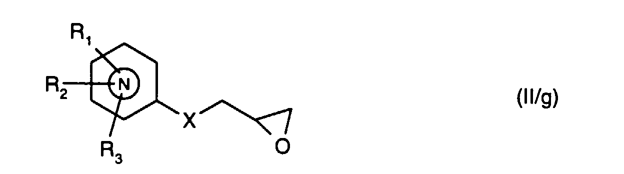 dans laquelle R₁, R₂, R₃ et X sont tels que définis dans la formule (I),
pour obtenir un composé de formule (Il/h) : 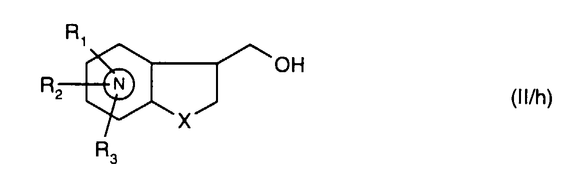 dans laquelle R₁, R₂, R₃ et X sont tels que définis précédemment,
qui est ensuite soumis à l'action du chlorure de tosyle pour obtenir un composé de formule (II/i) : 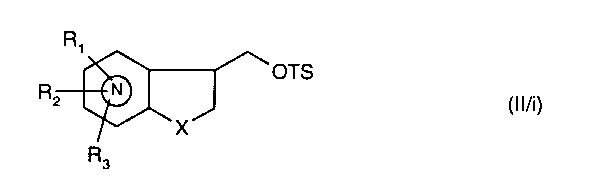 dans laquelle R₁, R₂, R₃ et X sont tels que définis précédemment et Ts représente un groupement tosyle, qui est ensuite mis en réaction avec le 1,4-diazacyclo[2-2-2]octane pour obtenir un composé de formule (II/j) : 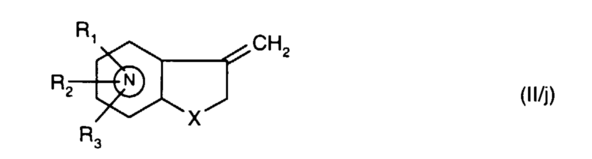 dans laquelle R₁, R₂, R₃ et X sont tels que définis précédemment,
qui est ensuite halogéné pour obtenir un composé de formule (Il/k) : 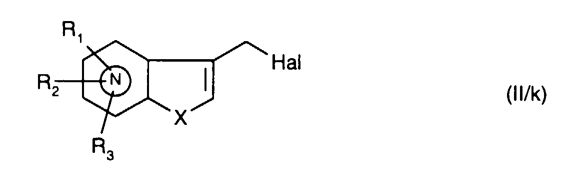 dans laquelle R₁, R₂, R₃ et X sont tels que définis précédemment et Hal représente un atome d'halogène, dont on prépare le dérivé cyano de formule (II/b) correspondant dans lequel A représente une chaîne éthylène : 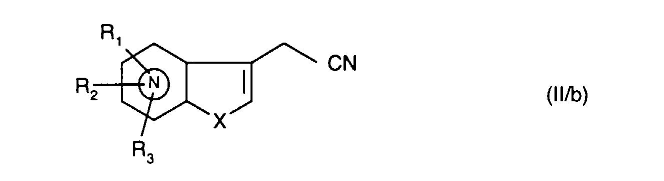 dans laquelle R₁, R₂, R₃ et X sont tels que définis précédemment.

Les composés de formule (Il/b"), lorsque A représente un éthylène, sont par exemple aisément accessibles à l'homme du métier par cyclisation d'un composé de formule (II/g") : 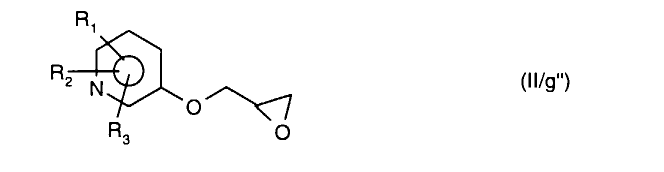 dans laquelle R₁, R₂ et R₃ sont tels que définis dans la formule (I),
pour obtenir un composé de formule (Il/h") : 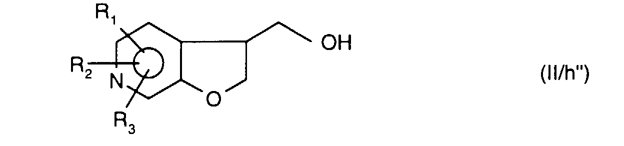 dans laquelle R₁, R₂ et R₃ sont tels que définis précédemment,
qui est ensuite soumis à l'action du chlorure de tosyle pour obtenir un composé de formule (II/i") : 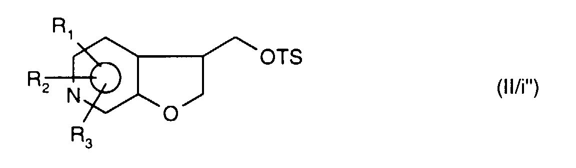 dans laquelle R₁, R₂ et R₃ sont tels que définis précédemment et Ts représente un groupement tosyle, qui est ensuite mis en réaction avec le 1,4-diazacyclo[2-2-2]octane pour obtenir un composé de formule (Il/j") : 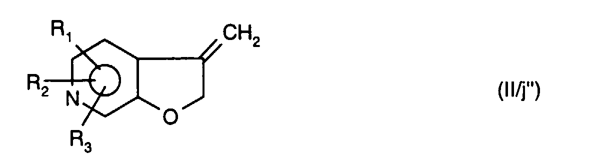 dans laquelle R₁, R₂ et R₃ sont tels que définis précédemment,
qui est ensuite halogéné pour obtenir un composé de formule (Il/k") : 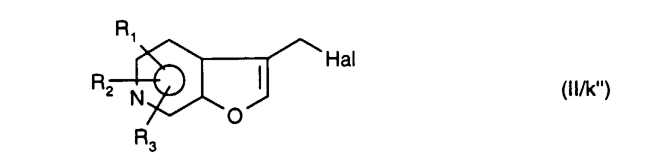 dans laquelle R₁, R₂ et R₃ sont tels que définis précédemment et Hal représente un atome d'halogène,
dont on prépare le dérivé cyano de formule (Il/b") correspondant dans lequel A représente une chaîne éthylène : 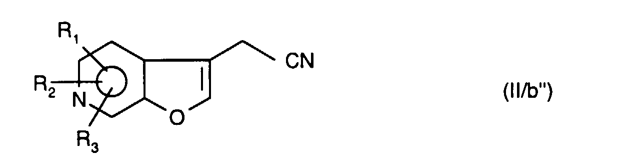 dans laquelle R₁, R₂ et R₃ sont tels que définis précédemment.

Les composés de formule (I) possèdent des propriétés pharmacologiques très intéressantes pour le clinicien.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils n'étaient pas toxiques, doués d'une très haute affinité sélective pour les récepteurs de la mélatonine et possédaient d'importantes activités sur le système nerveux central et en particulier, on a relevé des propriétés thérapeutiques sur les troubles du sommeil, des propriétés anxiolytiques, antipsychotiques, analgésiques ainsi que sur la microcirculation. Les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, du psoriasis, de l'obésité, de l'insomnie, des troubles psychotiques, de l'épilepsie, de la maladie de Parkinson, de la démence sénile, des divers désordres liés au vieillissement normal ou pathologique, de la migraine, des pertes de mémoire, de la maladie d'Alzheimer, ainsi que les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement anticancéreux.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant un composé de formule (I), ou un de ses sels d'addition à un acide ou une base pharmaceutiquement acceptable, en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures, plus particulièrement entre 1 à 100 mg, par exemple entre 1 à 10 mg.

Les exemples suivants illustrent l'invention, mais ne la limitent en aucune façon.

### PREPARATION 1 : 3-(2-AMINOETHYL)-5-METHOXY-1-METHYL-PYRROLO [2,3-b]PYRIDINE

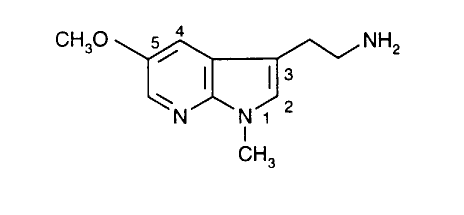

### Stade A : 1-méthyl-pyrrolo[2,3-b]pyridine

La pyrrolo[2,3-b]pyridine (2.00 g, 16.93 mmol) est dissous dans le diméthylformamide (DMF) (15.0cm³), sous atmosphère d'argon. On additionne à O°C sur une période de 30 min l'hydrure de sodium (60 % dans l'huile) (0.96 g, 40.0 mmol, 1.5 eq). Après 30 min d'agitation à 0°C, l'iodométhane (1.49 cm³, 24.02 mmol, 1.5 eq) est additionné goutte à goutte. Après retour à température ambiante, le milieu réactionnel est laissé sous agitation pendant 1 h. Le diméthylformamide est évaporé sous pression réduite, le produit est repris avec de l'eau et extrait avec du dichlorométhane. Une purification sur colonne de silice (Ether de pétrole (EP)) : (Acétate d'éthyle (AcOEt)) (7:3) permet d'isoler le composé du titre avec un rendement de 99 %. Ce dernier se présente sous la forme d'une huile orangée.

### ANALYSE SPECTROSCOPIQUE DANS L'INFRAROUGE (IR)

(film) : ν = 1597 cm⁻¹ (C=C, Ar)

### ANALYSE SPECTROSCOPIQUE DE RMN (RMN¹ H(CDCl₃))

δ (ppm) 3.85 (s, 3H, CH₃), 6.40 (d, 1H, H-3, J₃₋₂ = 3.3 Hz), 7.01 (dd, 1H, H-5, J₅₋₄ = 7.4 Hz, J₅₋₆ = 5.2 Hz), 7.13 (d, 1H, H-2, J₂₋₃ = 3.3 Hz), 7.85 (d, 1H, H-4, J₄₋₅ = 7.4 Hz), 8.29 (d, 1H, H-6, J₆₋₅ = 5.2 Hz).

### Stade B:3,3-dibromo-1-méthyl-2-oxo-2,3-dihydro-pyrrolo[2,3-b]pyridine

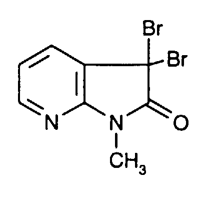

A une solution du composé obtenu au stade précédent (5.59 g, 42.3 mmol) dans le *tert-*butanol (80 cm³) est additioné le perbromate de pyridinium (40.58 g, 127.0 mmol, 3.0 eq). Le milieu est agité à température ambiante pendant 2 h. Les solvants sont concentrés par évaporation sous pression réduite, le produit brut est repris avec de l'eau puis extrait avec de l'acétate d'éthyle. Après évaporation on obtient un solide orangé avec un rendement de 93 %.
Point de fusion (F) : 180°C
IR (KBr) : ν = 1738 cm⁻¹ (C=O),
RMN ¹H (CDCl₃) δ (ppm) : 3.33 (s, 3H, CH₃), 7.11 (dd, 1H, H-5, J₅₋₄ = 7.4 Hz, J₅₋₆ = 5.2 Hz), 7.85 (d, 1H, H-4, J₄₋₅ = 7.4Hz), 8.27 (d, 1H, H-6, J₆₋₅ = 5.2 Hz).

### Stade C : 3,3,5-tribromo-1-méthyl-2-oxo-2,3-dihydro pyrrolo[2,3-b]pyridine

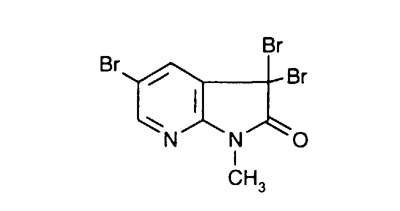

### α) synthèse à partir du composé obtenu au stade B

Le composé obtenu au stade B (12.46 g, 40.7 mmol) est dissous dans le DMF (50 cm³). Après addition goutte à goutte de brome (4.17 cm³, 81.4 mmol, 2 eq), le milieu réactionnel est laissé sous agitation pendant 15 h à température ambiante. Après évaporation du solvant sous pression réduite, le produit est repris avec de l'eau puis extrait au dichlorométhane. Le solvant une fois évaporé, le solide orangé est lavé avec de l'éther de pétrole. Après séchage on obtient le composé du titre avec un rendement de 85 %.

### β) synthèse à partir du composé obtenu au stade A

Le composé obtenu au stade A (2.00 g, 15.1 mmol) est dissous dans le *tert*-butanol (132 cm³). Une quantité équivalente d'eau (132 cm³) est ajoutée lentement. Le brome (9.28 cm³, 81.2 mmol, 12.0 eq) est additionné goutte à goutte à l'aide d'une ampoule à brome. Après 24 h d'agitation à température ambiante, le *tert*-butanol est éliminé par évaporation sous pression réduite. Le mélange est repris avec une solution de NaHCO₃ jusqu'à pH neutre, puis filtré. Après séchage on obtient le composé du titre avec un rendement de 91 %.
F = 210°C
IR (KBr) : ν = 1747 cm⁻¹ (C=O)
RMN 1H (CDCl₃) δ (ppm) : 3.33 (s, 3H, CH₃), 7.95 (d, 1H, H-4, J₄₋₆ = 2.2 Hz), 8.31 (d, 1H, H-6, J₆₋₄ = 2.2 Hz).

### Stade D : 5-bromo-1-méthyl.2-oxo-2,3-dihydro-pyrrolo[2,3-b]pyridine

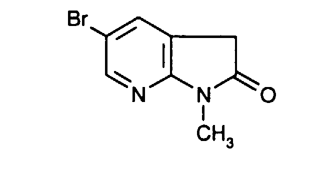

Le composé obtenu au stade précédent (0.327 g, 0.85 mmol) est dissous dans l'acide acétique (8 cm³). On additionne à température ambiante et sous argon du zinc (4.3 g, 8.5 mmol, 10 eq). Après 30 min d'agitation à la même température, le milieu réactionnel est filtré puis évaporé sous pression réduite. Le produit brut est extrait avec de l'acétate d'éthyle à pH neutre puis purifié sur colonne de silice (EP : AcOEt, 7 : 3). Le composé du titre est obtenu avec un rendement de 98 % sous la forme d'un solide orangé.
F = 149°C
IR (KBr) : ν = 1713 cm⁻¹ (C=O).
RMN 1H (CDCI₃) δ = 3.28 (s, 3H, CH₃), 3.55 (s, 2H, CH₂), 7.59 (s, 1H, H-4), 8.25 (s, 1H, H-6).

### Stade E:5-bromo-1-méthyl-pyrrolo[2,3-b]pyridine

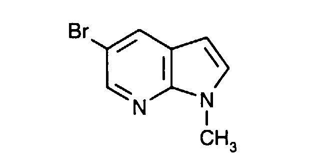

A 2 g (8.81 mmol) du composé obtenu au stade précédent en solution dans 40 cm³ de tétrahydrofurane (THF) anhydre, sont additionnés goutte à goutte 26.4 cm³ (52.85 mmol - 6 eq) de complexe borane diméthylsulfure en solution dans le THF (2M). Après 2 h à reflux, le solvant est évaporé et le brut réactionnel est repris par 20 cm³ de méthanol et 30 cm³ d'HCI 2N. Après 30 min à reflux, le méthanol est évaporé puis le milieu est neutralisé par une solution saturée de bicarbonate de soude. Le produit est extrait au dichlorométhane. La phase organique est séchée sur sulfate de magnésium puis concentrée. Le brut réactionnel est directement engagé dans l'étape d'oxydation.

4.72 g (17.61 mmol) de dihydrate d'acétate de manganèse (III) sont mis en suspension dans 77 cm³ d'acide acétique glacial. A cette suspension est additionné le brut réactionnel précédent en solution dans 81 cm³ d'acide acétique glacial. Après 1 h à 75°C, le solvant est évaporé. Le milieu est hydrolysé, neutralisé avec une solution saturée de bicarbonate de soude puis extrait à l'acétate d'éthyle, séché sur sulfate de magnésium et concentré. Après purification sur colonne de gel de silice (éluant : CH₂Cl₂), le produit du titre est obtenu sous forme d'un solide jaune pâle (1.38 g).
Rendement : 74 %
F = 48°C
IR (KBr) : ν = 3015 cm⁻¹ (C=C), ν = 1575 cm⁻¹ (C=C)
RMN ¹H (CDCl₃ : δ 3.87 (s, 3H, CH₃), 6.39 (d, 1H, J₂₋₃ = 3,3 Hz, H-3), 7.18 (d, 1H, J₃₋₂ = 3.3 Hz, H-2), 8.01 (d, 1H, J₄₋₆ = 2.2 Hz, H-4), 8.34 (d, 1H, J₆₋₄ = 2.2 Hz, H-6).

### Stade F : 1-méthyl-5-méthoxy-pyrrolo[2,3-b]pyridine

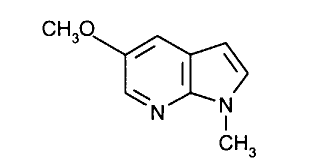

A 1.37 g (6.49 mmol) du composé obtenu au stade précédent en solution dans 23.6 cm³ de méthanol et 36.5 cm³ de DMF anhydre, sont additionnés 18.6 g (344.02 mmol) de méthylate de sodium et 1.86 g (12.98 mmol) de bromure de cuivre. Après 1 h à reflux, les solvants sont éliminés sous vide et le brut réactionnel est hydrolysé par 50 cm³ d'eau puis neutralisé par une solution d'acide chlorhydrique (6N). Le produit est extrait au dichlorométhane. La phase organique est séchée sur sulfate de magnésium puis concentrée. Après purification sur colonne de silice (éluant : AcOEt : EP, 1 : 1), on obtient 930 mg de produit attendu sous forme d'un liquide jaune.
Rendement : 88 %
IR (NaCI) : : ν = 3100 cm⁻¹ (C=C), ν = 1590 cm⁻¹ (C=C)
RMN ¹H (CDCl₃) : δ 3.86 (s, 3H, CH₃), 3.88 (s, 3H, CH₃), 6.36 (d, 1H, J₃₋₂ = 3.3 Hz, H-3), 7.15 (d, 1H, J₂₋₃ = 3.3 Hz, H-2), 7.41 (d, 1H, J₄₋₆ = 2.7 Hz, H-4), 8.12 (d, 1H, J₆₋₄ = 2.7 Hz, H-6).

### Stade G : 3-formyl-5-méthoxy-1-méthyl-pyrrolo[2,3-b]pyridine

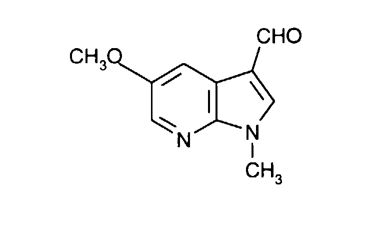

A 20.6 cm³ de DMF refroidis à 0°C, sont additionnés lentement sans augmentation de température 1.09 cm³ (1.80 g - 11.73 mmol) d'oxychlorure de phosphore puis toujours goutte à goutte 1.73 g (10.67 mmol) du composé obtenu au stade précédent en solution dans 17 cm³ de DMF. Après 15 min à 80°C, le solvant est évaporé, le brut réactionnel est hydrolysé à l'eau, neutralisé avec une solution de soude (50 %) puis extrait à l'acétate d'éthyle. La phase organique est séchée sur sulfate de magnésium puis concentrée. Après purification sur colonne de gel de silice (éluant : AcOEt) puis recristallisation dans un mélange cyclohexane/isopropanol (60 cm³ / 5 cm³), l'aldéhyde du titre est obtenu sous forme d'un solide blanc.
Rendement : 71 %
F = 105 - 106°C
IR (KBr) : ν = 3100 cm⁻¹ (C=C), ν = 1660 cm⁻¹ (C=O)
RMN ¹H (CDCI₃) : δ 3.92 (s, 3H, CH₃), 3.95 (s, 3H, CH₃), 7.79 (s, 1H, H-2), 8.06 (d, 1H, J₄₋₆ = 2.9 Hz, H-4), 8.19 (d, 1H, J₆₋₄ = 2.9 Hz, H-6), 9.95 (s, 1H, CHO).

### Stade H : 5-méthoxy-1-méthyl-3-(2-nitrovinyl)-pyrrolo[2,3-b]pyridine

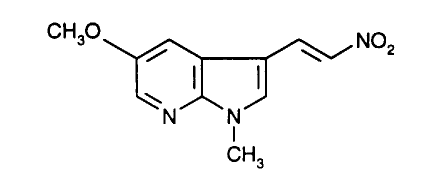

1.44 g (7.55 mmol) du composé obtenu au stade précédent sont mis en solution dans 36 cm³ de nitrométhane. 1.16 g (18.93 mmol) d'acétate d'ammonium sec y sont additionnés puis le mélange est maintenu à 120°C pendant 3 h 30. Après refroidissement total, le produit est précipité dans le milieu et est alors filtré sur verre fritté, lavé à l'éther plusieurs fois. On obtient 1.47 g d'un solide jaune.
Rendement : 83 %
F = 185°C
IR (KBr) : ν = 1610 cm⁻¹ (NO₂)
RMN ¹H (CDCI₃) : δ 3.93 (s, 3H, CH₃), 3.95 (s, 3H, CH₃), 7.49 (d, 1H, J₄₋₆ = 2.6 Hz, H-4), 7.63 (s, 1H, H-2), 7.65 (d, 1H, J = 14.2 Hz, CH), 8.20 (d, 1H, J = 14.2 Hz, CH), 8.21 (d, 1H, J₆₋₄ = 2.6 Hz, H-6).

### Stade I: 5-méthoxy-1-méthyl-3(2-nitroéthyl)pyrrolo[2,3-b]pyridine

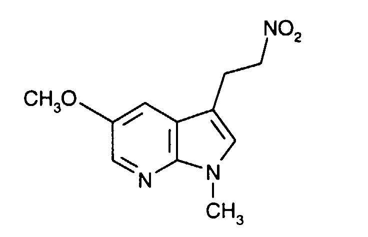

A 100 mg (4.29 10⁻⁴ mmol) du composé obtenu au stade précédent en solution dans 7.8 cm³ de chloroforme et 2.7 cm³ d'isopropanol, sont additionnés 215 mg de silice (230 - 400 mesh) puis par portions 40 mg (1.07 mmol) de borohydrure de sodium. Après 30 min à température ambiante, 40 mg (1.07 mmol) de borohydrure de sodium sont à nouveau additionnés. Après 30 min, le mélange est filtré sur célite. Le filtrat est concentré puis purifié sur colonne de gel de silice (éluant: AcOEt : EP, 1 : 1). On obtient 65 mg de produit attendu sous forme d'un solide jaune pâle.
Rendement : 64 %
F = 59 - 60°C
RMN ¹H (CDCl₃) : δ 3.43 (t, 2H, J = 7.1 Hz, CH₂), 3.82 (s, 3H, CH₃), 3.90 (s, 3H, CH₃), 4.63 (t, 2H, J = 7.1 Hz, CH₂), 7.02 (s, 1H, H-2), 7.32 (d, 1H, J₄₋₆ = 2.9 Hz, H-4), 8.13 (d, 1H, J₆₋₄ = 2.9 Hz, H-6).

### Stade J : 3-(2-aminoéthyl)-5-méthoxy-1-méthyl-pyrrolo[2,3-b]pyridine

### α) synthèse à partir du composé du stade I

50 mg (2.12 10⁻⁴ mmol) du composé obtenu au stade précédent sont dissous dans 1 cm³ de méthanol. Après addition de 10 mg de nickel de Raney, le système est maintenu sous pression d'hydrogène à 60°C pendant 2,5 h. Après refroidissement, le nickel est filtré sur célite, rincé au dichlorométhane. Le filtrat est concentré et on obtient 40 mg d'amine attendue sous forme d'huile. Le produit est engagé dans les étapes suivantes sans purification.
Rendement : 92 %
IR (NaCI) : : ν = 3300 - 3000 cm-1 (NH₂)
RMN ¹H (CDCl₃) : δ 1.56 (s large, 2H, NH₂), 2.84 (t, 2H, J = 6.7 Hz, CH₂), 3.00 (t, 2H, J = 6.7 Hz, CH₂), 3.82 (s, 3H, CH₃), 3.89 (s, 3H, CH₃), 7.00 (s, 1H, H-2), 7.38 (d, 1H, J₄₋₆ = 2.8 Hz, H-4), 8.11 (d, 1H, J₆₋₄ = 2.8 Hz, H-6).

### β) synthèse à partir du composé du stade H

A 90 mg (2.36 mmol) d'hydrure double de lithium et d'aluminium en suspension dans 2 cm³ de tétrahydrofurane anhydre sont additionnés 100 mg (4.29 10⁻⁴ mmol) du composé obtenu au stade H en solution dans 4 cm³ de tétrahydrofurane et 4 cm³ de dioxane. Dès la fin de l'addition la totalité du produit de départ est consommée. L'excès d'hydrure est détruit par addition progressive de 0.09 cm³ d'eau, 0.09 cm³ de solution de soude à 15 % et 0.27 cm³ d'eau. Après 15 min, le précipité est filtré, lavé au dichlorométhane. Le filtrat est concentré et on obtient 70 mg d'amine sous forme d'huile et est engagé dans les étapes suivantes de synthèse sans purification.
Rendement : 79 %

### PREPARATION 2 : 3-(2-AMINOETHYL)-1H-PYRROLO[2,3-b]PYRIDINE

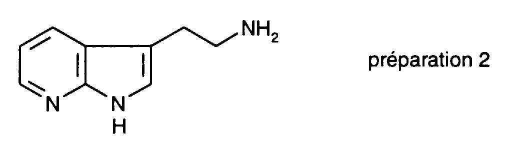

### Stade A : 3-(2-nitrovinyl)-1H-pyrrolo[2,3-b]pyridine

En procédant comme dans la préparation 1 mais en partant du stade H et en utilisant la 3-formyl-1H-pyrrolo[2,3-b]pyridine (Verbiscar A.J., J. *Med. Chem.*, **15**, 1972, 149-152) comme produit de départ.
Rendement : 88 %
F = décomposition > 255°C
IR (KBr) : ν = 3300 - 3000 cm⁻¹ (NH) ; ν = 1615 cm⁻¹ (NO₂)
RMN ¹H (DMSO-d₆) : δ 7.26 (dd, 1H, J_{5,4} = 4.7 Hz, J_{5,6} = 8.1 Hz, H-5), 8.07 (d, 1H, J = 13.5 Hz, CH), 8.37 (d+s, 3H, J = 13.5 Hz, CH + H-6 + H-2), 8.49 (d, 1H, J₅₋₄ = 8.1 Hz, H-4), 12.7 (s large, 1H, NH).

### Stade B : 3-(2-nitroéthyl)-1H-pyrrolo[2,3-b]pyridine

Rendement : 61 %
F = 145°C
IR (KBr) : ν = 3300 - 2500 cm⁻¹ (NH); ν = 1525 cm⁻¹ (NO₂)
RMN ¹H (CDCl₃) : δ 3.49 (t, 1H, J = 7.1 Hz, CH₂), 4.67 (t, 1H, J = 7.1 Hz, CH₂), 7.13 (dd, 1H, J₅₋₄ = 7.8 Hz, J₅₋₆ = 4.6 Hz, H-5), 7.23 (s, 1H, H-2), 7.92 (dd, 1H, J₄₋₆ = 1.3 Hz, J₄₋₅ = 7.8 Hz, H-4), 8.35 (dd, 1H, J₆₋₄ = 1.3 Hz, J₆₋₅ = 4.6 Hz, H-6), 10.25 (s large, 1H, NH).

### Stade C : 3-(2-aminoéthyl)-1H-pyrrolo[2,3-b]pyridine

Rendement : quantitatif
Huile
IR (NaCI) : : ν = 3300 - 3000 cm⁻¹ (NH₂)
RMN ¹H (DMSO-d₆) : δ 1.70 (s large, 2H, NH₂), 2.69 - 2.84 (m, 4H, 2xCH₂), 7.00 (dd, 1H, J₅₋₄ = 7.8 Hz, J₅₋₆ = 4.7 Hz, H-5), 7.21 (s, 1H, H-2), 7.92 (dd, 1H, J₄₋₆ = 1.5 Hz, J₄₋₅ = 7.8 Hz, H-4), 8.16 (dd, 1H, J₆₋₄ = 1.5 Hz, J₆₋₅ = 4.7 Hz, H₆), 11.29 (s large, 1H, NH).

### PREPARATION 3 : 3-(2-AMINOETHYL)-5-METHOXY-1H-PYRROLO[2,3-b]PYRIDINE

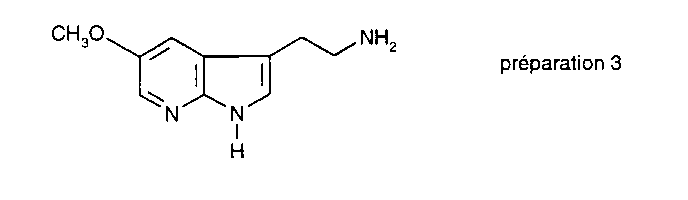

### Stade A: 3,3,5-tribromo-2-oxo-1,3-dihydro-pyrrolo[2,3-b]pyridine

A température ambiante, le brome (54 cm³ ; 1,05 mol) est ajouté goutte à goutte à une solution de 1H-pyrrolo[2,3-*b*]pyridine (10 g ; 0.084 mol) dans le t-butanol (660 cm³) et l'eau (660 cm³). Après 19 h d'agitation, le tefl-butanol est évaporé, la phase aqueuse résiduelle est alcalinisée par une solution aqueuse saturée d'hydrogénocarbonate de sodium. Le produit désiré est récupéré par filtration et après séchage sous vide en présence de pentoxyde de phosphore 26.7 g d'un solide marron, le composé du titre est obtenu.
Rendement : 85 %
F = 157°C
IR (KBr) : ν = 3300 - 3000 cm⁻¹ (NH₂), ν = 1746 cm⁻¹ (C=O)
RMN ¹H (CDCl₃) : δ 7.98 (d, 1H, J₄₋₆ = 2.7 Hz, H-4), 8.33 (d, 1H, J₆₋₄ = 2.7 Hz, H-6), 10.39 (s, 1H, NH)

### Stade B: 5-bromo-2-oxo-1,3-dihydro-pyrrolo[2,3-b]pyridine

Sous argon et à température ambiante, du zinc en poudre (8.8 g ; 135 mmol) est ajouté, portion par portion, à une solution du composé obtenu au stade précédent (5 g ; 13.5 mmol) dans l'acide acétique (100 cm³). Après 3 h de forte agitation, le milieu réactionnel est hydrolysé par l'eau et extrait trois fois par l'acétate d'éthyle. Après séchage sur MgSO₄, la phase organique est évaporée et coévaporée avec du toluène. Le résidu solide est purifié par chromatographie sur gel de silice (éluant : CH₂Cl₂ : MeOH, 95:5), ce qui permet d'obtenir le composé du titre sous forme de solide orangé (2.2 g).
Rendement : 76 %
F = 250°C
IR (KBR) : ν : 3300-3000 cm⁻¹ (NH), ν : 1728 cm⁻¹ (C=O)
RMN ¹H (DMSO-d₆) : δ 4.10 (s, 2H, CH₂), 7.67 (d, 1H, J₄₋₆ = 2.3 Hz, H-4), 8.07 (d, 1H, J₆₋₄ = 2.3 Hz, H-6), 11.06 (s, 1H, NH).

### Stade C: 5-bromo-1H-pyrrolo[2,3-b]pyridine

Sous atmosphère d'argon et en milieu anhydre, une solution 1,0 M de complexe borohydrure-tétrahydrofurane dans le tétrahydrofurane (37,6 cm³ ; 37,6 mmol) est additionnée goutte à goutte au composé obtenu au stade précédent (2 g ; 9,4 mmol) en suspension dans le tétrahydrofurane (50 cm³) à 0°C. La réaction est agitée 35 min à température ambiante et évaporée à sec. Le résidu est repris dans une solution aqueuse d'acide chlorhydrique 6 N et chauffé jusqu'à dissolution complète du solide. Après refroidissement, la solution est alcalinisée par une solution aqueuse de soude 6 N et extraite par l'acétate d'éthyle. La phase organique est séchée sur sulfate de magnésium et évaporée. Le résidu mis en solution dans l'acide acétique (20 cm³) est ajouté à température ambiante à une suspension d'acétate de manganèse (III) dihydraté (4,1 g ; 15.28 mmol) dans l'acide acétique (20 cm³). Après 45 min d'agitation à 75°C, la solution est évaporée à sec et coévaporée avec du toluène ; le résidu, repris dans l'eau, est alcalinisé par une solution aqueuse saturée d'hydrogénocarbonate de sodium. Après extraction par l'acétate d'éthyle (4 fois), les phases organiques sont séchées sur sulfate de magnésium et évaporées. Le résidu obtenu est purifié par chromatographie sur gel de silice (éluant : EP : AcOEt, 6:1) pour donner 920 mg d'un solide légèrement jaune.
Rendement : 50 %
F =176°C
IR (KBr) : ν = 3300-3000 cm⁻¹ (NH)
RMN ¹H (CDCl₃) : δ 6.39 (d, 1 H, J₃₋₂ = 2.9 Hz, H-3), 7.30 (d, 1H, J₂₋₃ = 2.9 Hz, H-2), 8.01 (d, J₄₋₆ = 2.2 Hz, H-4), 8.29 (d, 1H, J₆₋₄ = 2.2 Hz, H-6), 10.9 (s, 1 H, 1 H, NH).

### Stade D: 5-méthoxy-1H-pyrrolo[2,3-b]pyridine

Un mélange du composé obtenu au stade précédent (986 mg ; 5,0 mmol), de méthylate de sodium (14.3 g ; 265 mmol) et de bromure cuivreux (1.43 g ; 10.01 mmol) est mis en suspension dans le diméthylformamide (32 cm³) et le méthanol (20 cm³) puis chauffé à reflux pendant 2 h 30. Après évaporation des solvants, le résidu est repris dans l'eau et extrait par l'acétate d'éthyle ; la phase aqueuse est ramenée à pH neutre par une solution aqueuse d'acide chlorhydrique 2 N et extraite deux nouvelles fois par l'acétate d'éthyle. Les phases organiques sont lavées avec de l'eau, séchées sur MgSO₄ et évaporées pour donner un solide qui est purifié par chromatographie sur gel de silice (éluant : CH₂Cl₂/MeOH : 99:1). Le produit méthoxylé est obtenu sous la forme d'un solide légèrement jaune (530 mg).
Rendement : 72 %
F = 162°C
IR (KBr) : ν = 3300-3000 cm⁻¹ (NH)
RMN ¹H (CDCl₃) : δ 3.83 (s, 3 H, OCH₃), 6.38 (d, 1 H, J₃₋₂ = 2.9 Hz, H-3), 7.28 (d, 1 H, J_{2,3} = 2.9 Hz, H-2), 7.41 (d, 1 H, J₄₋₆ = 2.6 Hz, H-4), 8.06 (d, 1 H, J₆.₄ = 2.6 Hz, H-6), 10.26 (s, 1H, NH).

### Stade E : 3-formyl-5-méthoxy-1H-pyrrolo[2,3-b]pyridine

Sous argon et en milieu anhydre, l'oxychlorure de phosphore (1,5 cm³ ; 15.5 mmol) est ajouté goutte à goutte au diméthylformamide (20 cm³) maintenu à 0°C. Dix minutes après, une solution du composé obtenu au stade précédent (230 mg ; 1.55 mmol) dans le diméthylformamide (5 cm³) y est ajouté à l'aide d'une aiguille de transfert. La réaction est agitée 30 min à 0°C avant d'être chauffée progressivement jusqu'a 80°C. Après 2 h d'agitation, le mélange réactionnel est évaporé à sec et repris dans l'eau, la phase aqueuse est alcalinisée par de la soude aqueuse à 50 % et extraite par de l'acétate d'éthyle. Une fois les phases organiques lavées par l'eau, séchées sur sulfate de magnésium et évaporées, le résidu est purifié par chromatographie sur gel de silice (éluant : EP-AcOEt, 2 : 1, puis 1:1 puis 1 : 2). Le composé du titre est obtenu sous forme de solide jaune (113 mg).
Rendement : 41 %
F = dégradation au-dessus de 191°C
IR (KBr) : ν = 3300-3000 cm⁻¹ (NH), 1657 cm⁻¹ (C=O)
RMN ¹H (DMSO-d₆) : δ 3.79 (s, 3H, OCH₃), 7.85 (d, 1 H, J_{4,6} = 3.0 Hz, H-4), 8.04 (d, 1 H, J₆₋₄ = 3.0 Hz, H-6), 8.32 (s, 1 H, H-2), 9.83 (s, 1 H, CHO), 12.50 (s, 1 H, NH).

### Stade F : 5-méthoxy-3(2-nitrovinyl)-1H-pyrrolo[2,3-b]pyridine

Le composé obtenu au stade précédent (260 mg ; 1.48 mmol) dans le nitrométhane (8 cm³) est chauffé à 90°C en présence d'acétate d'ammonium (285 mg ; 3.7 mmol) pendant 3 h. Après évaporation du solvant, le composé nitrovinylique est obtenu sous forme de solide jaune (166 mg).
Rendement : 51 %
F = 231°C
IR (KBr) : ν = 3300 - 3000 cm⁻¹ (NH), ν = 1500 cm⁻¹ (NO₂)
RMN ¹ H (DMSO-d₆) : à 3.91 (s, 3H, OCH₃), 7.97 (d, 1 H, J_{4,6} = 2.7 Hz, H-4), 8.08 (d, 1 H, J_{6,4} = 2.7 Hz, H-6), 8.14 (d, 1H, J = 13.4 Hz, CH), 8.30 (s, 1 H, H-2), 8.37 (d, 1 H, J = 13.4 Hz, CH), 12.57 (s, 1 H, NH).

### Stade G : 5-méthoxy-3-(2-nitroéthyl)-1H-pyrrolo[2,3-b]pyridine

Sous argon et en milieu anhydre, le dérivé nitrovinylique obtenu au stade précédent (155 mg ; 0.71 mmol) est mis en suspension dans l'isopropanol (14 cm³) et le chloroforme (4.3 cm³) en présence de silice (355 mg). Le borohydrure de sodium (140 mg ; 3,55 mmol) est ajouté portion par portion. Après 2 h d'agitation à température ambiante, le mélange réactionnel est filtré sur célite, le résidu solide est lavé plusieurs fois avec le chloroforme et l'alcool isopropylique. Le produit obtenu après l'évaporation du solvant est purifié par chromatographie sur gel de silice (éluant : CH₂Cl₂ : MeOH, 99:1) ; le composé du titre est obtenu sous forme de solide blanc (90 mg).
Rendement : 58 %
F = décomposition au-dessus de 125°C
IR (KBr): ν = 3300-3000 cm⁻¹ (NH) ; ν = 1540 cm⁻¹ (NO₂)
RMN ¹H (DMSO-d₆) : δ 3.31 (t, 2 H, J = 7.1 Hz, CH₂), 3.81 (s, 3 H, OCH₃), 4.82 (t, 2 H, J = 7.1 Hz, CH₂), 7.67 (s, 1 H, H-2), 8.05 (d, 1 H, J₄₋₆ = 2.8 Hz, H-4), 8.40 (d, 1 H, J₆₋₄ = 2.8 Hz, H-6), 12.0 (s, 1 H, NH).

### Stade H : 3-(2-aminoéthyl)-5-méthoxy-1H-pyrrolo[2,3-b]pyridine

Sous atmosphère d'hydrogène, le composé obtenu au stade précédent (85 mg ; 0.38 mmol) est fortement agité dans le méthanol (2 cm³) à 60°C en présence de nickel de Raney pendant 22 h. Le milieu réactionnel est filtré sur célite ; le résidu solide est lavé par le méthanol. Après évaporation du solvant, le résidu (72 mg ; 0.38 mmol) est mis en suspension dans le dichlorométhane (1 cm³) sous argon.

### PREPARATION 4 : 7-CHLORO-3-CYANOMETHYL-FURO[2,3-c]PYRIDINE

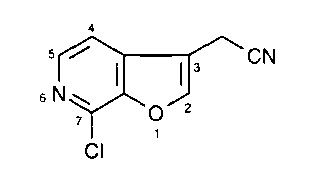

### Stade A : 2-chloro-3-(oxiran-2-yl-méthyloxy)pyridine:

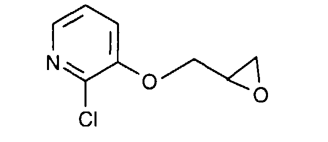

15 g (132 mmol) de 2-chloro-3-pyridinol sont dissous dans 100 cm³ de DMF puis on ajoute à O°C, spatule par spatule, 6,34 g (158,6 mmol) d'hydrure de sodium à 60 % (préalablement lavé). Le mélange est maintenu sous agitation pendant 45 min à température ambiante sous argon. On additionne ensuite 103.5 cm³ (1.32 mmol) d'épichlorhydrine en solution dans 25 cm³ de DMF. L'agitation est maintenue 3 h à 60 °C. Après retour à température ambiante, le DMF est évaporé, le mélange est hydrolysé avec 200 cm³ d'eau puis extrait au CH₂Cl₂. La phase organique est séchée sur MgSO₄ puis évaporée. L'époxyde est purifié par chromatographie sur colonne de silice (éluant : AcOEt : EP, 7:3). On récupère 20.1 g de produit pur, sous forme d'un solide blanc.
Rendement : 81 %
F = 34-36°C
IR (KBr) : ν = 3030 cm⁻¹ (CH₂ époxyde), ν = 1280 et 1200 cm⁻¹ (C-O-C)
RMN ¹H (CDCl₃) : δ 2.78 (dd, 1H, J = 2.9 Hz, J = 4.6 Hz, Ar-O-CH₂-CH-CH₂) ; 2.88 (t, 1H, J = 4.6 Hz, Ar-O-CH₂-CH-CH₂), 3.32 - 3.36 (m, 1H, CH-CH₂), 4.00 (dd, 1H, J = 5.9 Hz, J = 11.4 Hz, Ar-O-CH₂-CH), 4.32 (dd, 1H, J = 2.9 Hz, J = 11.4 Hz, Ar-O-CH₂-CH), 7.14 (dd, 1H, J = 4.4 Hz, J = 8.1 Hz, H_{pyr}), 7.23 (d, 1H, J = 8.1 Hz, Hpyr), 7.97 (d, 1H, J = 4.4 Hz, H_{pyr}).

### Stade B : 7-chloro-3-hydroxyméthyl-2,3-dihydrofuro[2,3-c]pyridine

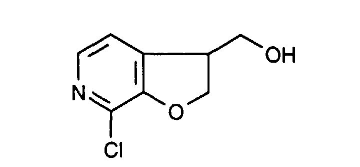

1 g (5.39 mmol) du composé obtenu au stade précédent sont dissous dans 10 cm³ de tétrahydrofurane puis on additionne à -78°C pendant 15 min 5.4 cm³ (10.8 mmol) de diisopropylamidure de lithium (2M) en solution dans 8 cm³ de THF. Après 5 min d'agitation, on hydrolyse avec 20 cm³ d'eau à - 78°C, puis on laisse remonter jusqu'à température ambiante. Le THF est évaporé. Après extraction avec CH₂Cl₂, la phase organique est séchée sur MgSO4. L'évaporation suivie d'une chromatographie sur colonne de silice (éluant : AcOEt:EP : 3:1) conduit à 0.7 g du composé du titre sous la forme d'un solide blanc.
Rendement : 70 %
F =110-112°C
IR (KBr) : ν= 3500 - 3000 cm⁻¹ (OH) ; ν = 1215 cm-1 (C-O-C)
RMN ¹H (CDCl₃) : δ 1.96 (t, 1H, J = 4.1 Hz, OH), 3.72 - 3.81 (m, 1H, CH), 3.82 - 3.86 (m, 2H, CH₂-OH), 4.60 (dd, 1H, J = 5.9 Hz, J = 8.8 Hz, O-CH₂), 4.78 (t, 1H, J = 8.8 Hz, O-CH₂), 7.17 (d, 1H, J = 5.1 Hz, H_{pyr}), 7.93 (d, 1H, J = 5.1 Hz, H_{pyr}).
MS *m/z* 186 (M+1).

### Stade C : 7-chloro-3-tosyloxyméthyl-2,3-dihydrofuro[2,3-c]pyridine

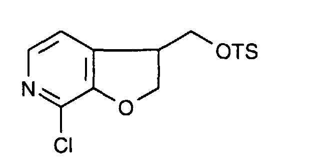

Une solution de 1.54 g (8.08 mmol) de chlorure de tosyle, dissous dans 15 cm³ de CH₂Cl₂ anhydre, est additionné, à 0°C, à un mélange de 1 g (5.39 mmol) du composé obtenu au stade précédent dissous dans 25 cm³ de CH₂Cl₂ et 2.25 cm³ (16.2 mmol) de triéthylamine. L'agitation est maintenue à température ambiante pendant 24 h. Après évaporation sous vide, le produit brut est chromatographié sur colonne de silice (éluant : EP : AcOEt, 3:1). On isole ainsi 1.72 g de produit pur sous forme d'un solide blanc.
Rendement : 94 %
F = 139 - 140°C
IR (KBr) : ν = 1634 et 1171 cm⁻¹ (O-SO₂)
RMN ¹H (CDCl₃) : δ 2.45 (s, 3H, CH₃), 3.88 - 3.97 (m, 1H, CH), 4.09 - 4.21 (m, 2H, CH₂ OTS), 4.44 (dd, 1H, J = 9.9 Hz, J = 6.0 Hz, O-CH₂), 4.71 (t, 1H, J = 9.9 Hz, O-CH₂), 7.06 (d, 1H, J = 4.9 Hz, H_{pyr}), 7.34 (d, 2H, J = 8.2 Hz, Hₐᵣₒₘ), 7.71 (d, 2H, J = 8.2 Hz, Hₐᵣₒₘ), 7.75 (d, 1H, J = 4.9 Hz, H_{pyr}).
MS *m/z* 340(M+1)

### Stade D : 7-chloro-3-méthylène-2,3-dihydrofuro[2,3-c]pyridine

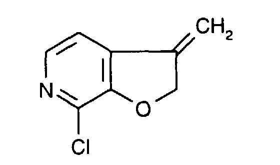

3 g (8.84 mmol) du composé obtenu au stade précédent sont dissous dans 30 cm³ d'acétonitrile puis 1.24 g (10.60 mmol) de 1.4-diazabicyclo[2.2.2]octane sont additionnés à température ambiante. L'agitation est maintenue 4 h à 80°C sous atmosphère inerte. Après évaporation sous vide, le produit brut est chromatographié sur colonne de silice (éluant : EP/AcOEt:1/1) pour donner 1.28 g de produit pur sous forme d'un solide blanc.
Rendement : 87 %
F = 98 -100°C
IR (KBr) : ν = 1640 cm⁻¹ (C=CH₂)
RMN ¹H (CDCl₃) : δ 5.24 (t, 2H, J = 2.9 Hz, O-CH₂), 5.31 (m, 1H, = CH₂), 5.67 (m, 1H, CH₂), 7.24 (d, 1H, J = 4.4 Hz, H_{pyr}), 7.99 (d, 1H, J = 4.4 Hz, H_{pyr}).

### Stade E : 3-bromométhyl-7-chloro-furo[2,3-c]pyridine

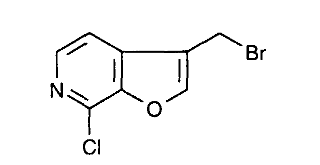

A 1 g (5.97 mmol) du composé obtenu au stade précédent dissous dans 30 cm³ de CCl₄, on additionne une spatule de 2,2'-azobisisoobutyronitrile et 6.56 g (1.08 mmol) de N-bromosuccinimide. Le milieu réactionnel est chauffé à reflux à l'aide d'une ampoule de 75 W, sous atmosphère inerte, pendant 4 h. Après retour à température ambiante, le solvant est évaporé. On hydrolyse avec 50 cm³ d'eau puis on extrait au CH₂Cl₂. La phase organique est séchée sur MgSO₄ puis évaporée. Le produit brut est purifié par chromatographie sur colonne de silice (éluant : EP : AcOEt, 8 : 2). On récupère 1.08 g de composé du titre pur sous forme d'un solide blanc.
Rendement : 74 %
F =105°C
RMN ¹H (CDCl₃) : δ 4.51 (s, 2H, CH₂), 7.55 (d, 1H, J = 5.4 Hz, H_{pyr}), 7.80 (s, 1H, CH), 8.28 (d, 1H, J = 5.4 Hz, H_{pyr}).

### Stade F : 7-chloro-3-cyanométhyl-furo[2,3-c]pyridine

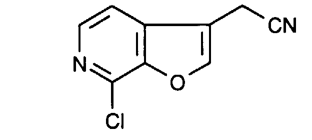

A 680 mg (2.77 mmol) du composé obtenu au stade précédent dissous dans 15 cm³ de DMF, on additionne, à température ambiante, 270 mg (4.15 mmol) de KCN. L'agitation est maintenue pendant 5 heures. Le DMF est évaporé sous vide puis le produit brut est purifié par chromatographie sur colonne de silice (éluant : EP : AcOEt, 6:4). On récupère 477 mg du composé du titre sous forme d'un solide blanc.
Rendement : 90 %
F = 125°C
IR (KBr) : ν = 2230 cm⁻¹ (CN)
RMN ¹H (CDCl₃) : δ 3.69 (s, 2H, CH₂) ; 7.44 (d, 1H, J = 5.2 Hz, H_{pyr}) ; 7.76 (s, 1H, CH) ; 8.20 (d, 1H, J = 5.2 Hz, H_{pyr}).

### PREPARATION 5 : 3-(2-AMINOETHYL)-4-METHYL-1-PHENYL-PYRROLO[2,3-c]PYRIDINE

### PREPARATION 6 : 3-(2-AMINOETHYL)-1H-PYRROLO[3,2-b]PYRIDINE

### PREPARATION 7 : 3-(2-AMINOETHYL)-1,4-DIMETHYL-PYRROLO[2,3-b] PYRIDINE

### PREPARATION 8 : 3-(2-AMINOETHYL)-1,2-DIMETHYL-PYRROLO[2,3-b]PYRIDINE

### PREPARATION 9 : 3-(2-AMINOBUTYL)-1-PHENYL-PYRROLO[2,3-b]PYRIDINE

### PREPARATION 10 : 3-(2-AMINOETHYL)-5-METHOXY-1-METHYL-2-PHENYL-PYRROLO[2,3-b]PYRIDINE PREPARATION 11 : 3-(2-AMINOETHYL)-2-PHENYL-PYRROLO[2,3-b]PYRIDINE

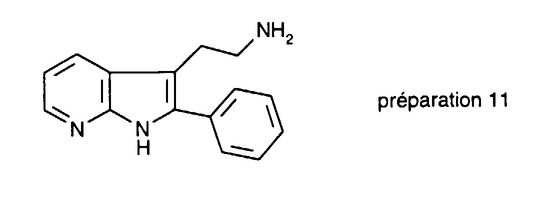

### Stade A: 1-benzènesulfonylpyrrolo[2,3-b]pyridine

Sous argon et en milieu anhydre, de la soude pilée (5,3 g ; 132 mmol) est mise en suspension dans le dichlorométhane (55 cm³) à 0°C ; sont successivement ajoutés le chlorure de benzyltriéthylammonium (250 mg ; 1,1 mmol), le 7-azaindole (5 g ; 42,3 mmol) et goutte à goutte le chlorure de benzènesulfonyle (6,8 cm³ ; 52,9 mmol) dans le dichlorométhane (17 cm³). Après 15 min d'agitation à 0°C puis 2 h à température ambiante, le milieu est filtré sur verre fritté ; le solide est lavé au dichlorométhane. Après évaporation du solvant, le résidu est chromatographié sur gel de silice (éluant : éther de pétrole : acétate d'éthyle, 3 : 1) pour donner 10,7 g de solide blanc.
Rendement : 98 %
F = 134°C
IR (KBr) : ν = 1370 cm⁻¹ (SO₂), ν = 1175 cm⁻¹ (SO₂)
RMN ¹H (CDCl₃) : δ 6.45 (d, 1H, J_{3,2} = 2.8 Hz, H-3), 7.48-7.63 (massif, 5H aromatiques, H-2, H-4), 7.11 (dd, 1H, J₅₋₆ = 4.4 Hz, J₅₋₄ = 8.0 Hz, H-5), 7.76 (d, 1H, J = 7.4 Hz, aromatique), 8.15 (d, 1H, J = 7.4 Hz, aromatique), 8.32 (d, 1H, J₆₋₅ = 4.4 Hz, H-6).

### Stade B : 1-benzènesulfonyl-2-triméthylétainpyrrolo[2,3-b]pyridine

Sous argon et en milieu anhydre, le composé obtenu au stade précédent (5 g ; 19,4 mmol) est dissous dans le tétrahydrofuranne (100 cm³) en présence de N,N,N'N'-tétraméthyléthylènediamine (2,9 cm³ ; 19,4 mmol) ; une solution de diisopropylamidure de lithium 2 M est ajoutée à - 22°C au mélange précédent. Après 30 min d'agitation à - 22°C, le chlorure de triméthylétain (7,7 g ; 38,7 mmol) dans le tétrahydrofuranne (40 cm³) est ajouté à l'anion ; la réaction est agitée 30 min avant d'ajouter de l'eau. Après extraction au dichlorométhane (3 fois), la phase organique est séchée sur sulfate de magnésium et évaporée pou donner un solide qui est purifié sur gel de silice (éluant : éther de pétrole : acétate d'éthyle, 8:1) ; 6,15 g de solide blanc sont obtenus.
Rendement : 76 %
F = 139-140°C
IR (KBr) : ν = 1364 cm⁻¹ (SO₂), ν = 1167 cm⁻¹ (SO₂)
RMN ¹H (CDCI₃) : δ 0.49 (s, 9H, Sn(CH₃)₃), 6.73 (s, 1H, H-3), 7.10 (dd, 1H, J₅₋₆ = 4.4 Hz, J₅₋₄ = 8.1 Hz, H-5), 7.42-7.56 (m, 4H, 3 aromatiques, H-4), 7.75, (d, 1H, J = 7.35 Hz, aromatique), 8.12 (d, 1H, J = 7.35 Hz, aromatique), 8.31 (d, 1H, J₆₋₅ = 4.4 Hz, H-6).

### Stade C : 1-benzènesulfonyl-2-phénylpyrrolo[2,3-b]pyridine

Sous argon et en milieu sec, le mélange de composé obtenu au stade précédent (6,44 g ; 15,3 mmol), de iodobenzène (2 cm³ ; 18,36 mmol), de chlorure de benzyltriéthylammonium (3,5 g ; 15,3 mmol) et de chlorure de bis(triphénylphosphine)palladium (Il) (540 mg, 0,76 mmol) dans l'acétonitrile (180 cm³) est chauffé à reflux pendant 44 h ; après une nouvelle addition de chlorure de bis-(triphénylphosphine)palladium (Il) (540 mg ; 0,76 mmol), le mélange est chauffé à reflux pendant 20 h. Le mélange réactionnel est évaporé à sec ; la purification du résidu sur gel de silice (éluant : éther de pétrole : acétate d'éthyle, 5 : 1) permet d'obtenir 2,7 g de solide marron.
Rendement : 53 %
F = 66°C
IR (KBr) : ν = 1399 cm⁻¹ (SO2), ν = 1187 cm⁻¹ (SO₂)
RMN ¹H (CDCl₃) : δ 6.50 (s, 1H, H-3), 7.16-7.56 (m, 10 H, aromatiques), 7.77 (dd, 1H, J = 1,5 Hz, J₅₋₄= 8.1 Hz, H-5), 7.87 (d, 1H, J₄₋₅ = 8.1 Hz, H-4), 8.47 (d, 1H, J = 6.4 Hz, H-6)

### Stade D : 3-formyl-2-phényl-1H-pyrrolo[2,3-b]pyridine

Sous argon et en milieu anhydre, de l'oxychlorure de phosphore (0,550 cm³ ; 5.86 mmol) est ajouté goutte à goutte à 0°C au DMF (10 cm³) ; à ce mélange agité 10 min à 0°C, le composé obtenu au stade précédent (1.31 g ; 3,9 mmol) dans le DMF (30 cm³) est additionné. Le milieu est agité 30 mn à 0°C, puis chauffé à 80°C pendant 14 h ; le mélange réactionnel est évaporé à sec en présence de toluène. Le résidu est repris dans l'eau et amené à pH basique par de la soude à 50 % ; la phase aqueuse est extraite par l'acétate d'éthyle (4 fois). Les résidus insolubles dans l'eau et l'acétate d'éthyle sont dissous dans l'acide chlorhydrique 6 M en chauffant ; la solution acide est alcalinisée par de la soude à 50 % et extraite par l'acétate d'éthyle (3 fois). Les phases organiques cumulées sont séchées sur sulfate de magnésium et évaporées pour donner un solide qui est purifié par chromatographie sur gel de silice (éluant : dichlorométhane : méthanol, 99 :1).
Rendement : 76 %
F = supérieur à 250°C
IR (KBr) : ν = 3447 cm⁻¹ (NH), ν = 1667 cm⁻¹ (C=O)
RMN ¹H (CDCl₃) : δ 7.30 (dd, 1H, J₅₋₆ = 5.2 Hz, J₅₋₄ = 7.7 Hz, H-5), 7.58-7.62 (m, 3H, aromatiques), 7.80-7.83 (m, 2H, aromatiques), 8.38 (d, 1H, J₆₋₅ = 5.2 Hz, H-6), 8.50 (d, 1H, J = 8.6 Hz, H-4), 9.96 (s, 1H, CHO), 12.94 (s, 1H, H-1).

### Stade E : 3-(2-nitrovinyl-2-phényl-1H-pyrrolo[2,3-b]pyridine

Sous argon et en milieu anhydre, le composé obtenu au stade précédent (675 mg ; 3,0 mmol) est mis en solution dans le nitrométhane (45 cm³) en présence d'acétate d'ammonium (705 mg ; 9,1 mmol). Après 20 h de reflux, la réaction est refroidie à 0°C et filtrée sur verre fritté ; le solide jaune récupéré est lavé par du dichlorométhane et de l'eau. Après séchage, 593 mg de solide jaune sont récupérés.
Rendement : 74 %
F = supérieur à 250°C
IR (KBr) : ν = 3431 cm⁻¹ (NH), ν = 1584 cm⁻¹ (NO₂), ν = 1315 cm⁻¹ (NO₂)
RMN ¹H (DMSO-d₆) : δ 7.29-8.53 (série de massifs, aromatiques et vinyliques, 10 H), 13,10 (s, 1H, H-1).

### Stade F : 3-(2-nitroéthyl)-2-phényl-1H-pyrrolo[2,3-b]pyridine

Sous argon et en milieu anhydre, le composé obtenu au stade précédent (585 mg ; 2,2 mmol) est mis en suspension dans le chloroforme (46 cm³) et l'isopropanol (23 cm³) en présence de silice 230-400 mesh (1,14 g) et de borohydrure de sodium (210 mg, 5,5 mmol). Après 4 h d'agitation à température ambiante, de l'acide acétique est ajouté jusqu'à arrêt de dégagement gazeux ; le mélange est filtré sur célite, le résidu solide étant lavé par le dichlorométhane et le méthanol. Après évaporation des solvants et purification sur gel de silice (éluant : dichlorométhane : méthanol, 99 : 1), 554 mg de solide blanc sont obtenus.
Rendement : 94 %
F = 180°C
IR (KBr) : ν = 3449 cm⁻¹ (NH), ν = 1539 cm⁻¹ (NO₂)
RMN ¹H (DMSO-d₆) : δ 3.52 (t, 2H, J = 7.3 Hz, CH₂), 4.81 (t, 2H, J = 7.3 Hz, CH₂-NO₂), 7.09 (dd, 1H, J = 5.2 Hz, J = 8.6 Hz, H-5), 7.41-7.66 (massif, 5 H aromatiques), 8.07 (d, 1H, J = 8.6 Hz, H-4), 8.23 (d, 1H, J = 5.2 Hz, H-6), 11.92 (s, 1H, H-1).

### Stade G : 3-(2-aminoéthyl)-2-phényl-1H-pyrrolo[2,3-b]pyridine

Sous une pression d'hydrogène de 55 p.s.i., le composé obtenu au stade précédent (50 mg ; 0,19 mmol) est agité dans l'acide acétique (5 cm³) en présence d'oxyde de platine en quantité catalytique. Après 21 h d'agitation à température ambiante, filtration sur célite et évaporation des solvants, une huile est obtenue qui est engagée dans l'acétylation directement.

### PREPARATION 12 : 3-(2-AMINOETHYL)-5-BROMO-1H-PYRROLO[2,3-b]PYRIDINE

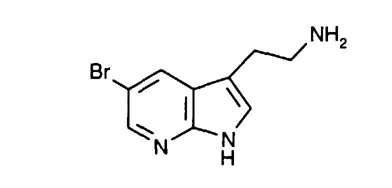

### Stade A : 5-bromo-3-diméthylaminométhyl-1H-pyrrolo[2,3-b]pyridine

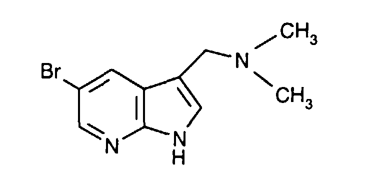

Le mélange de 5-bromo-1H-pyrrolo[2,3-b] pyridine obtenue au stade C de la préparation 3 (1,0 g ; 5,1 mmol), de paraformaldéhyde (330 mg) et de chlorure de diméthylammonium (840 mg) dans le tertiobutanol (30 cm³) est chauffé à reflux pendant 24 h. Du paraformaldéhyde (110 mg) et du chlorure de diméthylammonium (380 cm³) sont de nouveau ajoutés au milieu réactionnel avant de l'agiter à reflux pendant 18 h. La réaction est évaporée à sec ; au résidu repris dans l'eau est ajouté de l'acide chlorhydrique concentré jusqu'à pH = 1. La phase aqueuse est lavée 3 fois par de l'éther diéthylique avant d'être amenée à pH = 10 par de la soude à 50 %. Après extraction de la phase aqueuse par l'acétate d'éthyle (3 fois), la phase organique est séchée sur sulfate de magnésium puis évaporée. L'huile obtenue est directement engagée dans la réaction suivante.

### Stade B : iodure de 3-(5-bromo-1H-pyrrolo[2,3-b]pyridinyl)méthyltriméthyl-ammonium

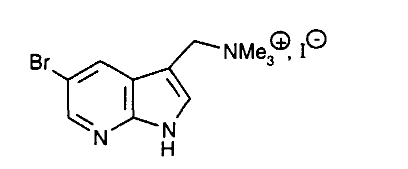

Le composé obtenu au stade précédent (5,1 mmol, quantité de 5-bromo-1H-pyrrolo [2,3-b]pyridine engagée) est repris dans l'acétone (20 cm³) et le iodométhane (0.635 cm³ ; 10.2 mmol). Du méthanol est ajouté en petite quantité pour éviter l'agglomération de l'ammonium formé. La réaction est agitée 24 h à température ambiante puis évaporée à sec pour donner un solide blanc qui est engagé dans la réaction suivante.

### Stade C : 5-bromo-3-cyanométhyl-1H-pyrrolo[2,3-b]pyridine

Le composé obtenu au stade précédent est dissous dans le diméthylformamide (25 cm³) en présence de cyanure de potassium (1,65 g ; 25,5 mmol = 5 équivalents par rapport à la quantité de 5-bromo-1H-pyrrolo[2,3-b]pyridine). Après agitation à température ambiante pendant 24 h, puis à 50°C pendant 48 h, du cyanure de potassium (1,65 g ; 25,5 mmol) est additionné au milieu réactionnel qui est agité 15 h de plus, à 50 °C. La réaction est évaporée en présence de toluène ; le résidu, repris dans l'acétate d'éthyle est lavé par de l'eau (3 fois) puis de la saumure (1 fois). La phase organique est séchée sur sulfate de magnésium et évaporée pour donner un solide. La purification sur colonne de silice (éluant : éther de pétrole : acétate d'éthyle, 2:1) permet d'obtenir 460 mg de solide blanc.
Rendement : 38 % sur 3 étapes
F = 169°C
IR (KBr) : ν = 2260 cm⁻¹ (CN), ν = 3132 cm⁻¹ (NH)
RMN ¹H (DMSO-d₆) : δ 4.07 (s, 2H, CH₂-CN), 7.56 (s, 1H, H-2), 8.30 (s, 1H, H-4), 8.32 (s, 1H, H-6), 11.94 (s, 1H, H-1).

### Stade D : 3-(2-aminoéthyl)-5-bromo-1H-pyrrolo[2,3-b]pyridine

Au composé obtenu au stade précédent (100 mg ; 0,42 mmol) dans le tétrahydrofuranne anhydre (5 cm³) sous atmosphère d'argon, est ajouté goutte à goutte à 0°C une solution de borane-tétrahydrofuranne (1,26 cm3 d'une solution 1,0 M ; 1,26 mmol). La réaction est agitée à reflux pendant 6 h avant d'être évaporée à sec ; le résidu est repris dans l'acide chlorhydrique 6 M tout en chauffant jusqu'à dissolution du précipité. Après refroidissement de la solution, de la soude à 50 % est ajoutée jusqu'à pH = 10 ; la phase aqueuse est extraite par l'acétate d'éthyle (2 fois). Après séchage sur sulfate de magnésium, la phase organique est évaporée pour donner une huile qui est engagée dans la réaction suivante.

### PREPARATION 13 : 1-AMINOETHYL-PYRROLO[2,3-b]PYRIDINE

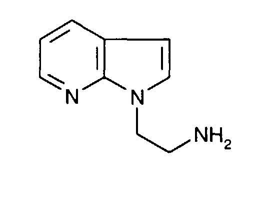

### Stade A : 1-bromoethyl-pyrrolo[2,3-b]pyridine

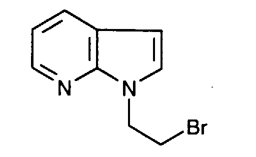

Le 7-azaindole (ou pyrrolo[2,3-*b*]pyridine) (1 g, 8.47 mmol) est dissous dans le N,N-diméthylformamide (DMF) (5 cm³) sous atmosphère inerte. L'hydrure de sodium (60 % dans l'huile) (0.51 g, 21.2 mmol) est additionné lentement au mélange réactionnel. Le milieu est agité pendant 1 h à température ambiante. L'anion du 7-azaindole préalablement formé est additionné goutte à goutte au moyen d'une ampoule isobare à une solution de 1,2-dibromoéthane (7.3 cm³, 84.7 mmol) en solution dans le DMF (4 cm³). Après évaporation du DMF, le résidu est hydrolysé avec H₂O puis extrait avec de l'acétate d'éthyle : la phase organique est séchée sur MgSO₄. L'évaporation du solvant, suivie d'une purification sur colonne de silice (EP/AcOEt, 8 : 2) permet d'isoler le composé du titre avec un rendement de 67 % sous la forme d'une huile.
RMN ¹H (CDCl₃) : δ (ppm) : 3.76 (t, 2H, CH₂, J_{CH2-CH2} = 6.6 Hz), 4.68 (t, 2H, CH₂, J_{CH2-CH2} = 6.6 Hz), 6.46 (d, 1 H, H-3, J₃₋₂ = 2.7 Hz), 7.07 (dd, 1H, H-5, J₅₋₆ = 4.8 Hz, J₅₋₄ = 7.9 Hz), 7.27 (d, 1H, H-2, J₂₋₃ = 3.7 Hz), 7.91 (dd, 1H, H-4, J₄₋₅ = 7.9 Hz, J₄₋₆ = 1.5 Hz), 8.30 (dd, 1H, H-6, J₆₋₅ = 4.8 Hz, J₆₋₄ = 1.5 Hz).

### Stade B : 1-azidoethyl-pyrrolo[2,3-b]pyridine

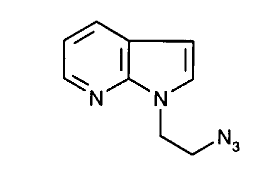

Le composé obtenu au stade précédent (1.2 g, 5.33 mmol) dissous dans le DMF (30 cm³) est additionné à une solution d'azoture de sodium (NaN₃) (1.04 g, 16 mmol) dans le DMF (30 cm³) sous atmosphère inerte. Le milieu réactionnel est maintenu 15 h à température ambiante. Après évaporation du solvant suivie d'une hydrolyse (H₂O) et d'une extraction au moyen de l'AcOEt, la phase organique est séchée sur MgSO₄ puis évaporée. Le résidu brut est purifié sur colonne de silice (EP/AcOEt, 8 : 2) pour conduire au composé attendu sous la forme d'une huile avec un rendement de 84 %.

RMN ¹H (CDCl₃) : δ (ppm) : 3.60 (t, 2H, CH₂, J_{CH2-CH2} = 5.2 Hz), 4.31 (t, 2H, CH₂, J_{CH2-CH2} = 5.2 Hz), 4.31 (t, 2H, CH₂, J_{CH2-CH2} = 5.2 Hz), 6.38 (d, 1H, H-3, J₃₋₂ = 3.3 Hz), 6.96 (dd, 1H, H-5, J₅₋₄ = 7.7 Hz, J₅₋₆ = 4.7 Hz), 7.13 (d, 1H, H-2, J₂₋₃ = 3.3 Hz), 7.8 (dd, 1H, H-4, J₄₋₅ = 7.7 Hz, J₄₋₆ = 1.5 Hz), 8.22 (dd, 1H, H-6, J₆₋₄ = 1.5 Hz, J₆₋₅ = 4.7 Hz).

### Stade C : 1-aminoéthyl-pyrrolo[2,3-b]pyridine

Le composé obtenu au stade précédent (1,15 g ; 6,14 mmol) est mis en solution dans l'éthanol (7 cm³). On additionne du palladium de Lindlar (165 mg, 15 % en masse) et on place le milieu réactionnel sous atmosphère d'hydrogène pendant 2 h à température ambiante. Après filtration sur celite suivie d'une évaporation on obtient le composé sous la forme d'une huile avec un rendement quantitatif.

RMN ¹H (CDCl₃) : δ (ppm) : 3.17 (t, 2H, CH₂, J_{CH2-CH2} = 5.9 Hz), 4.3 (t, 2H, CH₂, J_{CH2-CH2} = 5.9 Hz), 6.44 (d, 1H, H-3, J₃₋₂ = 3.3 Hz), 7.04 (dd, 1H, H-5, J₅₋₄ = 7.7 Hz, J₅₋₆ = 4.8 Hz), 7.23 (d, 1H, H-2, J₂₋₃ = 3.3 Hz), 7.89 (dd, 1H, H-4, J₄₋₅ = 7.7 Hz, J₄₋₆ = 1.5 Hz), 8.27 (dd, 1H, H-6, J₆₋₄ = 1.5 Hz, J₆₋₅ = 4.8 Hz).

### PREPARATION 14: 3-CYANOMETHYL-5-METHOXY-FURO-[3,2-b]PYRIDINE

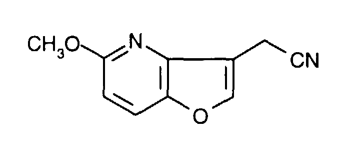

### Stade A : 2-iodo-5-(oxyran-2-yl-methyloxy)pyridine

Refroidir à 0°C, 1,84 g (8,33 mmol) de 5-hydroxy-2-iodopyridine en solution dans 18 cm³ de DMF. Additionner 220 mg (9,10 mmol) d'hydrure de sodium en plusieurs fois. Après 30 min à température ambiante, additionner goutte à goutte 6,5 cm³ (7.70 g - 53,26 mmol) d'épichlorhydrine en solution dans 3 cm³ de DMF. Maintenir à 60 °C pendant 2 h. Evaporer le solvant. Reprendre le résidu par H₂O et extraire au CH₂Cl₂. Après avoir séché et concentré la phase organique, purifier le produit sur colonne de silice, éluant Acétate d'éthyle : Ether de pétrole, 5 : 5.
On obtient 2 g d'un solide orangé (F = 43-44°C). Rendement 84 %

### Stade B : (2,3-dihydro-5-iodo-furo[3,2-b]pyridin-3-yl)méthanol

Refroidir à - 78°C, 1 g (3,01 mmol) de composé obtenu au stade précédent en solution dans 10 cm³ de tétrahydrofurane anhydre. Goutte à goutte, additionner 3,01 cm³ (7,22 mmol) de lithium diisopropylamide 2 M dilués dans 5 cm³ de tétrahydrofurane. Après 2h à - 78°C, hydrolyser avec 20 cm³ d'eau, extraire au CH₂Cl₂. Après séchage sur MgSO₄ et concentration de la phase organique, purifier le produit sur colonne de silice normale: éluant : Acétate d'éthyle : Ether de pétrole, 2 : 1. On récupère 620 mg d'un solide jaune (F = 120°C). Rendement 62 %.

### Stade C : 5-iodo-3-tosyloxyméthyl-furo[3,2-b]pyridine

Refroidir à 0°C, 1,85g (6,68.10⁻³ mmol) du composé obtenu au stade précédent en solution dans 60 cm³ de dichlorométhane anhydre. Additionner goutte à goutte 2,3 cm³ (2,03 g, 20,03 mmol) de triéthylamine puis 1,91 g (10,01 mmol) du chlorure de tosyle en solution dans 20 cm³ de dichlorométhane. Après 30 h à température ambiante, évaporer le solvant. Purifier sur colonne de silice normale ; éluant Acétate d'éthyle : Ether de pétrole, 1:3.
On récupère 2,54 g d'un solide blanc (F : 138-139 °C). Rendement : 88 %.

### Stade D: 5-méthoxy-3-méthyl-furo[3,2-b]pyridine

A 2,54 g (5,89 mmol) de composé obtenu au stade précédent en solution dans 25 cm³ de DMF, additionner 1,27 g (23,57 mmol) de méthylate de sodium. Après 1h30 à 80°C, additionner 320 mg (5,89 mmol-1eq) de méthylate de sodium. Après 3 h, évaporer le solvant, reprendre le résidu par H₂O. Extraire au CH₂Cl₂, Sécher la phase organique sur MgSO₄. Après évaporation du solvant, purifier sur colonne de silice ; éluant Acétate d'éthyle : Ether de pétrole, 1:3.
On obtient 580 mg d'une huile incolore. Rendement 60 %.

### Stade E : 3-bromométhyl-5-méthoxy-furo[3,2-b]pyridine

Chauffer à reflux pendant 9h30, 350 mg (2,14 mmol) de composé obtenu au stade précédent dans 7 cm³ de tétrachlorure de carbone anhydre, 400 mg (2,25 mmol) de NBS (N-bromo succinimide) recristallisé dans l'eau et une pointe de spatule de 2,2'-azobis-2-méthylproprionitrile. Evaporer alors le solvant et purifier le résidu sur colonne de silice ; éluant : Acétate d'éthyle : éther de pétrole, 5 : 95.
On obtient 340 mg d'un solide orangé (F : 86-87°C). Rendement : 65 %.

### Stade F: 3-cyanométhyl-5-méthoxy-furo[3,2-b]pyridine

A 340 mg (1,40 mmol) de composé obtenu au stade précédent en solution dans 10 cm³ de DMF, additionner 150 mg (2,25 mmole) de cyanure de potassium. Après 10 h à température ambiante, évaporer le solvant. Purifier le résidu sur colonne de silice. Eluant : Acétate d'éthyle : Ether de pétrole, 1: 2.
On obtient 220 mg d'un solide blanc (F : 95-96°C). Rendement : 83 %.

### PREPARATION 15 : 3-CYANOMETHYL-7-METHOXY-FURO[2,3-c]PYRIDINE

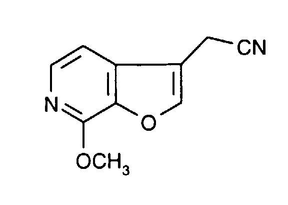

### Stade A : 3-méthyl-7-méthoxy-2,3-dihydro-furo[2,3-c]pyridine

A 2,25 g (6,65 mmol) du composé obtenu au stade C de la préparation 4 dissous dans 30 cm³ de N,N-diméthylformamide, 1,44 g (26,6 mmol) de méthylate de sodium sont additionnés à température ambiante. L'agitation est maintenue à 80°C sous atmosphère inerte. Après évaporation sous vide, le produit brut est extrait au CH₂Cl₂. La phase organique est séchée sur MgSO₄ puis évaporée sous vide. Le produit est chromatographié sur colonne de silice (éluant : EP : AcOEt : 6 : 4) pour donner 0,84 g de produit pur sous forme d'une huile jaune.
Rendement: 78 %
MS *m/z* 164 (M+1)
¹H RMN (CDCl₃) : δ 2,18 (s, 3H, CH₃), 4,08 (s, 3H, O-CH₃), 7,03 (d, 1H, J = 6Hz, H_{pyr}), 7,87 (d, 1H, J = 5,1 Hz, H_{pyr}).

### Stade B : 3-bromométhyl-7-méthoxy-furo[2,3-c]pyridine :

A 0,840 g (5,15 mmol) du composé obtenu au stade précédent dissous dans 15 cm³ de CCl₄, on additionne une spatule d'A.I.B.N. et 0,963 g (5,411 mmol) de N-bromosuccinimide. Le milieu réactionnel est chauffé à reflux à l'aide d'une ampoule de 75 W, sous atmosphère inerte, pendant 4 h. Après retour à température ambiante, le solvant est évaporé. On hydrolyse avec 30 cm³ d'eau puis on extrait au CH₂Cl₂. La phase organique est séchée sur MgSO₄ puis évaporée. Le produit brut est purifié par chromatographie sur colonne de silice (éluant : EP : AcOEt, 8 : 2). On récupère 0,573 g du composé attendu.
Rendement : 46 %
F : 110-111°C
IR (cm-1) (KBr) : 1230 et 1210 (C-O-C-)
¹H RMN (CDCl₃) δ 4,07 (s, 3H, O-CH₃), 4,51 (s, 2H, CH₂), 7,19 (d, 1H J = 5,15 Hz, H_{pyr}), 7,69 (s, 1H, CH), 7,94 (d, 1H, J = 5,15 H_{pyr}).

### Stade C : 3-cyanométhyl-7-méthoxy-furo[2,3-c]pyridine :

A 360 mg (1,48 mmol) du composé obtenu au stade précédent dissous dans 10 cm³ de DMF, on additionne, à température ambiante, 155 mg (2,38 mmol) de KCN. L'agitation est maintenue pendant 5 h. Le DMF est évaporé sous vide puis le produit brut est purifié par chromatographie sur colonne de silice (éluant : EP : AcOEt, 6 : 4). On récupère 210 mg du produit attendu, sous forme d'un solide blanc.
Rendement : 75 %
IR (KBr) : 2235 cm⁻¹ (CN)
¹H RMN (CDCl₃) : δ 3,72 (s, 2H, CH₂), 4,11 (s, 3H, O-CH₃), 7,12 (d, 1H, J = 5,5 Hz, H_{pyr}), 7,72 (s, 1H, CH), 7,97 (d, 1H, J = 5,5 Hz, H_{pyr}).

### PREPARATION 16 : 4-ACETAMIDO-1-TRIMETHYLSILYL-BUT-1-YNE

### Stade A: O-tosyl-but-3-yn-1-ol

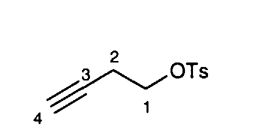

Sous argon et en milieu anhydre à une solution de 3-butyn-1-ol (0,540 cm³ ; 7,13 mmol) de triéthylamine (2 cm³ ; 14,3 mmol) dans le dichlorométhane (15 cm³) est ajouté à 0°C le chlorure de tosyle (1,5 g ; 7,85 mmol) dans le dichlorométhane (10 cm³). Après 6 h d'agitation à température ambiante, la réaction est hydrolysée par l'eau et extraite par le dichlorométhane (2 fois). La phase organique est lavée avec de l'eau (3 fois), séchée sur sulfate de magnésium et évaporée. La purification sur gel de silice (éluant : éther de pétrole : acétate d'éthyle, 5:1) du résidu permet d'obtenir une huile (1,37 g).
Rendement : 86 %

### Stade B : O-tosyl-4-triméthylsilyl-but-3-yn-1-ol

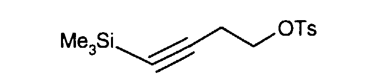

Sous argon et en milieu anhydre, à une solution du composé obtenu au stade précédent (6,45 g ; 28,8 mmol) dans le tétrahydrofuranne (130 cm³) à - 78°C est additionné goutte à goutte une solution de butyllithium 1,6 M dans l'hexane (18,9 cm³ ; 30,2 mmol). Au bout d'une heure d'agitation à - 78°C, le chlorure de triméthylsilyle (5,5 cm³ ; 43,1 mmol) est ajouté lentement. La réaction est agitée 15 min à -78°C et 1 h à température ambiante avant d'être hydrolysée par de l'eau ; le milieu est extrait par le dichlorométhane (2 fois), séché sur sulfate de magnésium. Après évaporation des solvants, la purification sur gel de silice (éluant : éther de pétrole : acétate d'éthyle , 95 : 5) du résidu permet d'obtenir 7,71 g d'une huile.
Rendement : 90 %

### Stade C : 4-azido-1-triméthylsilyl-but-1-yne

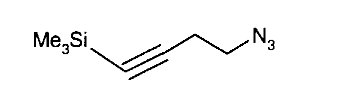

Sous argon et en milieu sec, le mélange du composé obtenu au stade précédent (5,45 g ; 18,4 mmol) et d'azoture de sodium (3,6 g ; 55,2 mmol) dans le diméthylformamide (25 cm³) est agité à température ambiante pendant 48 h. Le milieu est dilué dans l'acétate d'éthyle et lavé par l'eau (5 fois). Après séchage sur sulfate de magnésium l'évaporation des solvants permet d'obtenir une huile (3,08 g) qui est directement réduite.

### Stade D : 4-amino-1-triméthylsilyl-but-1-yne

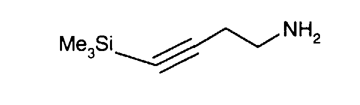

Sous argon et en milieu anhydre, une solution du composé obtenu au stade précédent (1,96 g ; 11,7 mmol) dans l'éther diéthylique (80 cm³) est ajouté goutte à goutte à une suspension d'hydrure d'aluminium lithium (560 mg ; 14,6 mmol) dans l'éther diéthylique (40 cm³). Après 6 h d'agitation à température ambiante, la réaction est hydrolysée par 0,570 cm³ d'eau, 0,570 cm³ de soude à 15 % et 1,71 cm³ d'eau ; le milieu est filtré sur célite, le résidu étant lavé par l'éther diéthylique et le 1,4-dioxanne. L'évaporation des solvants permet d'obtenir une huile qui est directement acétylée.

### Stade E: 4-acetamido-1-triméthylsilyl-but-1-yne

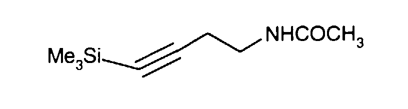

Sous argon et en milieu sec, au composé obtenu au stade précédent dissous dans le dichlorométhane (30 cm³) et la pyridine (2,6 cm³) est ajouté lentement et à 0°C l'anhydride acétique (1,3 cm³ ; 14,0 mmol). Après 22 h d'agitation à température ambiante, le milieu est hydrolysé par l'eau et extrait par le dichlorométhane. La phase organique est lavée successivement par de l'eau (5 fois) et par de la saumure (2 fois), séchée sur sulfate de magnésium et évaporée. Le résidu huileux est purifié sur gel de silice (éluant : éther de pétrole : acétate d'éthyle, 2:1) pour donner 1,50 g de solide blanc.
Rendement : 70 %, 3 étapes
F = 84°C
IR (KBr) : ν : 3268 cm⁻¹ (NH), ν : 1655 cm⁻¹ (C=O)
RMN ¹H (CDCl₃) : δ 0.15 (s, 9H, Si (CH₃)₃), 1.99 (s, 3H, COCH₃), 2.43 (t, 2H, J₃₋₄ = 6.0 Hz, H-3), 3.38 (q, 2H, J₄₋₃ = J₄₋₅ = 6.0 Hz, H-4), 5.76 (s, 1H, NH).

### PREPARATION 17: 4-AMINO-3-IODO-PYRIDINE

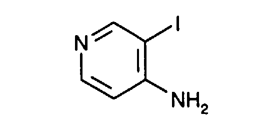

### Stade A: 4-tertiobutanamido-pyridine

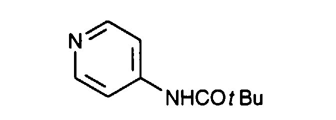

Sous argon et en milieu sec, à une solution de 4-aminopyridine (1 g ; 10,6 mmol) et de triéthylamine (1,9 cm³ ; 13,25 mmol) dans le dichlorométhane (15 cm³) est additionné lentement à 0°C le chlorure de pivaloyle (1,4 cm³ ; 11,7 mmol) dans du dichlorométhane (2 cm³). La réaction est agitée 10 min à 0°C puis 2 h à température ambiante avant d'être diluée dans le dichlorométhane et lavée par de l'eau (2 fois). Après séchage sur sulfate de magnésium et évaporation des solvants, le résidu est purifié sur gel de silice (éluant : dichlorométhane : méthanol, 99 : 1) pour donner 1,7 g de solide blanc.
Rendement : 91 %
F : 135°C

### Stade B: 3-IODO-4-TERTIOBUTANAMIDO-PYRIDINE

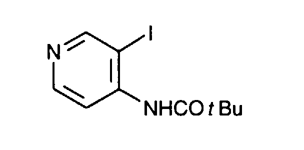

Sous argon et en milieu anhydre, à une solution du composé obtenu au stade précédent (4,4 g ; 24,7 mmol) et de tétraméthyléthylènediamine (8,9 cm³ ; 59,25 mmol) dans le tétrahydrofuranne (100 cm³) est ajouté goutte à goutte à -78°C une solution de butyllithium 1,6 M dans l'hexane (37 cm³ ; 59,25 mmol). Après 1 h 30 min d'agitation à une température comprise entre - 20°C et - 10°C, une solution d'iode (9,4 g ; 37,05 mmol) dans le THF (20 cm³) est ajoutée à la réaction par transfert à - 78°C. Le milieu est agité 10 min à - 78°C et 30 min à température ambiante avant d'additionner de l'eau ; le milieu est extrait par l'acétate d'éthyle (2 fois), la phase organique étant lavée par une solution saturée de thiosulfate de sodium et par de l'eau. Après séchage sur sulfate de magnésium et évaporation des solvants, le résidu est purifié sur gel de silice (éluant : éther de pétrole : acétate d'éthyle, 1:1) pour donner 6,16 g de solide blanc.
Rendement : 82 %
F : 158°C

### Stade C : 4-amino-3-iodopyridine

Le composé obtenu au stade précédent (2,21 g ; 7,3 mmol) en suspension dans une solution d'acide sulfurique à 10 % dans l'eau (73 cm³) est agité à reflux pendant 15 h. Après refroidissement, la solution est alcalinisée par de la soude à 50 % et extraite par l'acétate d'éthyle (2 fois). Après séchage sur sulfate de magnésium et évaporation du solvant, le résidu est purifié par chromatographie sur gel de silice (éluant : dichlorométhane : méthanol, 95 : 5) pour donner 1,54 g de solide blanc.
Rendement : 97 %
F : 80-81°C
IR (KBr) : ν : 3525 cm⁻¹ (NH₂)
RMN¹H (CDCl₃) : δ 4.69 (s, 2H, NH₂), 6.59 (d, 1H, J₅₋₆ = 5.15 Hz, H-5), 8.11 (d, 1H, J₆₋ ₅ = 5.15 Hz, H-6), 8.57 (s, 1H, H-2).

### EXEMPLE 1 : N-[2-(5-METHOXY-1-METHYL-PYRROLO[2,3-b]PYRIDIN-3-YL)ETHYL]ACETAMIDE

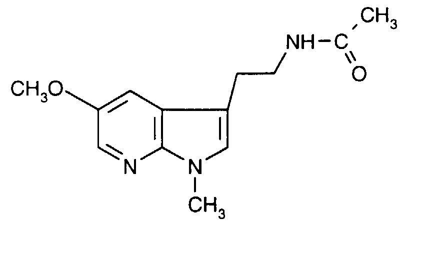

A 70 mg (3.42 10⁻⁴ mmol) du composé obtenu dans la préparation 1 en solution à 0°C dans 0.7 cm³ de dichlorométhane anhydre sont additionnés 0.08 cm³ de pyridine et 0.04 cm³ d'anhydride acétique (4.09 10⁻⁴ mmol - 42 mg). Après 30 min à 0°C, le milieu est hydrolysé par 1 cm³ d'eau, neutralisé par une solution saturée de bicarbonate de soude et extrait au dichlorométhane. Après séchage sur sulfate de magnésium, la phase organique est évaporée. Le produit est purifié sur colonne de gel de silice et obtenu sous forme de solide (70 mg).
Rendement : 83 %
F =115 - 116°C
IR (KBr) : ν = 3300 cm⁻¹ (NH), ν = 1630 cm⁻¹ (C=O)
RMN ¹H (CDCl₃) : δ 1.98 (s, 3H, CO-CH₃) ; 2.91 (t, 2H, J = 6.8 Hz, CH₂), 3.56 (q, 2H, J = 6.8 Hz, CH₂), 3.83 (s, 3H, CH₃), 3.91 (s, 3H, CH₃), 5.50 (s, large, 1H, NH), 6.99 (s, 1H, H-2), 7.38 (d, 1H, J₄₋₆ = 2.7 Hz, H-4), 8.12 (d, 1H, J₆₋₄ = 2.7 Hz, H-6).
MS *m/z* 248 (M+1)

### EXEMPLE 2 : N-[2-(1-H-PYRROLO[2,3-b]PYRIDIN-3-YL)ETHYL]ACETAMIDE

En procédant comme dans l'exemple 1, mais en partant de la préparation 2, on obtient le composé du titre.
Rendement : 54 %
F =168°C
RMN ¹H (DMSO-d₆) : δ 1.75 (s, 3H, CH₃) ; 2.78 (t, 2H, J = 7.2 Hz, CH₂), 3.29 (t, 2H, J = 7.2 Hz, CH₂) 7.05 (dd, 1H, J₅₋₄ = 7.8 Hz, J₅₋₆ = 4.7 Hz, H-5), 7.22 (s, 1H, H-2), 7.95 (d, 1H, J₄₋₅ = 7.8 Hz, H-4), 8.13 (d, 1H, J₆₋₅ = 4.7 Hz, H-6).
MS *m/z* 204 (M+1)

### EXEMPLE 3 : N-[2-(5-METHOXY-1H-PYRROLO[2,3-b]PYRIDIN-3-YL)ETHYL] ACETAMIDE

A une suspension de la préparation 3 dans le dichlorométhane (1 cm³) sous argon, à 0°C la pyridine (86 cm³) puis l'anhydride acétique (43 cm³ ; 0.46 mmol) sont ajoutés (la solution devient alors limpide) ; la réaction est agitée 2 h 30 min à 0°C et 1 h à température ambiante (présence d'un précipité) ; le milieu est hydrolysé par l'eau. La phase aqueuse est extraite deux fois par le dichlorométhane et deux fois par l'acétate d'éthyle, les phases organiques étant lavées par l'eau. Après séchage sur MgSO₄ et évaporation des solvants, le résidu est purifié par chromatographie sur gel de silice (éluant : CH₂Cl₂ : MeOH, 9 : 1). Le composé du titre est obtenu sous forme de solide légèrement jaune (33 mg).
Rendement : 37 % sur deux étapes
F = 159°C
IR (KBR) : ν = 3300-3000 cm⁻¹ (NH), ν = 1630 cm⁻¹ (CO)
RMN ¹H (DMSO-d₆) : δ 1.73 (s, 3H, CO-CH₃) ; 2.81 (t, 2H, J = 7.6 Hz, CH₂), 3.38 (q, 2 H, J = 7.6 Hz, CH₂), 3.93 (s, 3 H, OCH₃), 7.63 (d, 1H, J_{2,1} = 2.4 Hz, H-2), 7.95 (d, 1 H, J_{4,6} = 2.7 Hz, H-4), 8.37 (t, 1 H, J = 7.6 Hz, NH-CO), 8.40 (d, 1 H, J_{6,4} = 2.7 Hz, H-6), 11.9 (s large, 1 H, H-1)
MS *(m/z)* : 234 (M+1)

### EXEMPLE 4 : N-[2-(7-CHLORO-FURO[2,3-c]PYRIDIN-3-YL)ETHYL]ACETAMIDE

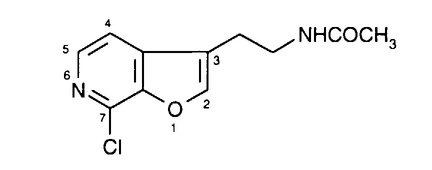

A 200 mg (1.04 mmol) du composé obtenu lors de la préparation 4 dissous dans 10 cm³ d'anhydride acétique, on additionne successivement 31 mg (0.52 mmol) de nickel de Raney et 125 mg (1.56 mmol) d'acétate de sodium. L'agitation est maintenue 12 h à 50 °C sous atmosphère d'hydrogène. Après retour à température ambiante, le catalyseur est filtré sur célite. Après évaporation à sec, le produit est hydrolysé avec 25 cm³ d'eau et extrait au CH₂Cl₂. La phase organique est séchée sur MgSO₄ puis évaporée sous vide. Le produit est purifié par chromatographie sur colonne de silice (éluant : CH₂Cl₂ : MeOH, 95 : 5). On récupère 187 mg de produit pur sous forme d'un solide.
Rendement : 75 %
F = 104°C
IR (KBr) : ν = 3310 cm⁻¹ (NH), ν = 1660 cm⁻¹ (C=O)
RMN ¹H (CDCl₃ + D₂O) : δ 1.97 (s, 3H, CH₃), 2.92 (t, 2H, J = 7.0 Hz, CH₂), 3.57 (t, 2H, J = 7.0 Hz, CH₂), 7.48 (d, 1H, J = 5.3 Hz, H_{pyr}), 7.66 (s, 1H, CH), 8.21 (d, 1H, J = 5.3 Hz, H_{pyr}).
MS *m/z* 239 (M+1)

### EXEMPLE 5 : N-[2-(FURO[2,3-c]PYRIDIN-3-YL)ETHYL]ACETAMIDE

200 mg (0,84 mmol) du composé obtenu à l'exemple 4 sont dissous dans 15 cm³ d'acide acétique puis on additionne 308 mg (4,7 mmol) de zinc. L'agitation est maintenue à 60°C pendant 5 h sous atmosphère d'argon. Après retour à température ambiante, le catalyseur est filtré sur coton. Après évaporation à sec, le produit est hydrolysé avec NaHCO₃ 5 % et extrait au CH₂Cl₂. La phase organique est séchée sur MgSO₄ puis évaporée sous vide. Le produit est purifié par chromatographie sur colonne de silice (éluant : CH₂Cl₂ : MeOH, 95:5). On récupère 144 mg de produit pur sous forme d'un solide.
Rendement : 84 %
F : 90°C
IR (KBr) : ν = 3290 cm⁻¹ (NH), ν = 1660 cm⁻¹ (C=O)
RMN ¹H (CDCl₃ + D₂O) : δ 1.95 (s, 3H, CH₃), 2.91 (t, 2H, J = 6.7 Hz, CH₂), 3.57 (t, 2H, J = 6.7 Hz, CH₂), 7.52 (d, 1H, J = 5.1 Hz, H_{pyr}), 7.58 (s, 1H, H_{pyr}), 8.42 (d, 1H, J = 5.1 Hz, H_{pyr}), 8.85 (s, 1H, H_{pyr}).
MS *m/z* 205 (M+1)

### EXEMPLES 6 A 22 :

En procédant comme dans l'exemple 1 mais en remplaçant l'anhydride acétique par l'anhydride ou le chlorure d'acyle approprié, on obtient les composés des exemples suivants :

### EXEMPLE 6 : N-[2-(5-METHOXY-1-METHYL-PYRROLO[2,3-b]PYRIDIN-3-YL)ETHYL] PROPIONAMIDE

### EXEMPLE 7 : N-[2-(5-METHOXY-1-METHYL-PYRROLO[2,3-b]PYRIDIN-3-YL)ETHYL] BUTYRAMIDE

### EXEMPLE 8 : N-[2-(5-METHOXY-1-METHYL-PYRROLO[2,3-b]PYRIDIN-3-YL)ETHYL] PENTANAMIDE

### EXEMPLE 9 : N-[2-(5-METHOXY-1-METHYL-PYRROLO[2,3-b]PYRIDIN-3-YL)ETHYL] HEXANAMIDE

### EXEMPLE 10 : N-[2-[5-METHOXY-1-METHYL-PYRROLO[2,3-b]PYRIDIN-3-YL)ETHYL] 2-IODOACETAMIDE

### EXEMPLE 11 : N-[2-(5-METHOXY-1-METHYL-PYRROLO[2,3-b]PYRIDIN-3-YL)ETHYL] TRIFLUOROACETAMIDE

### EXEMPLE 12 : N-[2-(5-METHOXY-1-METHYL-PYRROLO[2,3-b]PYRIDIN-3-YL)ETHYL] ISOPENTANAMIDE

### EXEMPLE 13 : N-[2-(5-METHOXY-1-METHYL-PYRROLO[2,3-b]PYRIDIN-3-YL)ETHYL] CYCLOPROPYLCARBOXAMIDE

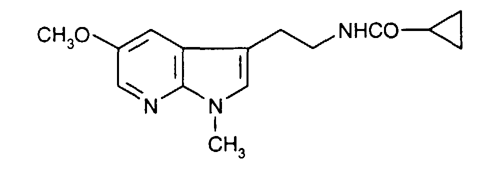

A 450 mg (2.19 mmol) du composé de la préparation 1 en solution à 0°C dans 9 cm³ de dichlorométhane anhydre sont additionnés 0,37 cm³ (2.63 mmol) de triéthylamine et 0.24 cm³ (2.63 mmol) de chlorure de cyclopropylcarbonyle. Après 1 heure à 0°C, le milieu est hydrolysé par de l'eau, neutralisé par une solution saturée de bicarbonate de soude et extrait au dichlorométhane. Après séchage sur sulfate de magnésium, la phase organique est évaporée. Le produit est purifié sur colonne de gel de silice (éluant = acétate d'éthyle) et obtenu sous forme de solide jaune.
Rendement : 77 %
F : 133°C
IR (KBr) : ν = 3233 cm⁻¹ (NH), ν = 1639 cm⁻¹ (C=O).
RMN¹H (CDCl₃) : δ 0.64-0.70 (m, 2H, cyclopropyle), 0.91-0.97 (m, 2H, cyclopropyle), 1.18-1.26 (m, 1H, cyclopropyle), 2.88 (t, 2H, J = 6.6 Hz, Ar-CH₂-), 3.54 (q, 2H, J = 6.6 Hz, -CH₂-NH), 3.79 (s, 3H, N-CH₃), 3.85 (s, 3H, OCH₃), 5.65-5.78 (massif, 1H, NH), 6.97 (s, 1H, H-2), 7.35 (d, 1H, J = 2.9 Hz, H-4), 8.08 (d, 1H, J = 2.9 Hz, H-6).
MS m/z : 274 (M+1)

### EXEMPLE 14 : N-[2-(5-METHOXY-1-METHYL-PYRROLO[2,3-b]PYRIDIN-3-YL)ETHYL] CYCLOBUTYLCARBOXAMIDE

### EXEMPLE 15: N-[2-(5-METHOXY-1-METHYL-PYRROLO[2,3-b]PYRIDIN-3-YL)ETHYL] CYCLOPENTYLCARBOXAMIDE

### EXEMPLE 16 : N-[2-(5-METHOXY-1-METHYL-PYRROLO[2,3-b]PYRIDIN-3-YL)ETHYL] CYCLOHEXYLCARBOXAMIDE

### EXEMPLE 17 : N-[2-(5-METHOXY-1-METHYL-PYRROLO[2,3-b]PYRIDIN-3-YL)ETHYL] CYCLOPROPYLMETHYLCARBOXAMIDE

### EXEMPLE 18 : N-[2-(5-METHOXY-1-METHYL-PYRROLO[2,3-b]PYRIDIN-3-YL)ETHYL] ALLYLCARBOXAMIDE

### EXEMPLE 19 : N-[2-(5-METHOXY-1-METHYL-PYRROLO[2,3-b]PYRIDIN-3-YL)ETHYL] ISOBUTYRAMIDE

### EXEMPLE 20 : N-[2-(5-METHOXY-1-METHYL-PYRROLO[2,3-b]PYRIDIN-3-YL)ETHYL] PROPENYLCARBOXAMIDE

### EXEMPLE 21 : N-[2-(5-METHOXY-1-METHYL-PYRROLO[2,3-b]PYRIDIN-3-YL)ETHYL] BROMOACETAMIDE

### EXEMPLE 22 : N-[2-(5-METHOXY-1-METHYL-PYRROLO[2,3-b]PYRIDIN-3-YL)ETHYL] HEPTANAMIDE

### EXEMPLE 23 : N-[2-(5-METHOXY-1-METHYL-PYRROLO[2,3-b]PYRIDIN-3-YL)ETHYL] FORMAMIDE

En procédant comme dans l'exemple 1 mais en utilisant l'acide formique au lieu de l'anhydride acétique, on obtient le composé du titre.

### EXEMPLES 24 A 32 :

En procédant comme dans l'exemple 1 mais en remplaçant l'anhydride acétique par l'isocyanate ou l'isothiocyanate approprié, on obtient les composés des exemples suivants :

### EXEMPLE 24 : N-[2-(5-METHOXY-1-METHYL-PYRROLO[2,3-b]PYRIDIN-3-YL)ETHYL] N'-METHYLUREE

### EXEMPLE 25 : N-[2-(5-METHOXY-1-METHYL-PYRROLO[2,3-b]PYRIDIN-3-YL)ETHYL] N'-ETHYLUREE

### EXEMPLE 26 : N-[2-(5-METHOXY-1-METHYL-PYRROLO[2,3-b]PYRIDIN-3-YL)ETHYL] N'-PROPYLUREE

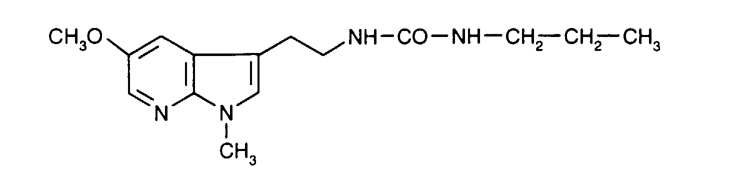

A 470 mg (2.29 mmol) du composé obtenu à la préparation 1 en solution à 0°C dans 5 cm³ de dichlorométhane anhydre sont additionnés 0,40 cm³ (2.75 mmol) de triéthylamine et 0.26 cm³ (2.75 mmol) de l'isocyanate de propyle. Après 2 heures à 0°C, le milieu est hydrolysé par de l'eau, neutralisé par une solution saturée de bicarbonate de soude et extrait au dichlorométhane. Après séchage sur sulfate de magnésium, la phase organique est évaporée. Le produit est purifié sur colonne de gel de silice (éluant = acétate d'éthyle) et obtenu sous forme de solide jaune (490 mg).
Rendement : 74 %
F : 102-103°C
IR (KBr) : ν = 3233 cm⁻¹ (NH), ν = 1625 cm⁻¹ (C=O).
RMN¹H (CDCl₃) : δ 0.88 (t, 3H, J = 7.4 Hz, CH₃), 1.46 (sextuplet, 2H, J = 7.4 Hz, CH₂-CH₃), 2.90 (t, 2H, J = 6.6 Hz, Ar-CH₂), 3.07 (q, 2H, J = 6.6 Hz, CH₂-NH-CO-), 3.48 (q, 2H, J = 7.4 Hz, CO-NH-CH₂), 3.79 (s, 3H, N-CH₃), 3.88 (s, 3H, OCH₃), 4.20-4.29 (massif, 1H, NH), 4.31-4.40 (massif, 1H, NH), 6.97 (s, 1H, H-2), 7.37 (d, 1H, J = 2.9 Hz, H-4), 8.09 (d, 1H, J = 2.9 Hz, H-6).
MS m/z : 291 (M+1)

### EXEMPLE 27 : N-[2-(5-METHOXY-1-METHYL-PYRROLO[2,3-b]PYRIDIN-3-YL)ETHYL] N'-BUTYLUREE

### EXEMPLE 28 : N-[2-(5-METHOXY-1 METHYL-PYRROLO[2,3-b]PYRIDIN-3-YL)ETHYL] N'-METHYLTHIOUREE

### EXEMPLE 29 : N-[2-(5-METHOXY-1 -METHYL-PYRROLO[2,3-b]PYRIDIN-3-YL)ETHYL] N'-ETHYLTHIOUREE

### EXEMPLE 30 : N-[2-(5-METHOXY-1-METHYL-PYRROLO[2,3-b]PYRIDIN-3-YL)ETHYL] N'-PROPYLTHIOUREE

### EXEMPLE 31 : N-[2-(5-METHOXY-1-METHYL-PYRROLO[2,3-b]PYRIDIN-3-YL)ETHYL] N'-BUTYLTHIOUREE

### EXEMPLE 32 : N-[2-(5-METHOXY-1-METHYL-PYRROLO[2,3-b]PYRIDIN-3-YL)ETHYL] N'-CYCLOPROPYLUREE

### EXEMPLES 33 A 38 :

En procédant comme dans l'exemple 2, mais en utilisant les réactifs appropriés, on obtient les composés des exemples suivants:

### EXEMPLE 33 : N-[2-(1-H-PYRROLO[2,3-b]PYRIDIN-3-YL) ETHYL]PROPIONAMIDE

### EXEMPLE 34 : N-[2-(1-H-PYRROLO[2,3-b]PYRIDIN-3-YL)ETHYL]CYCLOPROPYL CARBOXAMIDE

### EXEMPLE 35 : N-[2-(1-H-PYRROLO[2,3-b]PYRIDIN-3-YL) ETHYL]CYCLOBUTYL CARBOXAMIDE

### EXEMPLE 36 : N-[2-(1-H-PYRROLO[2,3-b]PYRIDIN-3-YL) ETHYL]TRIFLUORO ACETAMIDE

### EXEMPLE 37 : N-[2-(1-H-PYRROLO[2,3-b]PYRIDIN-3-YL)ETHYL] N'-METHYLUREE

### EXEMPLE 38 : N-[2-(1-H-PYRROLO[2,3-b]PYRIDIN-3-YL)ETHYL] N'-PROPYLUREE

### EXEMPLES 39 A 44 :

En procédant comme dans l'exemple 3, mais en utilisant les réactifs appropriés, on obtient les composés des exemples suivants:

### EXEMPLE 39 : N-[2-(5-METHOXY-1H-PYRROLO[2,3-b]PYRIDIN-3-YL)ETHYL] PROPIONAMIDE

### EXEMPLE 40 : N-[2-(5-METHOXY-1H-PYRROLO[2,3-b]PYRIDIN-3-YL) ETHYL] CYCLOPROPYLCARBOXAMIDE

### EXEMPLE 41 : N-[2-(5-METHOXY-1H-PYRROLO[2,3-b]PYRIDIN-3-YL) ETHYL] CYCLOBUTYLCARBOXAMIDE

### EXEMPLE 42 : N-[2-(5-METHOXY-1H-PYRROLO[2,3-b]PYRIDIN-3-YL) ETHYL] TRIFLUOROACETAMIDE

### EXEMPLE 43 : N-[2-(5-METHOXY-1H-PYRROLO[2,3-b]PYRIDIN-3-YL) ETHYL] N'-METHYLUREE

### EXEMPLE 44 : N-[2-(5-METHOXY-1H-PYRROLO[2,3-b]PYRIDIN-3-YL) ETHYL] N'-PROPYLUREE

### EXEMPLES 45 A 50 :

En procédant comme dans l'exemple 4, mais en utilisant les réactifs appropriés, on obtient les composés des exemples suivants:

### EXEMPLE 45 : N-[2-(7-CHLORO-FURO[2,3-c]PYRIDIN-3-YL) ETHYL]PROPIONAMIDE

### EXEMPLE 46 : N-[2-(7-CHLORO-FURO[2,3-c]PYRIDIN-3-YL) ETHYL]CYCLOPROPYLCARBOXAMIDE

### EXEMPLE 47 : N-[2-(7-CHLORO-FURO[2,3-c]PYRIDIN-3-YL) ETHYL]CYCLOBUTYLCARBOXAMIDE

### EXEMPLE 48 : N-[2-(7-CHLORO-FURO[2,3-c]PYRIDIN-3-YL) ETHYL]TRIFLUORO ACETAMIDE

### EXEMPLE 49 : N-[2-(7-CHLORO-FURO[2,3-c]PYRIDIN-3-YL) ETHYL] N'-METHYLUREE

### EXEMPLE 50 : N-[2-(7-CHLORO-FURO[2,3-c]PYRIDIN-3-YL) ETHYL] N'-PROPYLUREE

### EXEMPLES 51 A 56 :

En procédant comme dans l'exemple 5, mais en partant des exemples 45 à 50, on obtient les composés des exemples suivants:

### EXEMPLE 51 : N-[2-(FURO[2,3-c]PYRIDIN-3-YL)ETHYL]PROPIONAMIDE

### EXEMPLE 52 : N-[2-FURO[2,3-c]PYRIDIN-3-YL)ETHYL]CYCLOPROPYL CARBOXAMIDE

### EXEMPLE 53 : N-[2-(FURO[2,3-c]PYRIDIN-3-YL)ETHYL]CYCLOBUTYL CARBOXAMIDE

### EXEMPLE 54 : N-[2-(FURO[2,3-c]PYRIDIN-3-YL)ETHYL]TRIFLUOROACETAMIDE

### EXEMPLE 55 : N-[2-(FURO[2,3-c]PYRIDIN-3-YL)ETHYL]N'-METHYLUREE

### EXEMPLE 56 : N-[2-(FURO[2,3-c]PYRIDIN-3-YL)ETHYL]N'-PROPYLUREE

### EXEMPLES 57 A 62 :

En procédant comme dans l'exemple 1, mais en partant des préparations 5 à 10, on obtient les composés des exemples suivants :

### EXEMPLE 57 : N-[2-(4-METHYL-1-PHENYL-PYRROLO[2,3-c]PYRIDIN-3-YL)ETHYL] ACETAMIDE

### EXEMPLE 58 : N-[2-(1H-PYRROLO[3,2-b]PYRIDIN-3-YL)ETHYL]ACETAMIDE

### EXEMPLE 59 : N-[2-[1,4-DIMETHYL-PYRROLO[2,3-b]PYRIDIN-3-YL)ETHYL] ACETAMIDE

### EXEMPLE 60 : N-[2-(1,2-DIMETHYL-PYRROLO[2,3-b]PYRIDIN-3-YL)ETHYL] ACETAMIDE

### EXEMPLE 61 : N-[2-(1-PHENYL-PYRROLO[2,3-b]PYRIDIN-3-YL)BUTYL]ACETAMIDE

### EXEMPLE 62 : N-[2-(5-METHOXY-1-METHYL-2-PHENYL-PYRROLO[2,3-b]PYRIDIN-3-YL)ETHYL]ACETAMIDE

### EXEMPLES 63 A 71 :

En utilisant les méthodes de synthèse décrites précédemment, mais en utilisant les réactifs appropriés, on obtient les composés des exemples suivants :

### EXEMPLE 63 : N-[2-(5-ETHOXY-1-METHYL-PYRROLO[2,3-b]PYRIDIN-3-YL)ETHYL] ACETAMIDE

### EXEMPLE 64 : N-[2-(5-PROPOXY-1-METHYL-PYRROLO[2,3-b]PYRIDIN-3-YL)ETHYL] ACETAMIDE

### EXEMPLE 65 : N-[2-(5-BUTYLOXY-1-METHYL-PYRROLO[2,3-b]PYRIDIN-3-YL)ETHYL] ACETAMIDE

### EXEMPLE 66 : N-[2-(5-ALLYLOXY-1-METHYL-PYRROLO[2,3-b]PYRIDIN-3-YL)ETHYL] ACETAMIDE

### EXEMPLE 67 : N-[2-(5-(BUT-2-ENYLOXY)-1-METHYL-PYRROLO[2,3-b]PYRIDIN-3-YL) ETHYL]ACETAMIDE

### EXEMPLE 68 : N-[2-(5-(PROP-2-ENYLOXY)-1-METHYL-PYRROLO[2,3-b]PYRIDIN-3-YL)ETHYL] ACETAMIDE

### EXEMPLE 69 : N-[2-(5-METHOXY-1-METHYL-2-ETHYL-PYRROLO[2,3-b]PYRIDIN-3-YL)ETHYL] ACETAMIDE

### EXEMPLE 70 : N-[2-(5-METHOXY-1-METHYL-2-BENZYL-PYRROLO[2,3-b]PYRIDIN-3-YL)ETHYL]ACETAMIDE

### EXEMPLE 71 : N-[2-(5-METHOXY-1-METHYL-2-(4-FLUOROBENZYL)-PYRROLO[2,3-b]PYRIDIN-3-YL)ETHYL] ACETAMIDE

### EXEMPLE 72 : N-[2-(2-PHENYL-1H-PYRROLO[2,3-b]PYRIDIN-3-YL)-ETHYL] ACETAMIDE

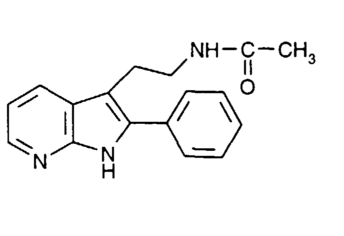

Sous argon et en milieu sec, la préparation 11 dans le dichlorométhane (3 cm³), quelques gouttes de 1,4-dioxanne et en présence de pyridine (0.030 cm³), est ajoutée, à 0°C et lentement, l'anhydride acétique (0.015 cm³ ; 0.15 mmol). Après 3 heures d'agitation à température ambiante, la réaction est hydrolysée par l'eau et extraite par l'acétate d'éthyle ; la phase organique est lavée par l'eau (2 fois) et séchée sur sulfate de magnésium. Après évaporation des solvants et purification sur gel de silice (éluant : dichlorométhane : méthanol, 98 : 2), 30 mg de solide blanc sont obtenus.
Rendement : 57 % sur 2 étapes
F : 217°C
IR (KBr) : ν = 3642 cm⁻¹ (NH), ν = 1636 cm⁻¹ (C=O).
RMN¹H (DMSO-d₆) : δ 1.75 (s, 3H, CH₃), 2.95 (t, 2H, J = 7.7 Hz, CH₂-CH₂-NH) 3.31 (q, 2H, J = 7.7 Hz, CH₂-NH), 7.07 (dd, 1H, J₅₋₆ = 5.4 Hz, J₅₋₄ = 8.7 Hz, H-5), 7.39 (m, 1H, aromatique), 7.50 (t, 2H, J = 8.5 Hz, aromatiques), 7.68 (d, 2H, J = 8.5 Hz, aromatiques), 7.96 (d, 1H, J₄₋₅ = 8.7 Hz, H-4), 7.98 (t, 1H, J = 7.7 Hz, NH-COCH₃), 8.21 (d, 1H, J₆₋₅ = 5.4 Hz, H-6), 11.75 (s, 1H, H-1).
MS m/z 280 (M+1)

### EXEMPLE 73 : N-[2-(5-BROMO-1H-PYRROLO[2,3-b]PYRIDIN-3-YL)-ETHYL] ACETAMIDE

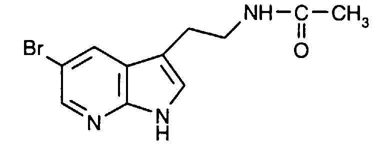

A une suspension du composé obtenu dans la préparation 12 dans le dichlorométhane (5 cm³) et quelques gouttes de 1,4-dioxanne sous argon, à 0°C, la pyridine (0.064 cm³) puis l'anhydre acétique (0.032 cm³ ; 0.34 mmol) sont ajoutés ; la réaction est agitée 2 h à température ambiante. Le milieu est hydrolysé par l'eau et extrait par l'acétate d'éthyle (2 fois). Après séchage sur sulfate de magnésium et évaporation des solvants, le résidu est purifié par chromatographie sur gel de silice (éluant : dichlorométhane : méthanol, 98 : 2) pour donner un solide blanc (60 mg).
Rendement : 51 % sur deux étapes
F : 209°C
IR (KBr) : ν = 3264 et 3151 cm⁻¹ (NH), ν = 1643 cm⁻¹ (C=O).
RMN¹H (DMSO-d₆): δ 1.77 (s, 3H, COCH₃), 2.77 (t, 2H, J = 7.4 Hz, CH₂), 3.28 (q, 2H, J = 7.4 Hz, CH₂-NH), 7.33 (s, 1H, H-2), 7.88 (t, 1H, J = 7.4 Hz, NH-CO-), 8.16 (d, 1H, J = 2.6 Hz, H-4), 8.22 (d, 1H, J = 2.6 Hz, H-6), 11.59 (s, 1H, H-1).
MS m/z : 283 (M+1)

### EXEMPLE 74 : N-[2-(PYRROLO[2,3-b]PYRIDIN-1 yl)-ETHYL]ACETAMIDE

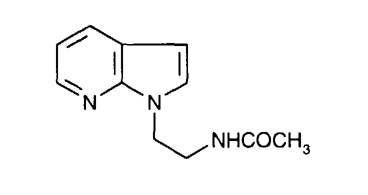

Le composé obtenu à la préparation 13 (500 mg, 310 mmol) est dissous dans 6 cm³ de dichlorométhane. On additionne 0,752 cm³ de pyridine et 0,879 cm³ d'anhydride acétique dans la glace. On laisse agir 2 h. On hydrolyse à l'eau et on extrait au dichlorométhane. On incorpore le dichlorométhane et on évapore la pyridine avec le toluène. Le produit est purifié sur colonne de silice (Eluant CH₂Cl₂ // CH₂Cl₂ / MeOH 5 %) puis recristallisé dans le cyclohexane.
Rendement : 73 %
F : 85°C
RMN¹H (CDCl₃) : δ 2.15 (s, 3H, COCH₃), 3.65-3.75 (m, 2H, CH₂-NH), 4.41-4.50 (m, 2H, N-CH₂), 6.50 (d, 1H, H₃), 6.72-6.80 (m, 1H, N-H), 7.12 (dd, 1H, H_{5 arom}), 7.22 (d, 1H, H₂), 7.98 (d, 1H, H₄), 8.32 (d, 1H, H₆)
MS m/z : 204 (M+1)
IR : ν = 3302 cm⁻¹ (NH), ν = 1635 cm⁻¹(C=O).

### EXEMPLE 75 : N-[2-(5-METHOXY-FURO[3,2-b]PYRIDIN-3-YL)-ETHYL]ACETAMIDE

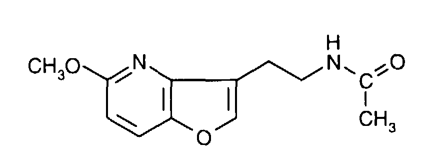

A 310 mg (1,65 mmol) du composé de la préparation 14 en solution dans 25 cm³ d'anhydride acétique, additionner 43 mg (8.24.10⁻⁴ mmol) de Nickel de Raney préalablement lavé dans l'éthanol puis dans l'anhydride acétique ainsi que 203 mg (2,47 mmol) d'acétate de sodium. Maintenir le milieu sous atmosphère d'hydrogène pendant 4 h 45 à 50°C, puis évaporer le solvant. Reprendre le résidu par H₂O et extraire au CH₂Cl₂. Après séchage sur MgSO₄, concentrer la phase organique. Purifier le produit sur colonne de silice, éluant Acétate d'éthyle. On obtient 290 mg d'un solide blanc (F = 85 °C) qui est recristallisé dans le cyclohexane.
Rendement : 75 %
IR : 3312 cm⁻¹ (NH) ; 1632 cm⁻¹ (C=O) ; 1620 cm⁻¹ (C=O)
RMN¹H (CDCl₃) : 2.01 (s, 3H, CH₃), 2.94 (t, 2H, CH₂-CH₂-NH), 3.02 (q, 2H, CH₂-CH₂-NH), 4.19 (s, 3H, CH₃-O), 5.35-5.55 (massif, 1H, NH), 7.18 (d, 1H, J = 5.5 Hz, H₂), 7.99 (d, 1H, J = 5.5 Hz, H₃).
MS m/z : 235 (M+1)

### EXEMPLES 76 A 80 :

En procédant de façon analogue mais en utilisant les réactifs appropriés aux modes opératoires décrits dans les exemples précédents, on obtient les composés des exemples suivants.

### EXEMPLE 76 : N-[2-(7-METHOXY-FURO[2,3-c]PYRIDIN-3-YL)-ETHYL] ACETAMIDE

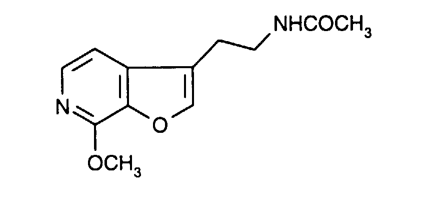

A 176 mg (0,935 mmol) du composé obtenu dans la préparation 15 dissous dans 10 cm³ d'anhydride acétique, on additionne successivement 27 mg (0,467 mmol) de nickel de Raney et 115 mg (1,4 mmol) d'acétate de sodium. L'agitation est maintenue 4 h à 50°C sous atmosphère d'hydrogène. Après retour à température ambiante, le catalyseur est filtré sur célite. Après évaporation à sec, le produit est hydrolysé avec 25 cm³ d'eau extrait au CH₂Cl₂. La phase organique est séchée sur MgSO₄ puis évaporée sous vide. Le produit est purifié par chromatographie sur colonne de silice (éluant : CH₂Cl₂ , MeOH : 95 : 5). On récupère 176 mg de produit sous forme d'un solide.
Rendement: : 82 %
F : 99-100°C
IR (KBr) : 3030 cm⁻¹ (NH) ; 1610 cm⁻¹ (C=O) ; 1080 cm⁻¹ (C-O-C)
RMN ¹H (CDCl₃ + D₂O) : δ 1.89 (s, 3H, CH₃), 2.83 (t, 2H, J = 6.6 Hz, CH₂), 3.50 (t, 2H, J = 6.6 Hz, CH₂), 4.07 (s, 3H, O-CH₃), 7.06 (d, 1H, J = 5.5 Hz, Hpyr), 7.48 (s, 1H, CH), 7.87 (d, 1H, J = 5.5 Hz, H_{pyr}).

### EXEMPLE 77 : N-[2-(5-METHOXY-2-PHENYL-PYRROLO[3,2-c]PYRIDIN-3-YL)-ETHYL] ACETAMIDE

### EXEMPLE 78 : N-[2-(2-TRYMETHYLSILYL-PYRROLO[3,2-c]PYRIDIN-3-YL)] ACETAMIDE

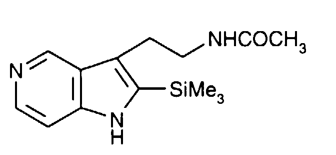

Sous argon et en milieu anhydre, les composé des préparations 16 (200 mg ; 0.91 mmol) et 17 (334 mg ; 1.82 mmol) en présence de triéthylamine (0.380 cm³ ; 2.7 mmol) et de chlorure de bistriphénylphosphinepalladium (Il) (64 mg ; 0.091 mmol) dans l'hexaméthylphosphotriamide (4 cm³) sont agités à 100°C pendant 15 h ; la réaction ayant peu évoluée, 64 mg de chlorure de bistriphénylphosphinepalladium (Il) et 0.380 cm³ de triéthylamine sont ajoutés au milieu. La réaction est agitée 22 h à 100°C avant d'être diluée dans l'acétate d'éthyle et le méthanol ; la phase organique est lavée par l'eau (6 fois), séchée sur sulfate de magnésium et évaporée. La purification du résidu sur gel de silice (éluant : dichlorométhane : méthanol, 85 : 15) permet d'obtenir 75 mg de solide beige.
Rendement : 30 %
F: décomposition au-dessus de 75°C
IR (KBr) : ν : 3253 cm⁻¹ (NH) ; ν : 1653 cm⁻¹ (C=O)
RMN¹H (DMSO-d₆): δ 0.37 (s, 9H, Si(CH₃)₃), 1.79 (s, 3H, COCH₃), 2.94 (t, 2H, J = 6.6 Hz, CH₂-N), 3.23 (q, 2H, J = 6.6 Hz, Ar-CH₂), 7.40 (d, 1H, J₇₋₆ = 5.8 Hz, H-7), 8.03 (t, 1H, J = 6.6 Hz, NH-CO), 8.15 (d, 1H, J₆₋₇ = 5.8 Hz, H-6), 8.90 (s, 1H, H-4), 11.20 (s,1H, H-1).
MS m/z : 276 (M+1)

### EXEMPLE 79 : N-[2-(5-PHENYL-PYRROLO[2,3-b]PYRIDIN-3-YL)ETHYL] ACETAMIDE F : 181°C

### EXEMPLE 80 : N-[2-(5-METHOXY-THIENO[3,2-b]PYRIDIN-3-YL)ETHYL] ACETAMIDE

### ETUDE PHARMACOLOGIQUE

### EXEMPLE A : ETUDE DE LA TOXICITE AIGUE

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée.

La DL 50 des produits testés est supérieure à 1 000 mg.kg⁻¹ pour la plupart des composés étudiés ce qui indique la faible toxicité des composés de l'invention.

### EXEMPLE B : ETUDE DE LIAISON AUX RECEPTEURS DE LA MELATONINE

### B1) Etude sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology 1989, vol. (1), pp 1-4).

### PROTOCOLE

1) Les membranes de pars tuberalis de mouton sont préparées et utilisées comme tissu cible dans des expériences de saturation pour déterminer les capacités et affinités de liaison pour la 2-[¹²⁵I] - iodomélatonine.
2) Les membranes de pars tuberalis de mouton sont utilisées comme tissu cible, avec les différents composés à tester, dans des expériences de liaison compétitive par rapport à la 2-iodomélatonine.

Chaque expérience est réalisée en triple et une gamme de concentrations différentes est testée pour chaque composé.

Les résultats permettent de déterminer, après traitement statistique, les affinités de liaison du composé testé.

### RESULTATS

II apparaît que les composés de l'invention possèdent une puissante affinité pour les récepteurs de la mélatonine supérieure à celle de la mélatonine elle-même.

### B2) Etude sur des membranes de cellules du cerveau de poulet (Gallus domesticus)

Les animaux utilisés sont des poulets (Gallus domesticus) agés de 12 jours. Ils sont sacrifiés entre 13 et 17 heures le jour de leur arrivée. Les cerveaux sont rapidement prélevés et congelés à - 200°C puis conservés à - 80°C. Les membranes sont préparées selon la méthode décrite par Yuan et Pang (Journal of Endocrinology 1991, 128, pp 475-482). La 2-[¹²⁵I]-iodomélatonine est incubée en présence des membranes dans une solution tamponnée à pH 7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman® LS 6000.

Les produits utilisés sont :
- 2-[¹²⁵I]-iodomélatonine
- mélatonine
- produits courants
- composés testés

En screening primaire, les molécules sont testées à 2 concentrations (10⁻⁷ et 10⁻⁵M). Chaque résultat est la moyenne de n=3 mesures indépendantes. Les composés testés font l'objet d'une détermination quantitative de leur efficacité (IC₅₀). Ils sont utilisés à 10 concentrations différentes.

Ainsi les valeurs d'IC₅₀ trouvées pour les composés préférés de l'invention, qui correspondent aux valeurs de l'affinité montrent que la liaison des composés testés est très puissante.

### EXEMPLE C : TEST DES QUATRE PLAQUES

Les produits de l'invention sont administrés par voie oesophagienne à des lots de dix souris. Un lot reçoit du sirop de gomme. 30 min après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passage ce qui montre l'activité anxiolytique des dérivés de l'invention.

### EXEMPLE D: COMPOSES DE L'INVENTION SUR LES RYTHMES CIRCADIENS D'ACTIVITE LOCOMOTRICE DU RAT

L'implication de la mélatonine dans l'entraînement, par l'alternance jour / nuit, de laplupart des rythmes circadiens physiologiques, biochimiques et comportementaux a permis d'établir un modèle pharmacologique pour la recherche de ligands mélatoninergiques.

Les effets des molécules sont testés sur de nombreux paramètres et en particulier sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### PROTOCOLE EXPERIMENTAL

Des rats mâles Long Evans âgés de un mois sont soumis, dès leur arrivée au laboratoire, à un cycle lumineux de 12 h de lumière par 24 h (LD 12 : 12).

Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité grâce par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens.

### RESULTATS :

Il apparaît clairement que les composés de l'invention permettent d'agir de façon puissante sur le rythme circadien via le système mélatoninergique.

### EXEMPLE E : ACTIVITE DES PRODUITS DE L'INVENTION SUR LA MICROCIRCULATION ISCHEMIQUE

L'étude expérimentale a été réalisée sur les muscles crémasters de rats mâles (Sprague-Dawley) après ligature de l'artère iliaque commune.

Les muscles ont été placés dans une chambre transparente, perfusés par une solution de tampon bicarbonate équilibrée par un mélange gazeux CO₂/N₂ 5/95%. La vélocité des globules rouges et le diamètre des artérioles de premier ou second ordre irriguant le crémaster ont été mesurés, le flux sanguin artériolaire a été calculé. Des informations identiques ont été obtenues pour quatre types de veinules.
On a effectué le même type de mesure simultanément :
- sur le crémaster perfusé normalement,
- sur le crémaster sous ligature, c'est à dire le crémaster ischémié 2, 7, 14 et 21 jours après ligature.
Deux groupes d'animaux ont été étudiés :
- un groupe témoin sans traitement,
- un groupe traité per os par un produit de l'invention, à raison de 0,1 mg.kg⁻¹ par jour.

On n'a constaté aucune différence dans la vélocité des globules ni dans le diamètre des vaisseaux dans les muscles crémasters normalement irrigués chez les animaux traités par rapport aux témoins.
Par contre, au niveau du muscle crémaster ischémié, le diamètre moyen des artérioles était amélioré chez les animaux traités par rapport aux témoins. La vélocité des globules rouges était normalisée par un traitement de 21 jours.

En fait, chez les animaux traités, la vélocité des globules rouges et le débit sanguin mesurés 7 jours après la ligature, ne présentent pas de différence significative avec les valeurs obtenues dans le crémaster non ischémié. Ces résultats sont obtenus sans modification de la pression artérielle.

Ces résultats indiquent que le traitement chronique par un composé de l'invention améliore la microcirculation et l'irrigation sanguine des territoires ischémiés.

### EXEMPLE F : STIMULATION DES REPONSES IMMUNITAIRES

A des groupes de six souris on a administré des globules rouges de moutons. Ces groupes de souris ont ensuite été traités par voie sous cutanée par les composés de l'invention pendant six jours et un groupe témoin a été traité par un placébo. Les souris sont ensuite laissées au repos pendant quatre semaines puis ont ensuite reçu une injection de rappel de globules rouges de mouton sans recevoir de nouvelles administrations de produit de l'invention. La réponse immunitaire a été évaluée 3 jours après l'injection de rappel. Elle est statistiquement accrue dans le groupe traité par les composés de l'invention.

### EXEMPLE G : ACTIVITE ANTIARRYTHMIQUE

### PROTOCOLE:

(ref : LAWSON J.W. et al. J. Pharmacol. Exper. Therap. 1968, 160, pp 22-31)

La substance testée est administrée en intrapéritonéal à un groupe de 3 souris 30 min avant l'exposition à une anesthésie par le chloroforme. Les animaux sont ensuite observées pendant 15 min. L'absence d'enregistrement d'arrythmies et de fréquences cardiaques supérieures à 200 battements/min (témoin : 400-480 battements/min) chez deux animaux au moins indique une protection significative.

### EXEMPLE H : COMPOSITION PHARMACEUTIQUE : COMPRIMES

1000 comprimés dosés à 5 mg de N-[2-(5-méthoxy-1-méthyl-pyrrolo[2,3-*b*]pyridin-3-yl) éthyl]acétamide

| | |
|---|---|
| N-[2-(5-méthoxy-1-méthyl-pyrrolo[2,3-b]pyridin-3-yl)éthyl]acétamide | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 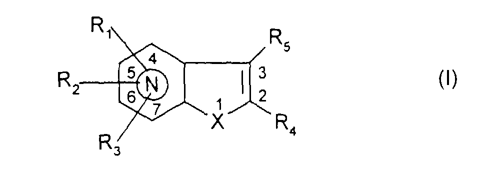 dans laquelle
• R₁, R₂, R₃ et R₄ représentent, chacun indépendamment l'un de l'autre, un hydrogène ou un radical choisi parmi halogène, hydroxy, Ra et -O-Ra ; avec Ra choisi parmi alkyl, alkyl substitué par un ou plusieurs halogènes, trialkylsilyl, alcényl, alcynyl, cycloalkyl, cycloalkylalkyl, aryl, aryl substitué, arylalkyl et arylalkyl substitué ;
• R₅ représente un groupement de formule -A-B-Y dans lequel
- A représente une chaîne (C₁-C₆) alkylène non substituée ou substituée par un ou plusieurs alkyles,
- B représente un groupement B₁, B₂ ou B₃: 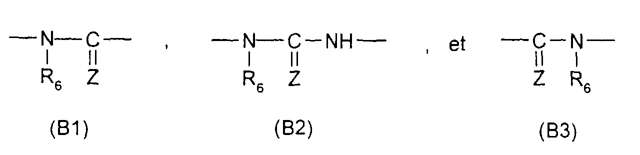 dans lesquels Z représente un oxygène ou un soufre et R₆ représente un hydrogène ou un radical choisi parmi alkyl, cycloalkyl, cycloalkylalkyl, aryl et arylalkyl,
- Y représente un radical Y₁ choisi parmi alkyl, alkyl substitué par un ou plusieurs halogènes, alcényl, alcynyl, cycloalkyl et cycloalkylalkyl ; Y peut également représenter un hydrogène lorsque B représente un groupement B₁ ou B₂,
• et X représente un oxygène, un soufre ou un groupement 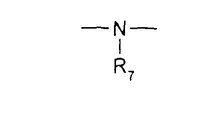 dans lequel R₇ représente un hydrogène ou un radical choisi parmi alkyl, aryl, aryl substitué, arylalkyl et arylalkyle substitué ; ou R₇ représente un groupement de formule -A-B-Y tel que défini précédemment et dans ce cas R₅ représente une valeur choisie parmi celles définies pour R₁, R₂, R₃ et R₄, telles que définies précédemment,
avec la réserve
- que le composé de formule (I) ne peut être le N-[2-(1H-pyrrrolo[3,2-*c*]pyridin-3-yl)éthyl]acétamide,
- et que R₇ ne peut pas représenter un phényl lorsque l'azote du noyau pyridine de la formule (I) est en position 7 de l'hétérocycle, R₁ est un groupement alkyle en position 4 de l'hétérocycle et R₂, R₃ et R₄ représentent des hydrogènes,
étant entendu que :
- les termes "alkyl" et "alkoxy" désignent des groupements linéaires ou ramifiés contenant de 1 à 6 atomes de carbone,
- les termes "alcényl" et "alcynyl" désignent des groupements insaturés linéaires ou ramifiés contenant de 2 à 6 atomes de carbone,
- le terme "cycloalkyl" désigne un groupement de 3 à 8 atomes de carbone,
- le terme "aryl" désigne un radical phényl, naphthyl ou pyridyl,
- le terme "substitué" affecté aux expressions "aryl" et "arylalkyl" signifie que ces groupements peuvent être substitués sur les noyaux aromatiques par un ou plusieurs radicaux choisis parmi halogène, alkyl, alkoxy, hydroxy et alkyl substitué par un ou plusieurs halogènes ;
leurs énantiomères et diastéréoisomères, et leurs sels d'addition à un acide ou une base pharmaceutiquement acceptable.

2. Composés de formule (I) selon la revendication 1 répondant aux formules (12) et (13) suivantes : 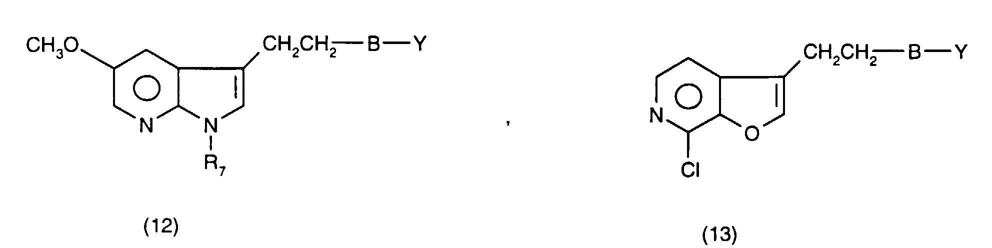 dans lesquelles R₇, B et Y sont tels que définis dans la revendication 1.

3. Composé selon la revendication 1 qui est le N-[2-(5-METHOXY-1-METHYL-PYRROLO [2,3-*b*]PYRIDIN-3-YL)ETHYL]ACETAMIDE.

4. Composé selon la revendication 1 qui est le N-[2-(7-CHLORO-FURO[2,3-*c*]PYRIDIN-3-YL)ETHYL]ACETAMIDE.

5. Procédé de préparation des composés de formule (I) selon la revendication 1 caractérisé en ce que, lorsque B représente un groupement B₁ ou B₂ tels que définis dans la revendication 1, on fait réagir une amine de formule (II) : 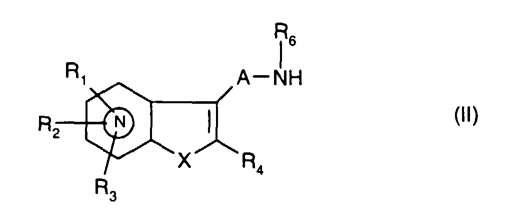 dans laquelle R₁, R₂, R₃, R₄, R₆, A et X sont tels que définis dans la revendication 1
- soit avec un composé de formule (IIIa) ou (IIIb) : 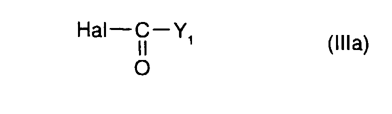 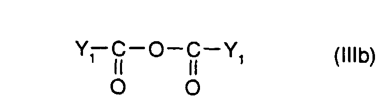 l'anhydride (IIIb) pouvant être mixte ou symétrique,
dans lesquelles Y₁ est tel que défini dans la revendication 1 est Hal représente un atome d'halogène afin d'obtenir les composés de formule (I/a) : 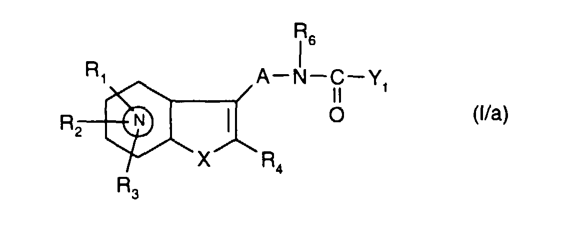 dans laquellle R₁, R₂, R₃, R₄, R₆, A, Y₁ et X sont tels que définis précédemment,
composé de formule (I/a) qui est ensuite soumis au réactif de Lawesson pour obtenir les composés de formule (I/b) : 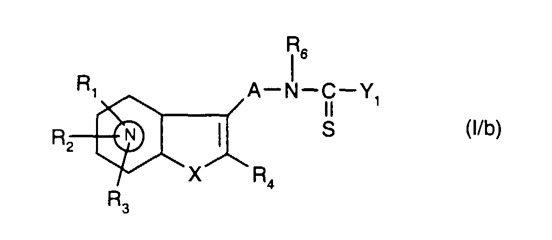 dans laquelle R₁, R₂, R₃, R₄, R₆, A, Y₁ et X sont tels que définis précédemment,
- soit avec l'acide formique pour obtenir un composé de formule (I/c) : 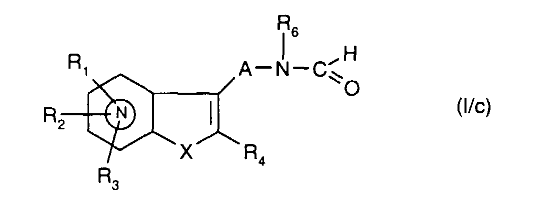 dans laquelle R₁, R₂, R₃, R₄, R₆, A et X sont tels que définis précédemment,
- soit avec un composé de formule (IV) :
Z=C=N-Y (IV)
dans laquelle Y et Z sont tels que définis dans la revendication 1 afin d'obtenir les composés de formule (I/d) : 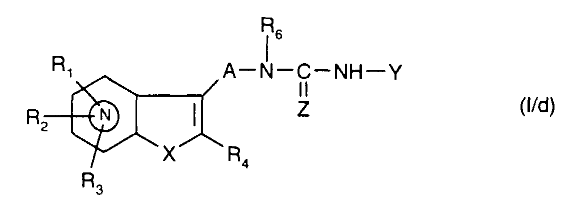 dans laquelle R₁, R₂, R₃, R₆, A, X, Y et Z sont tels que définis précédemment,
et caractérisé en ce que, lorsque B représente un groupement B₃ tel que défini dans la revendication 1 on fait réagir un composé de formule (V) : 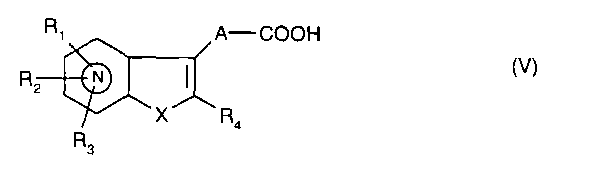 dans laquelle R₁, R₂, R₃, R₄, A et X sont tels que définis dans la revendication 1 avec une amine de formule (VI) : 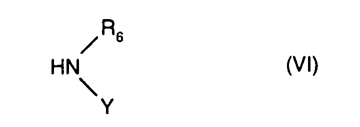 dans laquelle R₆ et Y sont tels que définis dans la revendication 1 pour obtenir le composé de formule (I/e) : 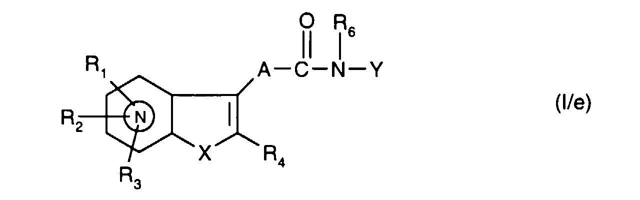 dans laquelle R₁, R₂, R₃, R₄, R₆, A, X et Y sont tels que définis précédemment,
composés de formule (I/e) qui sont soumis au réactif de Lawesson pour obtenir les composés de formule (I/f) : 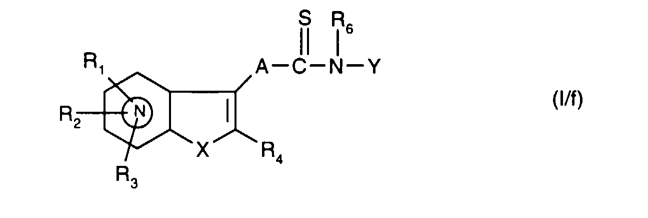 dans laquelle R₁, R₂, R₃, R₄, R₆, A, X et Y sont tels que définis précédemment,
étant entendu que, lorsque R₇ tel que défini dans la revendication 1 représente un groupement de formule -A-B-Y tel que défini dans la revendication 1, le procédé de préparation est analogue à celui décrit ci-dessus, les réactifs de formule (Illa), (IIIb) et (IV) et l'acide formique d'une part ou (VI) d'autre part réagissant respectivement sur des groupements de formule -A-NH-R₆ ou -A-COOH, fixés en position 1 de l'hétérocycle pyrrolopyridine présent dans la revendication 1, les composés de formule (I) obtenus pouvant être :
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

6. Procédé de préparation des composés de formule (I) selon la revendication 1 caractérisé en ce qu'on condense un composé de formule (VII) 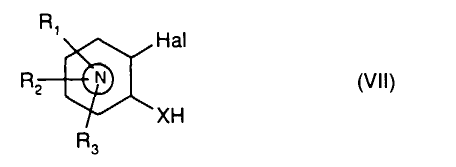 dans laquelle R₁, R₂, R₃ et X sont tels que définis dans la revendication 1 et Hal représente un atome d'halogène
avec un composé de formule (VIII) : 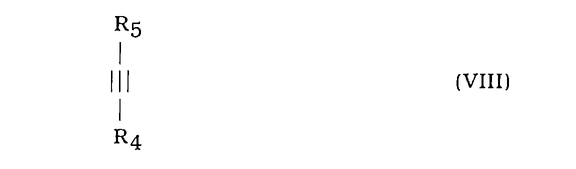 dans laquelle R₄ et R₅ sont tels que définis dans la revendication 1,
les composés de formule (I) obtenus pouvant être :
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

7. Procédé de préparation des composés de formule (I/i), cas particuliers des composés de formule (I) selon la revendication 1 : 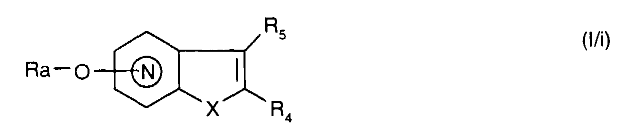 dans laquelle Ra, R₄, R₅ et X sont tels que définis dans la revendication 1 par greffage du radical Ra sur un composé de formule (I/j) : 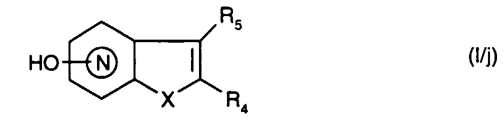 dans laquelle R₄, R₅ et X sont tels que définis précédemment,
composés de formule (I/i) qui sont, le cas échéant
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou un base pharmaceutiquement acceptable.

8. Procédé de préparation des composés de formule (I/k), cas particulier des composés de formule (I), selon la revendication 1 : 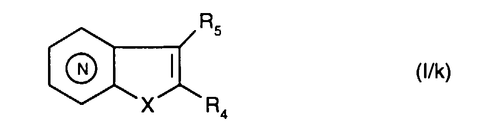 dans laquelle R₄, R₅ et X sont tels que définis dans la revendication 1, par traitement acide d'un composé de formule (I/ℓ), cas particulier des composés de formule (I) selon la revendication 1: 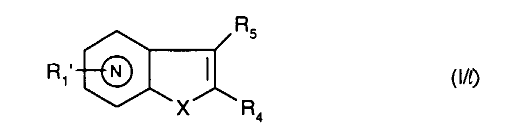 dans laquelle R₄, R₅ et X sont tels que définis précédemment et R_{1'} est un atome d'halogène,
composés de formule (I/k) qui sont, le cas échéant
- purifiés suivant une ou plusieurs méthodes de purification choisies parmi la cristallisation, la chromatographie, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou un base pharmaceutiquement acceptable.

9. Compositions pharmaceutiques contenant un composé de formule (I) selon la revendication 1, ou un de ses sels d'addition à un acide ou une base pharmaceutiquement acceptable, en combinaison avec un ou plusieurs excipients pharmaceutiquement acceptables.

10. Compositions pharmaceutiques selon la revendication 9, utiles pour le traitement des troubles du système mélatoninergique.

## Patentansprüche

1. Verbindungen der Formel (I): 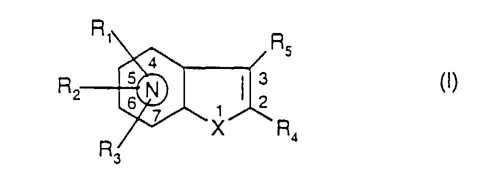 in der
• R₁, R₂, R₃ und R₄ jeweils unabhängig voneinander ein Wasserstoffatom oder eine Gruppe ausgewählt aus Halogen, Hydroxy, Ra und -O-Ra darstellen; worin Ra ausgewählt ist aus Alkyl, Alkyl substituiert durch ein oder mehrere Halogene, Trialkylsilyl, Alkenyl, Alkinyl, Cycloalkyl, Cycloalkylalkyl, Aryl, substituiertem Aryl, Arylalkyl und substituiertem Arylalkyl;
• R₅ eine Gruppe der Formel -A-B-Y darstellt, worin
- A eine (C₁-C₆)-Alkylenkette darstellt, die nicht substituiert oder durch eine oder mehrere Alkylgruppen substituiert ist,
- B eine Gruppe B₁, B₂ oder B₃ darstellt: 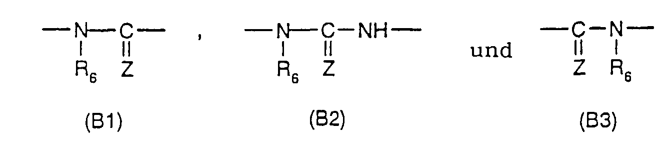 worin Z Sauerstoff oder Schwefel und R₆ Wasserstoff oder eine Gruppe ausgewählt aus Alkyl, Cycloalkyl, Cycloalkylalkyl, Aryl und Arylalkyl bedeuten,
- und Y eine Gruppe Y₁ bedeutet ausgewählt aus Alkyl, Alkyl substituiert durch ein oder mehrere Halogene, Alkenyl, Alkinyl, Cycloalkyl und Cycloalkylalkyl; worin Y auch Wasserstoff bedeuten kann, wenn B eine Gruppe B₁ oder B₂ darstellt,
• und X Sauerstoff, Schwefel oder eine Gruppe 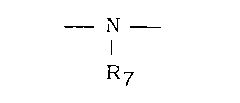 bedeutet, worin R₇ Wasserstoff oder eine Gruppe ausgewählt aus Alkyl, Aryl, substituiertem Aryl, Arylalkyl und substituiertem Arylalkyl darstellt; oder R₇ eine Gruppe der Formel -A-B-Y bedeutet, wie sie oben definiert worden ist, wobei in diesem Fall R₅ eine der Bedeutungen besitzt ausgewählt aus jenen, die für R₁, R₂, R₃ und R₄ oben definiert worden sind,
mit der Maßgabe,
- daß die Verbindung der Formel (I) nicht N-[2-(1H-Pyrrolo[3,2-c]pyridin-3-yl)-ethyl]-acetamid ist
- und daß R₇ nicht Phenyl bedeutet, wenn das Stickstoffatom des Pyridinkerns der Formel (I) in der 7-Stellung des Heterocyclus steht, R₁ eine Alkylgruppe in der 4-Stellung des Heterocyclus ist und R₂, R₃ und R₄ Wasserstoff bedeuten,
wobei es sich versteht, daß:
- die Begriffe "Alkyl" und "Alkoxy" für geradkettige oder verzweigte Gruppe stehen, die 1 bis 6 Kohlenstoffatome aufweisen,
- die Begriffe "Alkenyl" und "Alkinyl" für geradkettige oder verzweigte ungesättigte Gruppen stehen, die 2 bis 6 Kohlenstoffatome aufweisen,
- der Begriff "Cycloalkyl" für eine Gruppe mit 3 bis 8 Kohlenstoffatomen steht,
- der Begriff "Aryl" für eine Phenyl-, Naphthyl- oder Pyridylgruppe steht,
- der Begriff "substituiert", wenn er für die Begriffe "Aryl" und "Arylalkyl" benutzt wird, bedeutet, daß diese Gruppen an den aromatischen Kernen durch einen oder mehrere Reste ausgewählt aus Halogen, Alkyl, Alkoxy, Hydroxy und durch ein oder mehrere Halogene substituiertem Alkyl substituiert sein können;
deren Enantiomere und Diastereoisomere und deren Additionssalze mit einer pharmazeutisch annehmbaren Säure oder Base.

2. Verbindungen der Formel (I) nach Anspruch 1 der folgenden Formeln (12) und (13): 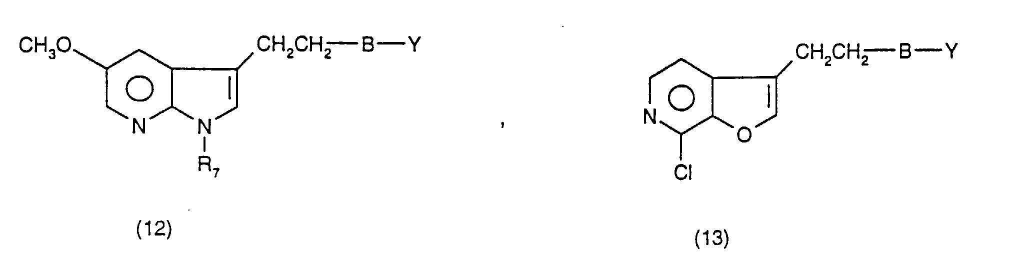 worin R₇, B und Y die in Anspruch 1 angegebenen Bedeutungen besitzen.

3. Verbindung nach Anspruch 1, nämlich N-[2-(5-Methoxy-1-methyl-pyrrolo-[2,3-*b*]pyridin-3-yl)-ethyl]-acetamid.

4. Verbindung nach Anspruch 1, nämlich N-[2-(7-Chlor-furo[2,3-*c*]pyridin-3-yl)-ethyl]-acetamid.

5. Verfahren zur Herstellung der Verbindungen der Formel (I) nach Anspruch 1, dadurch gekennzeichnet, daß, wenn B eine Gruppe B₁ oder B₂ darstellt, wie sie in Anspruch 1 definiert worden sind, man ein Amin der Formel (II): 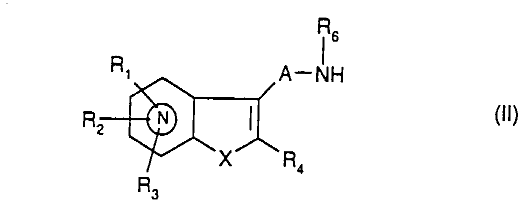 in der R₁, R₂, R₃, R₄, R₆, A und X die in Anspruch 1 angegebenen Bedeutungen besitzen,
- entweder mit einer Verbindung der Formel (IIIa) oder (IIIb): 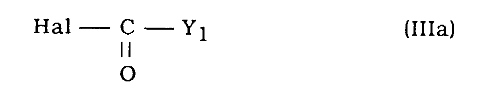 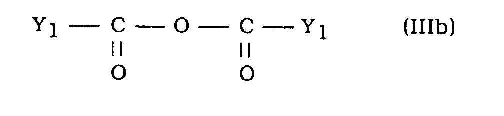 wobei das Anhydrid (IIIb) gemischt oder symmetrisch sein kann,
und worin Y₁ die in Anspruch 1 angegebenen Bedeutungen besitzt und Hal ein Halogenatom darstellt, umsetzt zur Bildung der Verbindungen der Formel (I/a): 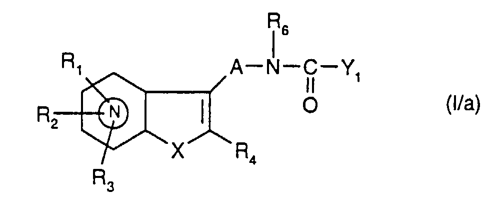 in der R₁, R₂, R₃, R₄, R₆, A, Y₁ und X die oben angegebenen Bedeutungen besitzen, welche Verbindung der Formel (I/a) anschließend der Einwirkung des Lawesson-Reagens unterworfen wird zur Bildung der Verbindungen der Formel (I/b): 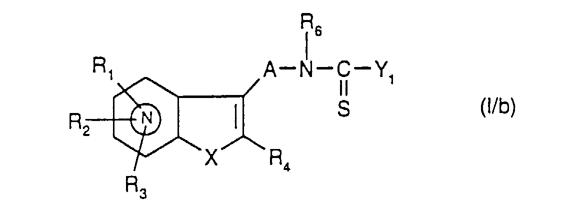 in der R₁, R₂, R₃, R₄, R₆, A, Y₁ und X die oben angegebenen Bedeutungen besitzen,
- oder mit Ameisensäure umsetzt zur Bildung einer Verbindung der Formel (I/c): 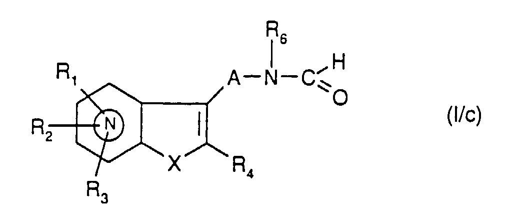 in der R₁, R₂, R₃, R₄, R₆, A und X die oben angegebenen Bedeutungen besitzen,
- oder mit einer Verbindung der Formel (IV):
Z = C = N - Y (IV)
in der Y und Z die in Anspruch 1 angegebenen Bedeutungen besitzen, umsetzt zur Bildung der Verbindungen der Formel (I/d): 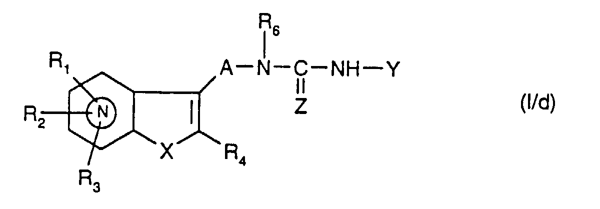 in der R₁, R₂, R₃, R₆, A, X, Y und Z die oben angegebenen Bedeutungen besitzen, und dadurch gekennzeichnet, daß, wenn B eine Gruppe B₃ darstellt, wie sie in Anspruch 1 definiert worden ist, man eine Verbindung der Formel (V): 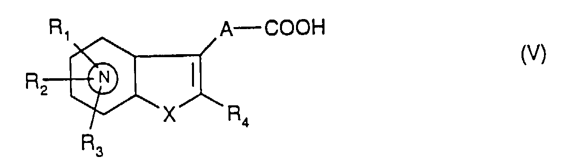 in der R₁, R₂, R₃, R₄, A und X die in Anspruch 1 angegebenen Bedeutungen besitzen, mit einem Amin der Formel (VI): 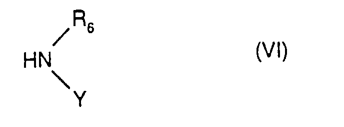 in der R₆ und Y die in Anspruch 1 angegebenen Bedeutungen besitzen, umsetzt zur Bildung der Verbindung der Formel (I/e): 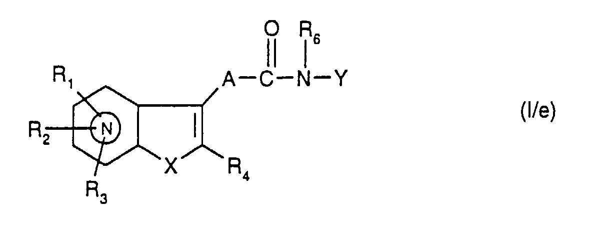 in der R₁, R₂, R₃, R₄, R₆, A, X und Y die oben angegebenen Bedeutungen besitzen, welche Verbindung der Formel (I/e) der Einwirkung des Lawesson-Reagens unterworfen wird zur Bildung der Verbindungen der Formel (I/f): 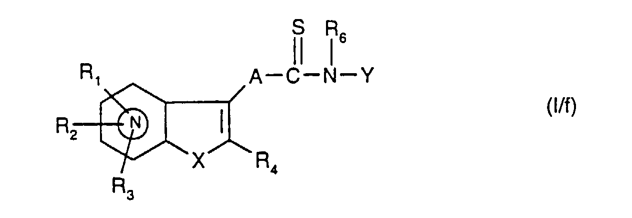 in der R₁, R₂, R₃, R₄, R₆, A, X und Y die oben angegebenen Bedeutungen besitzen,
wobei es sich versteht, daß, wenn R₇, wie es in Anspruch 1 definiert worden ist, eine Gruppe der Formel -A-B-Y darstellt, wie sie in Anspruch 1 definiert worden ist, das Verfahren analog zu dem oben beschriebenen ist, wobei die Reagenzien der Formeln (IIIa), (IIIb) und (IV) und die Ameisensäure einerseits oder (VI) andererseits auf die Gruppen der Formel -A-NH-R₆ oder -A-COOH, die an der 1-Stellung des in Anspruch 1 vorhandenen Pyrrolopyridin-Heterocyclus gebunden sind, einwirken,
wobei die erhaltenen Verbindungen der Formel (I):
- mit Hilfe einer oder mehrerer Reinigungsmethoden ausgewählt aus Kristallisation, Chromatographie, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung aufgetrennt werden können,
- oder mit Hilfe einer pharmazeutisch annehmbaren Säure oder Base in die Salze überführt werden können.

6. Verfahren zur Herstellung der Verbindungen der Formel (I) nach Anspruch 1, dadurch gekennzeichnet, daß man eine Verbindung der Formel (VII): 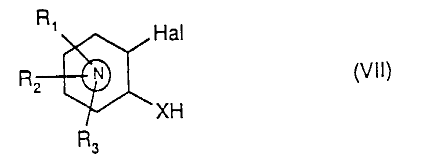 in der R₁, R₂, R₃ und X die in Anspruch 1 angegebenen Bedeutungen besitzen, und Hal ein Halogenatom darstellt,
mit einer Verbindung der Formel (VIII): 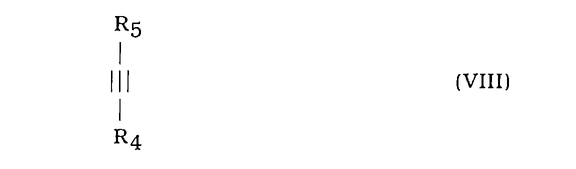 in der R₄ und R₅ die in Anspruch 1 angegebenen Bedeutungen besitzen, umsetzt, wobei die erhaltenen Verbindungen der Formel (I):
- mit Hilfe einer oder mehrerer Reinigungsmethoden ausgewählt aus Kristallisation, Chromatographie, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung aufgetrennt werden können,
- oder mit Hilfe einer pharmazeutisch annehmbaren Säure oder Base in die Salze überführt werden können.

7. Verfahren zur Herstellung der Verbindungen der Formel (I/i), einem Sonderfall der Verbindungen der Formel (I) nach Anspruch 1: 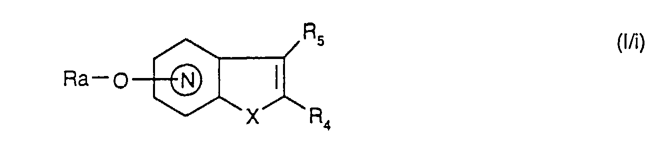 in der Ra, R₄, R₅ und X die in Anspruch 1 angegebenen Bedeutungen besitzen, durch Aufpfropfen des Rests Ra auf eine Verbindung der Formel (I/j): 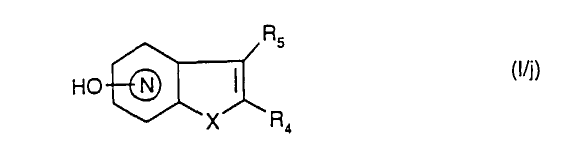 in der R₄, R₅ und X die oben angegebenen Bedeutungen besitzen,
welche Verbindungen der Formel (I/i) gegebenenfalls:
- mit Hilfe einer oder mehrerer Reinigungsmethoden ausgewählt aus Kristallisation, Chromatographie, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung aufgetrennt werden können,
- oder mit Hilfe einer pharmazeutisch annehmbaren Säure oder Base in die Salze überführt werden können.

8. Verfahren zur Herstellung der Verbindungen der Formel (I/k), einem Sonderfall der Verbindungen der Formel (I) nach Anspruch 1: 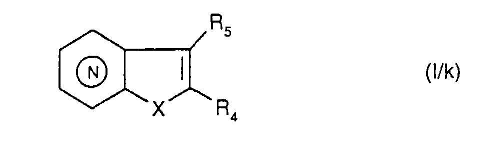 in der R₄, R₅ und X die in Anspruch 1 angegebenen Bedeutungen besitzen, durch saure Behandlung einer Verbindung der Formel (I/ℓ), einem Sonderfall der Verbindungen der Formel (I) nach Anspruch 1: 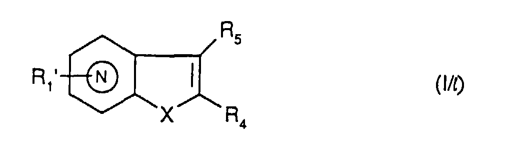 in der R₄, R₅ und X die oben angegebenen Bedeutungen besitzen und R₁' ein Halogenatom darstellt,
wobei die Verbindungen der Formel (I/k) gegebenenfalls
- mit Hilfe einer oder mehrerer Reinigungsmethoden ausgewählt aus Kristallisation, Chromatographie, Extraktion, Filtration und Überführung über Aktivkohle oder ein Harz gereinigt werden können,
- gegebenenfalls in ihre eventuellen Enantiomeren oder Diastereoisomeren in reiner Form oder in Form einer Mischung aufgetrennt werden können,
- oder mit Hilfe einer pharmazeutisch annehmbaren Säure oder Base in die Salze überführt werden können.

9. Pharmazeutische Zubereitungen enthaltend eine Verbindung der Formel(I) nach Anspruch 1 oder eines ihrer Additionssalze mit einer pharmazeutisch annehmbaren Base in Kombination mit einem oder mehreren pharmazeutisch annehbmaren Trägermaterialien.

10. Pharmazeutische Zubereitungen nach Anspruch 9 zur Behandlung von Störungen des melatoninergischen Systems.

## Claims

1. Compounds of formula (I): 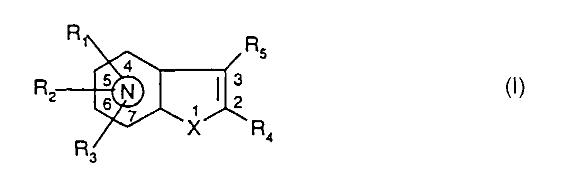 in which
• R₁, R₂, R₃ and R₄ each independently of the others represents a hydrogen atom or a radical selected from halogen, hydroxy, Ra and -O-Ra; wherein Ra is selected from alkyl, alkyl substituted by one or more halogen atoms, trialkylsilyl, alkenyl, alkynyl, cycloalkyl, cycloalkylalkyl, aryl, substituted aryl, arylalkyl and substituted arylalkyl;
• R₅ represents a group of the formula -A-B-Y wherein
- A represents a (C₁-C₆)alkylene chain which is unsubstituted or substituted by one or more alkyl radicals,
- B represents a group B₁, B₂ or B₃: 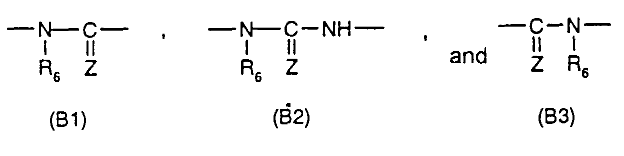 wherein Z represents an oxygen atom or a sulphur atom and R₆ represents a hydrogen atom or a radical selected from alkyl, cycloalkyl, cycloalkylalkyl, aryl and arylalkyl, and
- Y represents a radical Y₁ selected from alkyl, alkyl substituted by one or more halogen atoms, alkenyl, alkynyl, cycloalkyl and cycloalkylalkyl; Y may also represent a hydrogen atom when B represents a group B₁ or B₂; and
• X represents an oxygen atom, a sulphur atom or a group 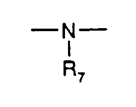 wherein R₇ represents a hydrogen atom or a radical selected from alkyl, aryl, substituted aryl, arylalkyl and substituted arylalkyl; or R₇ represents a group of the formula -A-B-Y as defined above, and in that case R₅ has a meaning selected from those defined for R₁, R₂, R₃ and R₄ as defined above,
with the proviso that
- the compound of formula (I) may not be N-[2-(1H-pyrrolo[3,2-*c*]pyridin-3-yl)ethyl]-acetamide,
- and R₇ may not represent a phenyl radical when the nitrogen atom of the pyridine ring of formula (I) is in the 7-position of the heterocycle, R₁ is an alkyl group in the 4-position of the heterocycle and R₂, R₃ and R₄ represent hydrogen atoms,
wherein:
- the terms "alkyl" and "alkoxy" denote linear or branched groups containing from 1 to 6 carbon atoms,
- the terms "alkenyl" and "alkynyl" denote unsaturated linear or branched groups containing from 2 to 6 carbon atoms,
- the term "cycloalkyl" denotes a group having from 3 to 8 carbon atoms,
- the term "aryl" denotes a phenyl, naphthyl or pyridyl radical,
- the term "substituted" associated with the expressions "aryl" and "arylalkyl" indicates that those groups may be substituted on the aromatic rings by one or more radicals selected from halogen, alkyl, alkoxy, hydroxy, and alkyl substituted by one or more halogen atoms;
their enantiomers and diastereoisomers, and addition salts thereof with a pharmaceutically acceptable acid or base.

2. Compounds of formula (I) according to claim 1 corresponding to formulae (12) and (13) below: 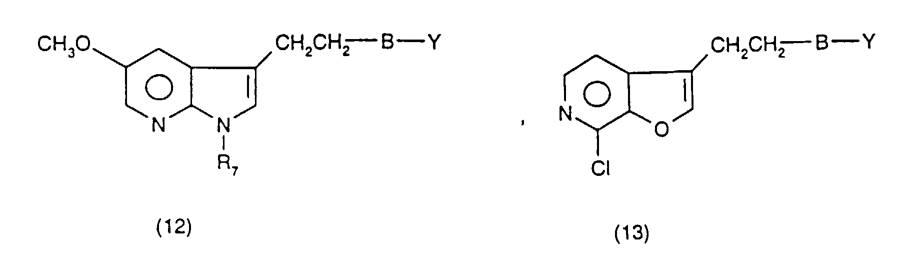 in which R₇, B and Y are as defined in claim 1.

3. Compound according to claim 1 which is N-[2-(5-methoxy-1-methylpyrrolo[2,3-*b*]-pyridin-3-yl)ethyl]acetamide.

4. Compound according to claim 1 which is N-[2-(7-chloro-furo[2,3-*c*]pyridin-3-yl)-ethyl]acetamide.

5. Process for the preparation of compounds of formula (I) according to claim 1, characterised in that, when B represents a group B₁ or B₂ as defined in claim 1, an amine of formula (II): 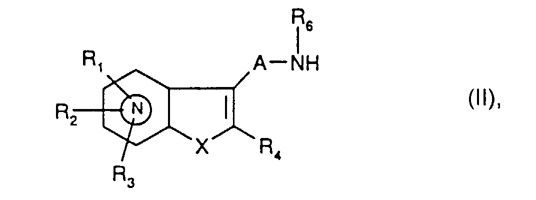 in which R₁, R₂, R₃, R₄, R₆, A and X are as defined in claim 1,
is reacted
- either with a compound of formula (Illa) or (Illb): 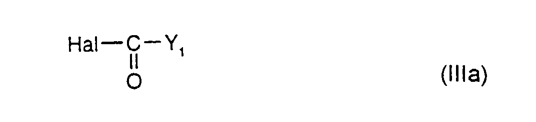 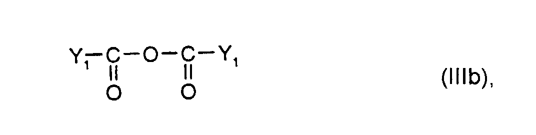 the anhydride (Illb) being mixed or symmetric,
in which Y₁ is as defined in claim 1 and Hal represents a halogen atom, in order to obtain a compound of formula (I/a): 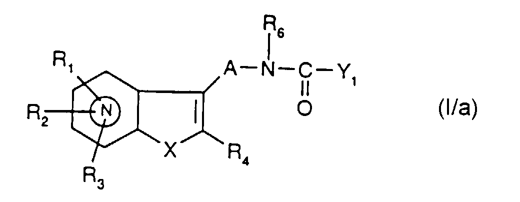 in which R₁, R₂, R₃, R₄, R₆, A, Y₁ and X are as defined above,
which compound of formula (I/a) is then subjected to Lawesson's reagent to obtain a compound of formula (I/b): 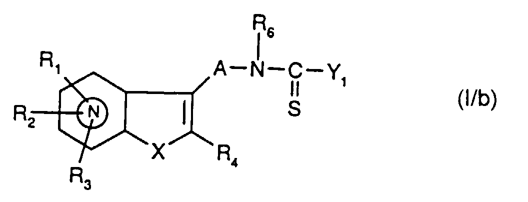 in which R₁, R₂, R₃, R₄, R₆, A, Y₁ and X are as defined above,
- or with formic acid to obtain a compound of formula (I/c): 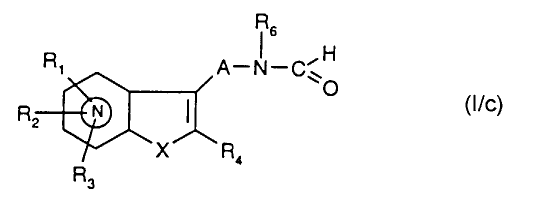 in which R₁, R₂, R₃, R₄, R₆, A and X are as defined above,
- or with a compound of formula (IV):
Z = C = N - Y (IV),
in which Y and Z are as defined in claim 1, in order to obtain a compound of formula (I/d): 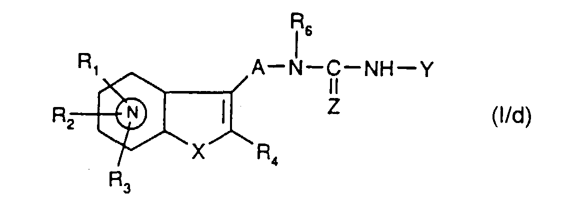 in which R₁, R₂, R₃, R₆, A, X, Y and Z are as defined above,
and characterised in that, when B represents a group B₃ as defined in claim 1, a compound of formula (V): 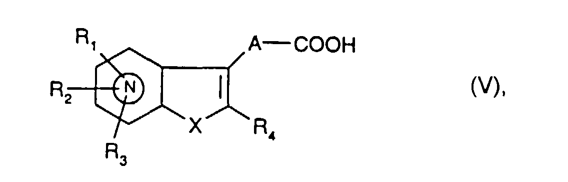 in which R₁, R₂, R₃, R₄, A and X are as defined in claim 1, is reacted with an amine of formula (VI): 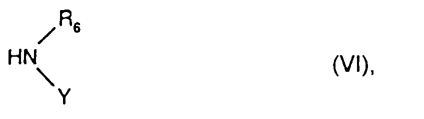 in which R₆ and Y are as defined in claim 1, to obtain a compound of formula (I/e): 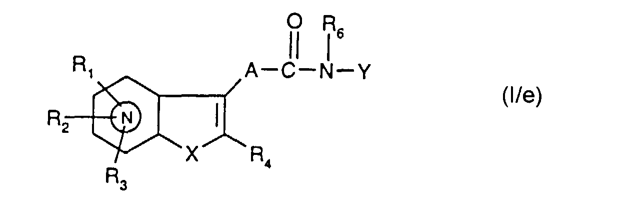 in which R₁, R₂, R₃, R₄, R₆, A, X and Y are as defined above,
which compound of formula (I/e) is subjected to Lawesson's reagent to obtain a compound of formula (I/f): 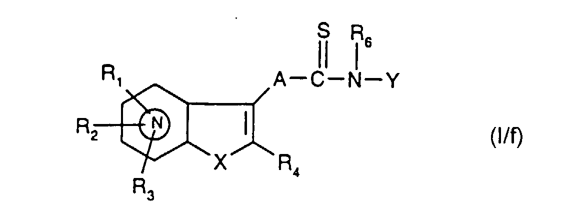 in which R₁, R₂, R₃, R₄, R₆, A, X and Y are as defined above,
it being understood that, when R₇ as defined in claim 1 represents a group of the formula -A-B-Y as defined in claim 1, the preparation process is analogous to that described above, the reagents of formulae (Illa), (Illb) and (IV) and the formic acid on the one hand and (VI) on the other hand being made to react respectively with groups of the formula -A-NH-R₆ or -A-COOH, which are fixed in the 1-position of the pyrrolopyridine heterocycle present in claim 1, it being possible for the resulting compounds of formula (I) to be:
- purified by one or more purification methods selected from crystallisation, chromatography, extraction, filtration, and passage over charcoal or resin,
- separated, where appropriate, in pure form or in the form of a mixture, into their possible enantiomers or diastereoisomers,
- or converted into a salt by means of a pharmaceutically acceptable acid or base.

6. Process for the preparation of compounds of formula (I) according to claim 1, characterised in that a compound of formula (VII) 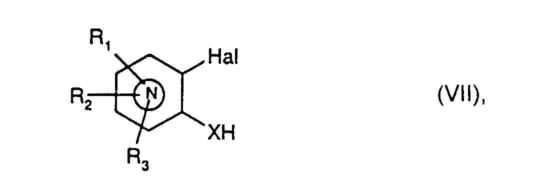 in which R₁, R₂, R₃ and X are as defined in claim 1 and Hal represents a halogen atom,
is condensed with a compound of formula (VIII): 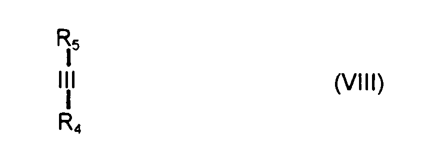 in which R₄ and R₅ are as defined in claim 1,
it being possible for the resulting compounds of formula (I) to be:
- purified by one or more purification methods selected from crystallisation, chromatography, extraction, filtration, and passage over charcoal or resin,
- separated, where appropriate, in pure form or in the form of a mixture, into their possible enantiomers or diastereoisomers,
- or converted into a salt by means of a pharmaceutically acceptable acid or base.

7. Process for the preparation of compounds of formula (I/i), which are a particular case of the compounds of formula (I) according to claim 1: 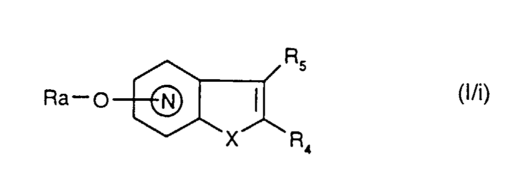 in which Ra, R₄, R₅ and X are as defined in claim 1, by grafting the radical Ra onto a compound of formula (I/j): 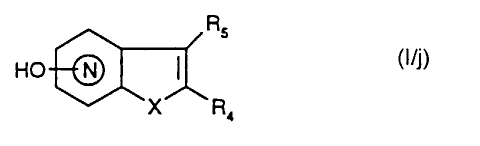 in which R_{4,} R₅ and X are as defined above,
which compounds of formula (I/i) are, where appropriate,
- purified by one or more purification methods selected from crystallisation, chromatography, extraction, filtration, and passage over charcoal or resin,
- separated, where appropriate, in pure form or in the form of a mixture, into their possible enantiomers or diastereoisomers,
- or converted into a salt by means of a pharmaceutically acceptable acid or base.

8. Process for the preparation of compounds of formula (I/k), which are a particular case of the compounds of formula (I) according to claim 1: 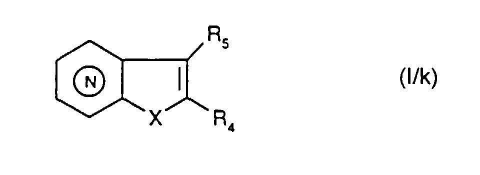 in which R_{4,} R₅ and X are as defined in claim 1, by acid treatment of a compound of formula (I/ℓ), which is a particular case of the compounds of formula (I) according to claim 1: 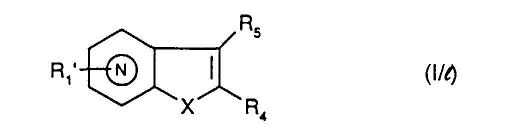 in which R_{4,} R₅ and X are as defined above and R₁' is a halogen atom,
which compounds of formula (I/k) are, where appropriate,
- purified by one or more purification methods selected from crystallisation, chromatography, extraction, filtration, and passage over charcoal or resin,
- separated, where appropriate, in pure form or in the form of a mixture, into their possible enantiomers or diastereoisomers,
- or converted into a salt by means of a pharmaceutically acceptable acid or base.

9. Pharmaceutical compositions comprising a compound of formula (I) according to claim 1 or an addition salt thereof with a pharmaceutically acceptable acid or base, in combination with one or more pharmaceutically acceptable excipients.

10. Pharmaceutical compositions according to claim 9 for use in the treatment of disorders of the melatoninergic system.
